# EUROPEAN PATENT SPECIFICATION

(11) **EP 0 229 329 B1**
(45) Date of publication and mention of the grant of the patent: **28.07.1993**
(21) Application number: 86117264.1
(22) Date of filing: 11.12.1986
(51) Int. Cl.: C07D 223/16, A61K 31/55

(54) **Benzazepine derivatives**
Benzazepinderivate
Dérivés de benzazépine

(30) Priority: 12.12.1985 US 808183; 09.10.1986 US 917130
(43) Date of publication of application: 22.07.1987
(73) Proprietor: E.R. Squibb & Sons, Inc., Princeton, N.J. 08540-4000 (US)
(72) Inventor: Das, Jagabandhu, Plainsboro, N.J. (US); Floyd, David Mack, Pennington, N.J. (US); Krapcho, John, Somerset, N.J. (US)
(74) Representative: VOSSIUS & PARTNER

(56) References cited:
- EP-A- 0 205 334
- US-A- 3 075 967
- US-A- 3 330 823
- THE MERCK INDEX, 9th Edition, 1976, page 425, abstract no. 3187 Diltiazem, Merck & CO., Rahway, N.J., US
- A. Burger, "Medicinal Chemistry", John Wiley and Sons Inc., New York, 1951, pages 75-76

## Description

Compounds having the formula 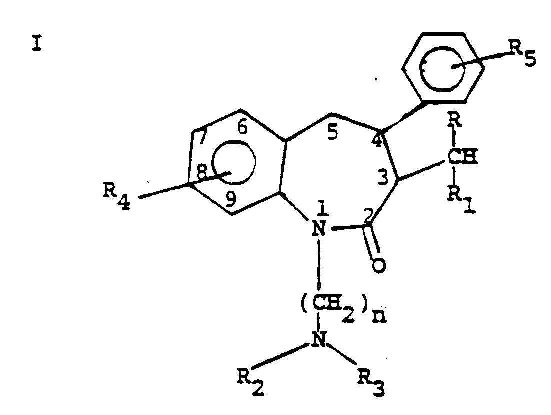
and the pharmaceutically acceptable salts thereof, have useful vasodilating activity. In formula I, and throughout the specification, the symbols are as defined below.
R and R₁ are each hydrogen or alkyl, R is hydrogen and R₁ is C₁₋₁₀-alkyl, C₂₋₁₀-alkenyl, C₂₋₁₀-alkynyl, aryl, or C₃₋₇-cycloalkyl,
R₂ and R₃ are each independently C₁₋₁₀-alkyl or C₃₋₇-cycloalkyl or R₂ and R₃ together with the nitrogen atom to which they are attached are pyrrolidinyl, piperidinyl, or morpholinyl;
R₄ and R₅ are each independently hydrogen, halogen, C₁₋₁₀-alkyl, C₁₋₁₀-alkoxy, aryloxy, aryl-C₁₋₁₀-alkoxy, aryl-C₁₋₁₀-alkyl, hydroxy, C₂₋₁₁-alkanoyloxy, 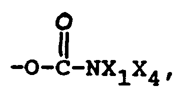
difluoromethoxy, trifluoromethyl, -NX₃X₄, -S(O)ₘC1-10-alkyl, or -S(O)ₘaryl;
n is 2 or 3;
m is 0, 1 or 2;
X₁ and X₂ are each indpendently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl;
with the provisio that if R₄ is a 7-alkyl group, it must have a tertiary carbon atom bonded to the ring;
Listed below are definitions of various terms used to describe the benzazepines of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred. Lower alkyl groups having 1 to 6 carbon atoms are especially preferred. Within those, methyl is most preferred.

The term "aryl" refers to phenyl and substituted phenyl. Exemplary substituted phenyl groups are phenyl groups substituted with 1, 2 or 3 amino (-NH₂), alkylamino, dialkylamino, nitro, halogen, hydroxyl, trifluoromethyl, alkyl (of 1 to 4 carbon atoms), alkoxy (of 1 to 4 carbon atoms), alkanoyloxy, carbamoyl, or carboxyl groups.

The term "alkanoyl" refers to groups having the formula 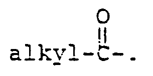
Those alkanoyl groups having 2 to 11 carbon atoms are preferred.

The term "heteroaryl" refers to an aromatic heterocyclic group having at least one heteroatom in the ring. Preferred groups are pyridinyl, pyrrolyl, imidazolyl, furyl, thienyl,and thiazolyl.

The term "nitrogen containing heteroaryl" refers to an aromatic heterocyclic group having at least one nitrogen atom in the ring. Preferred groups are pyridinyl, pyrrolyl, imidazolyl and thiazolyl.

The term "cycloalkyl" refers to groups having 3, 4, 5, 6 or 7 carbon atoms.

The term "halogen" refers to fluorine, chlorine, bromine and iodine.

The terms "alkenyl" and "alkynyl" refer to both straight and branched chain groups. Those groups having 2 to 10 carbon atoms are preferred.

The compounds of formula I form acid-addition salts with inorganic and organic acids. These acid-addition salts frequently provide useful means for isolating the products from reaction mixtures by forming the salt in a medium in which it is insoluble. The free base may then be obtained by neutralization, e.g., with a base such as sodium hydroxide. Any other salt may then be formed from the free base and the appropriate inorganic or organic acid. Illustrative are the hydrohalides, especially the hydrochloride and hydrobromide, sulfate, nitrate, phosphate, borate, acetate, tartrate, maleate, citrate, succinate, benzoate, ascorbate, salicylate, methanesulfonate, benzenesulfonate, toluenesulfonate and the like.

The carbon atoms in the 3 and 4-positions of the benzazepine nucleus of the compound of formula I are asymmetric carbons. The compounds of formula I, therefore, exist in enantiomeric and diastereomeric forms and as racemic mixtures thereof. All are within the scope of this invention. It is believed that those compounds of formula I which have the d-cis configuration are the most potent and are therefore preferred.

The compounds of formula I and the pharmaceutically acceptable salts thereof are useful as cardiovascular agents. These compounds act as vasodilators and are especially useful as anti-hypertensive agents. By the administration of a composition containing one (or a combination) of the compounds of this invention the blood pressure of a hypertensive mammalian (e.g., human) host is reduced. Daily doses of about 0.1 to 100 mg. per kilogram of body weight per day, preferably about 1 to about 50 mg. per kilogram per day, are appropriate to reduce blood pressure, and can be administered in single or divided doses. The substance is preferably administered orally, but parenteral routes such as the subcutaneous, intramuscular, or intravenous routes can also be employed.

As a result of the vasodilating activity of the compounds of formula I, it is believed that such compounds in addition to being anti-hypertensive may also be useful as anti-arrhythmic agents, as anti-anginal agents, as anti-fibrillatory agents, as anti-asthmatic agents, and in limiting myocardial infarction.

The compounds of this invention can also be formulated in combination with a diuretic, or a beta-adrenergic agent, or angiotensin converting enzyme inhibitor. Suitable diuretics include the thiazide diuretics such as hydrochlorothiazide and bendroflumethiazide, suitable beta-adrenergic agents include nadolol, and suitable angiotensin converting enzyme inhitibors include captopril.

US-A-3,075,967 relates to benzothiazepine derivatives useful in the treatment of Parkinsonism.

US-A-3,330,823 discloses benzoazepinones and benzoocinones which are also useful in the treatment of Parkinsonism and of hypertension.

The Merck Index, 9th Edition, 1976, page 425, abstract no. 3187 discloses the compound Diltiazem a vasodilatory drug.

EP-A-0,205,334 relates to benzoazepinones substituted in the third position by an oxygen-bridged group.

The compounds of formula I can be prepared by first reacting a 2-nitrotoluene having the formula 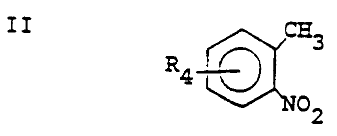
with a benzylidene malonate having the formula 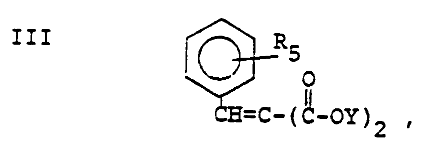
wherein Y is alkyl. The reaction can be run in a polar nonprotic solvent (e.g., dimethylformamide), in the presence of a strong base such as sodium hydride, and yields a product having the formula

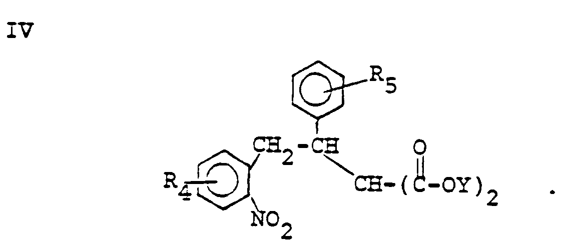

Reduction of a compound of formula IV yields the corresponding compound having the formula 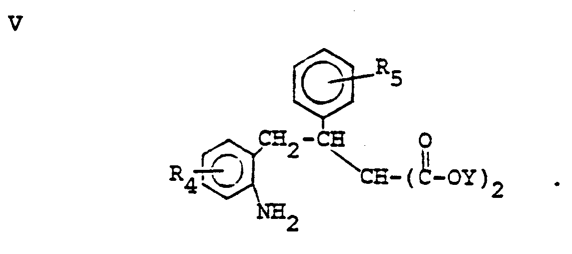
The reduction can be accomplished by catalytic hydrogenation (using, for example, palladium on charcoal as a catalyst) or using a chemical reducing agent (e.g., ferrous sulfate or stannous chloride).

Treatment of an amine of formula V with an alkali metal alkoxide (e.g., sodium methoxide) and an alcohol (e.g., methanol) yields the corresponding benzazepine having the formula

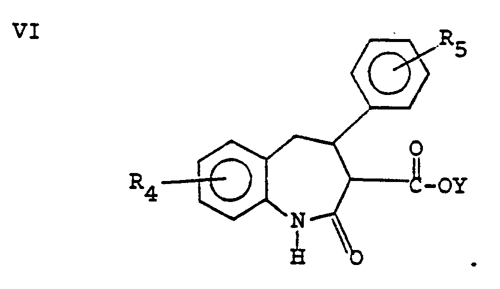

Reaction of a compound of formula VI is carried out with a strong base (e.g., lithium diisopropylamide or potassium hexamethyldisilazide) in an etheral solvent, such as tetrahydrofuran, at a reduced temperature. Alkylation is achieved by the addition of an alkylating agent (e.g., a compound 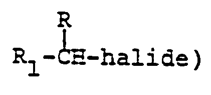
and yields a compound having the formula 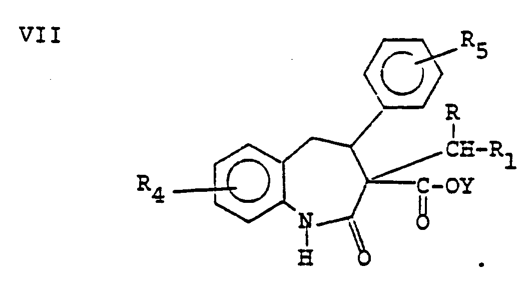
In some instances, the alkylation reaction may proceed more completely if the nitrogen atom in the benzazepine nucleus of a compound of formula VI is first protected from participation in the reaction; e.g., by treatment of a benzazepine of formula VI with a base such as sodium hydride followed by reaction with an alkoxymethyl bromide. After the alkylation reaction has been completed, the nitrogen protecting group is removed.

The preparation of a compound of formula VII from a compound of formula IV can be accomplished by alternate methodology. Alkylation of a compound of formula IV by treatment with a base such as sodium hydride followed by reaction with an alkylating agent (e.g., a compound 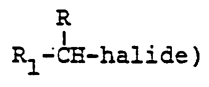
yields a compound having the formula

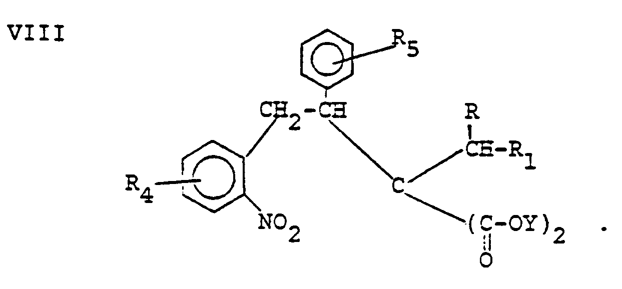

Reduction of a compound of formula VIII yields the corresponding compound having the formula 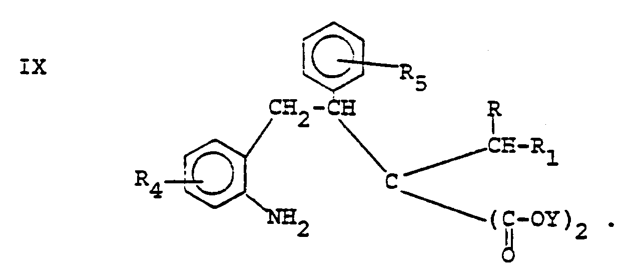
The reduction can be accomplished using a chemical reducing agent (e.g., ferrous sulfate or stannous chloride) or by catalytic hydrogenation (using, for example, palladium on charcoal as a catalyst).

Treatment of an amine of formula IX with an alkali metal alkoxide (e.g., sodium methoxide) and an alcohol (e.g., methanol) yields the corresponding benzazepine of formula VII.

Decarboxylation of a compound of formula VII can be accomplished by treating the compound with excess lithium iodide in hot pyridine to obtain a mixture of isomers having the formulas 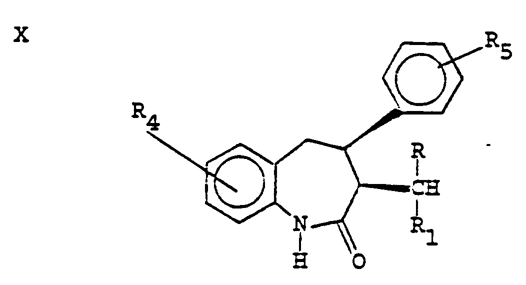
and cis isomer 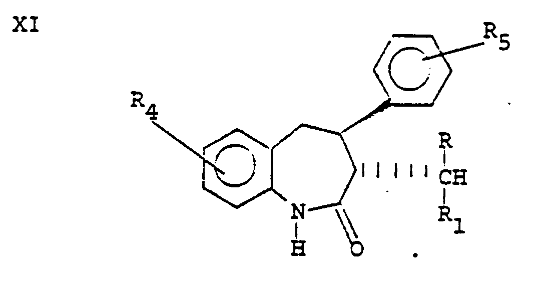
trans isomer
The preferred cis isomer is generally the predominant isomer formed during the above reaction. The use of a few drops of water in the above-described reaction mixture increases the ratio of cis isomer to trans isomer. The isomers can be separated using art recognized techniques such as crystallization or chromatography. Alternatively, the reactions described hereinafter can be run using the diastereomeric mixture (mixture of compounds of formulas X and XI). The isomeric mixture can be separated into its component isomers at any point during the reaction sequence.

Treatment of a compound of formula X or XI with an alkali metal hydride (e.g., sodium hydride) in an inert solvent such as dimethylformamide or dimethylsulfoxide, followed by reaction with a compound having the formula

XII halogen-(CH₂)ₙ-NR₂R₃,

yields the corresponding compound having the formula 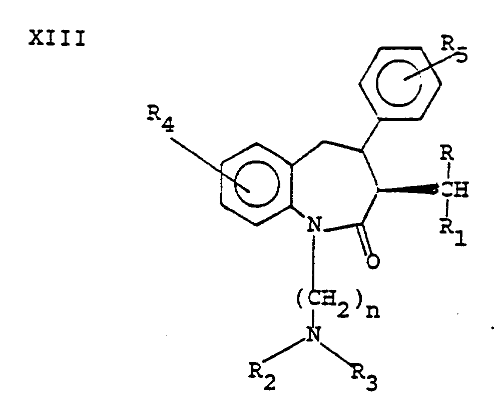
or corresponding trans isomer.

The resolved enantiomers of the compounds of this invention can be prepared by first hydrolyzing a compound of formula VI to obtain the corresponding carboxylic acid having the formula 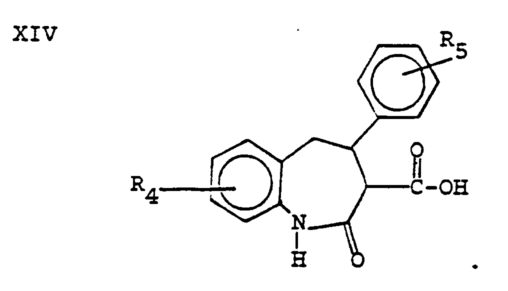
The hydrolysis can be accomplished, for example, by treating a compound of formula VI with an alkali metal hydroxide in an alcohol (e.g., potassium hydroxide in methanol).

A carboxylic acid of formula XIV can be resolved using a chiral amine. Reaction of the acid and amine in an appropriate solvent yields the diastereomeric salts which can be separated using conventional techniques such as crystallization. Regeneration of the carboxylic acid from the pure diastereomeric salt followed by esterification yields the desired nonracemic form of a compound of formula VI. This nonracemic compound can be converted to the corresponding nonracemic product of formula I via the nonracemic forms of intermediates of formulas X and XI using the procedures described above.

Additional methods for preparing the compounds described herein will be apparent to those skilled in the art. For example, those compounds of formula I wherein R₁ is alkyl can be prepared by reducing the corresponding compound of formula X or XI wherein R₁ is alkenyl or alkynyl, and then treating the resulting compound with an alkali metal hydride followed by reaction with a compound of formula XII.

Preferred members of each of the substituent groups of the benzazepines of formula I are as follows: R and R₁ are hydrogen or R is hydrogen and R₁ is vinyl or methyl; n is 2; R₂ and R₃ are each methyl; R₄ is halogen or trifluoromethyl (especially 6 or 7-trifluoromethyl) and R₅ is p-methoxy.

The following examples are specific embodiments of this invention.

### Example 1

### (cis)-7-Chloro-1-[2-(dimethylamino)ethyl]-1,3,4,5-tetrahydro-4-(4-methoxyphenyl)-3-methyl-2H-1-benzazepin-2-one, monohydrochloride

### A) [2-(5-Chloro-2-nitrophenyl)-1-(4-methoxyphenyl)ethyl]propanedioic acid, dimethyl ester

To a stirred mixture of dimethyl p-methoxybenzilidene malonate (40 g, 0.16 mole) and 60% dispersion of sodium hydride (9.6 g, 0.24 mole) in 350 ml of dry dimethylformamide, was added dropwise over 2 hours a solution of 5-chloro-2-nitrotoluene (30 g, 0.176 mole) in 30 ml of dimethylformamide. The reaction was stirred at room temperature for 6 hours, then quenched with glacial acetic acid (15.4 ml, 0.26 mole). The solvent was removed in vacuo and the residue was triturated with water. The yellow solids were filtered and triturated with methanol to yield 50.3 g of a white solid, melting point 128.5-130.5°C.

### B) [2-(2-Amino-5-chlorophenyl)-1-(4-methoxyphenyl)ethyl]propanedioic acid, dimethyl ester

To a refluxing mixture of [2-(5-chloro-2-nitropbenyl)-1-(4-methoxyphenyl)ethyl]propanedioic acid, dimethyl ester (40 g, 95.0 mmole) and hydrated ferrous sulfate (184.5 g, 0.663 mole) in a (1:10) solution of methanol:water (1.2 L) was added concentrated ammonium hydroxide (142.5 ml) over a 30 minute period. The reaction was stirred at reflux for 20 minutes then cooled to room temperature. Ethyl acetate and Celite® were added and the mixture was filtered through Celite®. The filtrate was partitioned between ethyl acetate and water. The organic phase was dried over magnesium sulfate and concentrated in vacuo. The product was recrystallized from isopropyl alcohol to yield 28.22 g of the title compound, melting point 114-116°C.

### C) 7-Chloro-1,3,4,5-tetrahydro-3-(methoxycarbonyl)-4-(4-methoxyphenyl)-2H-1-benzazepin-2-one

To a solution of [2-(2-amino-5-chlorophenyl)-1-(4-methoxyphenyl)ethyl]propanedioic acid, dimethyl ester (23.2 g, 59.2 mmole) in methanol (200 ml) was added a 25% solution of sodium methoxide in methanol (16 ml, 69.97 mmole). The solution was refluxed for 3 hours under argon. The reaction was cooled to room temperature and treated with 200 ml 1N hydrochloric acid. A white precipitate was filtered and washed with water, methanol, and dried in vacuo to yield 19.5 g of the title compound, melting point 189-190.5°C.

### D) 7-Chloro-1,3,4,5-tetrahydro-3-(methoxycarbonyl)-4-(4-methoxyphenyl)-3-methyl-2H-1-benzazepin-2-one

To 25 mmole (3 equiv.) of a freshly prepared solution of lithium diisopropylamide in tetrahydrofuran (prepared by addition of 9.6 ml of a 2.6 M solution of n-butyllithium in hexane to 7.5 ml of freshly distilled diisopropylamine in 50 ml of tetrahydrofuran at -78°C) was added 3.0 g of 7-chloro-1,3,4,5-tetrahydro-3-(methoxycarbonyl)-4-(4-methoxyphenyl)-2H-1-benzazepin-2-one (8.35 mmole). After stirring at -78°C for 1.5 hours, 1.5 ml of hexamethylphosphoric triamide was added along with 5.9 g of iodomethane (41.7 mmole, 5 equiv.). The reaction mixture was stirred at -78°C to 20°C for 6 hours, then quenched with 50 ml of 1N hydrochloric acid. The stirring was continued at 50°C for 30 minutes. The layers were separated. The aqueous layer was extracted with two 50 ml portions of ethyl acetate. The combined organic layer was washed with two 20 ml portions of saturated sodium bicarbonate, 20 ml of water then dried (magnesium sulfate) and concentrated. The residue was purified on a silica gel column. Elution with 25% ethyl acetate/hexanes gave 1.4 g of 7-chloro-1,3,4,5-tetrahydro-3-(methoxycarbonyl)-4-(4-methoxyphenyl)-3-methyl-2H-1-benzazepin-2-one as a white solid.

### E) (cis)-7-Chloro-1,3,4,5-tetrahydro-4-(4-methoxyphenyl)-3-methyl-2H-1-benzazepin-2-one

A mixture of 800 mg of 7-chloro-1,3,4,5-tetrahydro-3-(methoxycarbonyl)-4-(4-methoxyphenyl)-3-methyl-2H-1-benzazepin-2-one (2.16 mmole), 1.14 g of lithium iodide (8.56 mmoles, 4 equiv.) in 20 ml of pyridine was refluxed for 20 hours. The cooled mixture was diluted with 200 ml of ethyl acetate, then washed with four 30 ml portions of 1N hydrochloric acid, two 30 ml portions of saturated cupric sulfate, 30 ml of water. The organic layer was dried (magnesium sulfate) and concentrated. The residue was triturated with ether to yield 400 mg of (cis)-7-chloro-1,3,4,5-tetrahydro-4-(4-methoxyphenyl)-3-methyl-2H-1-benzazepin-2-one as a white solid.

### F) (cis)-7-Chloro-1-[2-(dimethylamino)ethyl]-1,3,4,5-tetrahydro-4-(4-methoxyphenyl)-3-methyl-2H-1-benzazepin-2-one, monohydrochloride

To a slurry of hexane washed sodium hydride (132 mg of 50% sodium hydride in mineral oil, 2.74 mmole) in 20 ml of dimethylformamide at 25°C was added 720 mg of (cis)-7-chloro-1,3,4,5-tetrahydro-4-(4-methoxyphenyl)-3-methyl-2H-1-benzazepin-2-one (2.28 mmole). After stirring at 25°C for 1 hour, the solution turned clear, 6.7 ml of a 1.7 M solution of 2-dimethylaminoethyl chloride (11.4 mmole, 5 equiv.) in toluene was added. The reaction mixture was stirred for 3 hours at 80°C, then cooled to 25°C and quenched with 1N hydrochloric acid, basified with 1N sodium hydroxide, and extracted with ethyl acetate. The organic layer was dried (magnesium sulfate) and concentrated. The residue was diluted with ether and treated with a saturated solution of hydrochloric acid in ether and concentrated. The residue was triturated with ether and filtered. The solid was washed with ether and dried to give 780 mg of the title compound as a white solid, melting point 228°-231°C.
TLC: silica gel; 10% methanol/dichloromethane;
R_{f}=0.48

| Analysis Calc'd. for C₂₂H₂₈N₂O₂Cl 0.8H₂O: | | | | |
|---|---|---|---|---|
| | C, 60.36; | H, 6.82; | N, 6.40; | Cl, 16.20 |
| Found: | C, 60.39; | H, 6.45; | N, 6.32; | Cl, 16.40 |

### Example 2

### (cis)-7-Chloro-1-[2-(dimethylamino)ethyl]-3-ethyl-1,3,4,5-tetrahydro-4-(4-methoxyphenyl)-2H-1-benzazepin-2-one, monohydrochloride

### A) 7-Chloro-3-ethyl-1,3,4,5-tetrahydro-3-(methoxycarbonyl)-4-(4-methoxyphenyl)-2H-1-benzazepin-2-one

To a freshly prepared solution of lithium diisopropylamide in tetrahydrofuran (6 equivalents; prepared by addition of 19.2 ml of a 2.6M solution of n-butyllithium in hexane to 15 ml of freshly distilled diisopropylamine in 80 ml of tetrahydrofuran) at -78°C, was added 3.0 g (8.35 mmole) of 7-chloro-1,3,4,5-tetrahydro-3-(methoxycarbonyl)-4-(4-methoxyphenyl)-2H-1-benzazepin-2-one (see Example 1C). After stirring at -78°C for 1.5 hours, 3 ml of hexamethylphosphoric triamide was added along with 13.6 ml of iodoethane (filtered through alumina to remove iodine). The mixture was stirred at -78°C for 30 minutes (under argon), and then at -20°C (dry-ice/carbon tetrachloride bath) for six days. The reaction was quenched with 1N hydrochloric acid (50 ml), and the aqueous layer was extracted with ethyl acetate (three times). The ethyl acetate extracts and the tetrahydrofuran were combined, washed with saturated sodium bicarbonate and saturated sodium chloride, and dried (magnesium sulfate). The solution was concentrated, combined with 0.31 g of previously prepared crude product and flash chromatographed (silica gel/3:1-hexane:ethyl acetate) yielding 0.61 g of product.

### B) 7-Chloro-3-ethyl-1,3,4,5-tetrahydro-4-(4-methoxyphenyl)-2H-1-benzazepin-2-one

Lithium iodide (0.65 g; 4.84 mmole) was added to 7-chloro-3-ethyl-1,3,4,5-tetrahydro-3-(methoxycarbonyl)-4-(4-methoxyphenyl)-2H-1-benzazepin-2-one (0.47 g; 1.21 mmole) in pyridine (15 ml) and water (three drops) and refluxed with stirring for 33 hours. The mixture was cooled, dissolved into ethyl acetate and washed with 1N hydrochloric acid (three times). The combined ethyl acetate layers were dried (magnesium sulfate) and concentrated giving 0.40 g of crude material. This material was combined with another batch of crude (0.49 g, total); flash chromatography purification (silica gel/1:1-hexane:ethyl acetate) gave 0.38 g of the title compound.

### C) (cis)-7-Chloro-1-[2-(dimethylamino)ethyl]-3-ethyl-1,3,4,5-tetrahydro-4-(4-methoxyphenyl)-2H-1-benzazepin-2-one, monohydrochloride

To a slurry of prewashed (hexane) sodium hydride (∼50% in mineral oil) (0.064 g; 1.34 mmole; 1.2 eq.) in dry dimethylformamide (10 ml) was added 7-chloro-3-ethyl-1,3,4,5-tetrahydro-4-(4-methoxyphenyl)-2H-1-benzazepin-2-one (370 mg; 1.12 mmole). After stirring the mixture at 25°C for 1 hour, 3.2 ml (5.6 mmole) of 1.7N N,N-dimethyl-2-chloroethylamine (in toluene) was added, and the solution was heated at 80°C for 24 hours. The reaction was quenched with 1N hydrochloric acid, made basic with 50% sodium hydroxide solution, extracted with ethyl acetate (two times) and dried (magnesium sulfate). After solvent removal, 0.49 g of a brown oil remained. This crude product was flash chromatographed (silica gel/3:1-hexane:ethyl acetate, followed by 2:1-hexane: ethyl acetate, followed by 1:1-hexane: ethyl acetate, followed by ethyl acetate). Those fractions which contained the cis amine product were dissolved into ether. The salt was precipitated out by the dropwise addition of hydrogen chloride saturated ether solution. The resultant white solid was collected by suction-filtration, yielding 200 mg. The lower R_{f} trans isomer of the title compound was likewise obtained (10 mg) as well as a cis-trans mixture (50 mg), and unreacted starting material (40 mg).

| Analysis Calc'd for C₂₃H₂₉ClN₂O₂·HCl·0.23H₂O: | | | | |
|---|---|---|---|---|
| | C, 62.56; | H, 6.95; | N, 6.36; | Cl, 16.06 |
| Found: | C, 62.56; | H, 6.80; | N, 6.22; | Cl, 16.00 |

### Example 3

### (cis)-7-Chloro-1-[2-(dimethylamino)ethyl]-1,3,4,5-tetrahydro-4-(4-methoxyphenyl)-3-(2-propenyl)-2H-1-benzazepin-2-one, monohydrochloride

### A) 7-Chloro-1,3,4,5-tetrahydro-3-(methoxycarbonyl)-1-(methoxymethyl)-4-(4-methoxyphenyl)-2H-1-benzazepin-2-one

To a solution of 7-chloro-1,3,4,5-tetrahydro-3-(methoxycarbonyl)-4-(4-methoxyphenyl)-2H-1-benzazepin-2-one (270 mg; 0.75 mmole; see Example 1C) in dimethylformamide (10 ml), cooled at 0-5°C in an ice-water bath, was added 50% sodium hydride (72 mg; 1.5 mmole). After stirring at 0-5°C for 15 minutes, methoxymethylbromide (240 µl; 3 mmole) was added dropwise. The reaction mixture was allowed to stir at 0-5°C for two additional hours. The excess sodium hydride was destroyed by the addition of water, and the resulting mixture was extracted with ether. The aqueous layer was extracted with ether (three times) and the combined organic layers were dried (magnesium sulfate) and concentrated. The crude residue was flash chromatographed (silica gel/5-20% ethyl acetate:hexane) yielding 233 mg of the title compound as an oil.

### B) 7-Chloro-1,3,4,5-tetrahydro-3-(methoxycarbonyl)-1-(methoxymethyl)-4-(4-methoxyphenyl)-3-(2-propenyl)-2H-1-benzazepin-2-one

To a suspension of 50% sodium hydride (48 mg; 1 mmole) in dimethylformamide (5 ml) cooled at 0-5°C was added dropwise a solution of 7-chloro-1,3,4,5-tetrahydro-3-(methoxycarbonyl)-1-(methoxymethyl)-4-(4-methoxyphenyl)-2H-1-benzazepin-2-one (102 mg; 0.25 mmole) in dimethylformamide (1 ml). After stirring at 0-5°C for 20 minutes, allyl bromide (360 µl; 4 mmole) was added dropwise, and the reaction mixture was allowed to stir at 0°C to room temperature for two hours. Excess sodium hydride was destroyed with water, and the mixture was extracted with ether. The aqueous layer was extracted with ether (two times), and the combined organic layers were dried (magnesium sulfate) and concentrated. The crude residue was triturated with hexane (20 ml) to obtain 93 mg of white, crystalline title compound. [The mother liquor contained additional product].

### C) 7-Chloro-1,3,4,5-tetrahydro-3-(methoxycarbonyl)-4-(4-methoxyphenyl)-3-(2-propenyl)-2H-1-benzazepin-2-one

To a suspension of 7-chloro-1,3,4,5-tetrahydro-3-(methoxycarbonyl)-1-(methoxymethyl)-4-(4-methoxyphenyl)-3-(2-propenyl)-2H-1-benzazepin-2-one (715 mg, 1.61 mmole) in methanol (40 ml) was added concentrated sulfuric acid (6 ml) with stirring. The reaction mixture was refluxed (bath temperature 75-80°C) for 8 hours, and was then diluted with dichloromethane. The sulfuric acid was carefully neutralized by the addition of saturated sodium bicarbonate solution. The mixture was extracted with dichloromethane (two times) and the combined extracts were dried over anhydrous magnesium sulfate. The concentrated solution was flash chromatographed (silica gel/10-30% ethyl acetate: hexane) to obtain 700 mg of the title compound.

### D) 7-Chloro-1,3,4,5-tetrahydro-4-(4-methoxyphenyl)-3-(2-propenyl)-2H-1-benzazepin-2-one

Lithium iodide (0.90 g; 6.7 mmole) was added to 7-chloro-1,3,4,5-tetrahydro-3-(methoxycarbonyl)-4-(4-methoxyphenyl)-3-(2-propenyl)-2H-1-benzazepin-2-one (670 mg; 1.68 mmole) in pyridine (5 ml), three drops of water were added, and the mixture was refluxed with stirring overnight. The solution was dissolved into ethyl acetate and washed with 1N hydrochloric acid (three times). The organic layer was dried (magnesium sulfate) and concentrated. The crude, brownish solid was dissolved into ethyl acetate and suction-filtered through a pad of silica gel (to remove some of the brown color). The silica gel was rinsed several times with ethyl acetate, and the organic solution was concentrated and vacuum dried, leaving 0.52 g of the title compound as an off-white solid.

### E) (cis)-7-Chloro-1-[2-(dimethylamino)ethyl]-1,3,4,5-tetrahydro-4-(4-methoxyphenyl)-3-(2-propenyl)-2H-1-benzazepin-2-one, monohydrochloride

To a slurry of washed (hexane) sodium hydride (∼50% in mineral oil) (0.09 g; 1.83 mmole; 1.2 eq.) in dimethylformamide (13 ml) was added 7-chloro-1,3,4,5-tetrahydro-4-(4-methoxyphenyl)-3-(2-propenyl)-2H-1-benzazepin-2-one (520 mg; 1.52 mmole) with stirring. After one hour stirring at 25°C, 1.7N N,N-dimethyl-2-chloroethylamine (in toluene) (4.5 ml; 7.60 mmole) was added, and the mixture was heated to 80°C with stirring for three hours. The reaction was quenched with 1N hydrochloric acid. The mixture was then made basic with 50% sodium hydroxide solution, extracted with ethyl acetate (two times), dried (magnesium sulfate), concentrated and dried in vacuo. The crude material was flash chromatographed (silica gel/1% methanol:dichloromethane, followed by 2% methanol:dichloromethane). The cis isomer containing fractions were combined and concentrated giving ∼400 mg of semi-solid. The residue was co-evaporated with ether producing 310 mg of a fluffy white solid. This material was dissolved in ether and hydrogen chloride saturated ether was added, giving a white precipitate that was collected by suction-filtration yielding 200 mg of the title compound. A cis/trans mixture and some pure trans product were also obtained from the flash chromatography.

| Analysis Calc'd for C₂₄H₂₉ClN₂O₂·HCl: | | | | |
|---|---|---|---|---|
| | C, 64.14; | H, 6.73; | N, 6.23; | Cl, 15.78 |
| Found: | C, 63.92; | H, 6.72; | N, 6.13; | Cl, 15.74 |

### Example 4

### (cis)-1-[2-(Dimethylamino)ethyl]-1,3,4,5-tetrahydro-4-(4-methoxyphenyl)-3-methyl-7-(trifluoromethyl)-2H-1-benzazepin-2-one, monohydrochloride

### A) [2-(2-Nitro-5-trifluoromethylphenyl)-1-(4-methoxyphenyl)ethyl]propanedioic acid, dimethyl ester

To a two liter three-neck flask (under nitrogen) was added 67.0 g (0.293 mol) of dimethyl p-methoxybenzylidene malonate and 450 ml of dimethylformamide. The stirred solution was treated with 18.7 g (0.39 mole) of a 50% sodium hydride dispersion. This mixture was treated dropwise with a solution of 60.5 g (0.253 mol) of 2-nitro-5-(trifluoromethyl)toluene in 50 ml of dimethylformamide, over a period of one hour while maintaining the temperature at 28-32°C (near the end of the addition, the temperature rose to 38°C and was rapidly cooled to 30°C). This mixture was stirred for four hours at room temperature, cooled, treated portionwise with 25 ml of acetic acid and poured onto 2.5 liters of ice water. The mixture was extracted with 250 ml of dichloromethane (three times), dried (magnesium sulfate), filtered and the solvent evaporated to give 126 g of a pale brown semi-solid. The latter was dissolved in 270 ml of methanol, cooled and filtered to give 72.8 g of a pale yellow product, melting point 110-112°C, R_{f}=0.74 (1:1 ethyl acetate-hexane). A sample recrystallized from methanol, melted at 111-113°C.

### B) α-Methyl-[2-(2-nitro-5-trifluoromethylphenyl)-1-(4-methoxyphenyl)ethyl]propanedioic acid, dimethyl ester

[2-(2-Nitro-5-trifluoromethylphenyl)-1-(4-methoxyphenyl)ethyl]propanedioic acid, dimethyl ester (7.00 g; 15.4 mmole) was dissolved in dry dimethylformamide (35 ml) under argon. Prewashed (hexane) 50% sodium hydride (0.89 g; 18.4 mmole) was added with stirring. Stirring was continued for 20 minutes before iodomethane (filtered through alumina) (11.6 g; 5.1 ml; 81.5 mmole; sp. gr. = 2.24-2.27; 5 eq.) was added dropwise. The mixture was allowed to stir a total of 4.5 hours. The solution was partitioned between ethyl acetate and 1N hydrochloric acid, and the organic layer was collected and washed again with 1N hydrochloric acid, saturated potassium carbonate, saturated sodium chloride, and dried (magnesium sulfate). The concentrated residue was flashed (silica gel/9:1-hexane:ethyl acetate, followed by 8:2-hexane:ethyl acetate). The product was collected from the appropriate fractions and concentrated giving 6.90 g of the title compound as a viscous oil.

### C) α-Methyl-[2-(2-amino-5-trifluoromethylphenyl)-1-(4-methoxyphenyl)ethyl]propanedioic acid, dimethyl ester

α-Methyl-[2-(2-nitro-5-trifluoromethylphenyl)-1-(4-methoxyphenyl)ethyl]propanedioic acid, dimethyl ester (6.80 g; 14.9 mmole) was dissolved in methanol (200 ml) under argon at room temperature. Powdered stannous chloride dihydrate (17.48 g; 77.5 mmole) was added, followed by concentrated hydrochloric acid (19 ml) with stirring. After 1.5 hours, Celite®, ethyl acetate and saturated potassium carbonate solution were added with stirring (the potassium carbonate was added portionwise). The suspension was filtered through a Celite® pad. The pad was then rinsed with ethyl acetate (three times). The filtrate was concentrated and the resulting white solid was triturated with 10% methanol:water and dried giving 6.02 g of the title compound.

### D) 1,3,4,5-Tetrahydro-3-(methoxycarbonyl)-4-(4-methoxyphenyl)-3-methyl-7-(trifluoromethyl)-2H-1-benzazepin-2-one

A 25% (by weight) sodium methoxide in methanol solution (14.2 ml; 625 mmole; 4.6 eq.; d=0.945) and α-methyl-[2-(2-amino-5-trifluoromethylphenyl)-1-(4-methoxyphenyl)ethyl]propanedioic acid, dimethyl ester (5.96 g; 13.56 mmole) in methanol (30 ml) and dry dimethylformamide (35 ml) were refluxed (∼95°C) overnight. 1N Hydrochloric acid was added with stirring producing a white precipitate that was collected by suction-filtration, washed with water (three times) and dried in vacuo giving 4.88 g of a white solid which contained an impurity as shown by NMR. The impurity disappeared after the sample stood overnight.

### E) 1,3,4,5-Tetrahydro-4-(4-methoxypheny)-3-methyl-7-(trifluoromethyl)-2H-1-benzazepin-2-one

Lithium iodide (5.26 g; 39.3 mmole) was added to 1,3,4,5-tetrahydro-3-(methoxycarbonyl)-4-(4-methoxyphenyl)-3-methyl-7-(trifluoromethyl)-2H-1-benzazepin-2-one (4.00 g; 9.82 mmole) in pyridine (40 ml) (five drops of water were added) and the mixture was refluxed with stirring for 8.5 hours. The solution was dissolved in ethyl acetate and washed with 1N hydrochloric acid (three times). The organic layer was dried (magnesium sulfate) and concentrated giving 3.43 g of the title compound as a solid.

### F) (cis)-1-[2-(Dimethylamino)ethyl]-1,3,4,5-tetrahydro-4-(4-methoxyphenyl)-3-methyl-7-(trifluoromethyl)-2H-1-benzazepin-2-one, monohydrochloride

To a solution of 1,3,4,5-tetrahydro-4-(4-methoxyphenyl)-3-methyl-7-(trifluoromethyl)-2H-1-benzazepin-2-one (3.37 g; 9.65 mmole) in dry dimethylformamide (75 ml) was added prewashed (hexane) 50% sodium hydride (5.56 g; 11.58 mmole; 1.2 eq.) with stirring. After ∼20 minutes, 1.7N (in toluene) N,N-dimethyl-2-chloroethylamine (24.0 ml; 40.8 mmole) was added, and the mixture was heated at 85°C for four hours. The mixture was made basic with 50% sodium hydride, and extracted with ethyl acetate (three times). The combined organics were dried (magnesium sulfate) and concentrated, and the dark oily residue was placed under vacuum. The crude material was flash chromatographed (silica gel/1% methanol:dichloromethane, followed by 3% methanol:dichloromethane). The acidified (hydrogen chloride saturated ether) produce weighed 1.06 g.

| Analysis Calc'd for C₂₃H₂₇F₃N₂O₂·H₂O·HCl: | | | | | |
|---|---|---|---|---|---|
| | C, 58.10; | H, 6.36; | N, 5.89; | Cl, 7.46; | F, 12.0 |
| Found: | C, 58.10; | H, 5.90; | N, 5.75; | Cl, 7.94; | F, 11.5 |

### Example 5

### (cis)-1-[2-(Dimethylamino)ethyl]-1,3,4,5-tetrahydro-4-(4-mothoxyphenyl)-3-(2-propenyl)-7-trifluoromethyl)-2H-1-benzazepin-2-one, monohydrochloride

### Method I

### A) [2-(2-Amino-5-trifluoromethylphenyl)-1-(4-methoxyphenyl)ethyl]propanedioic acid, dimethyl ester

A suspension of 25.0 g (0.055 mol) of [2-(2-nitro-5-trifluoromethylphenyl)-1-(4-methoxyphenyl)ethyl]propanedioic acid, dimethyl ester (see Example 4A) in 200 ml of methanol was treated with a cold suspension of 2.5 g of 5% palladium on charcoal in 50 ml of methanol (under nitrogen) and placed on the Parr apparatus at 58 lbs. of hydrogen. The theoretical amount of hydrogen was consumed in about 30 minutes and this mixture was then heated at 50-55°C for one hour to assure that all of the nitro compound had dissolved. The mixture was removed from the Parr and allowed to stand at room temperature overnight. The flask was heated to dissolve the crystallized product and the hot solution was filtered through Celite® (under nitrogen) and washed with hot methanol. The colorless filtrate was concentrated on a rotary evaporator to give 22.2 g of a nearly colorless solid. The latter was triturated with 100 ml of hexane and then with 50 ml of hexane. The solvent was decanted and the entrained solvent removed on a rotary evaporator to give 21.3 g of product, melting point 124-127°C. A sample of this material, after crystallization from methanol, melted at 125-127°C.

### B) 1,3,4,5-Tetrahydro-3-(methoxycarbonyl)-4-(4-methoxyphenyl)-7-(trifluoromethyl)-2H-1-benzazepin-2-one

Under an argon atmosphere, a stirred solution of [2-(2-amino-5-trifluoromethylphenyl)-1-(4-methoxyphenyl)ethyl]propanedioic acid, dimethyl ester (20.0 g; 0.047 mol) in 200 ml of methanol was treated with 13.3 ml of 25% sodium methoxide in methanol and heated to reflux. After about 2.75 hours of heating, the mixture was cooled in ice water and 1N hydrochloric acid was added to precipitate the product. Stirring in an ice water bath, followed by filtering, washing with water and air-drying yielded 19.0 g of product. The product was suspended in 30 ml of isopropanol, allowed to stand for one hour, filtered and washed with isopropanol and hexane to yield 13.64 g of the title compound, melting point 161-163°C.

### C) 1,3,4,5-Tetrahydro-3-(methoxycarbonyl)-1-(4-methoxymethyl)-4-(methoxyphenyl)-7-(trifluoromethyl)-2H-1-benzazepin-2-one

To a suspension of sodium hydride (360 mg; 7.5 mmole; 50% oil dispersion/prewashed with dry ether several times) in dry dimethylformamide (30 ml), cooled at 0-5°C was added a solution of 1,3,4,5-tetrahydro-3-(methoxycarbonyl)-4-(4-methoxyphenyl)-7-(trifluoromethyl)-2H-1-benzazepin-2-one (1.9 g; 5 mmole) in dry dimethylformamide (15 ml) dropwise and with stirring. The mixture was stirred for an additional 20 minutes at 0-5°C, whereupon bromomethylmethyl ether (800 µl; 10 mmole) was added dropwise, and stirring was continued at this temperature for an additional hour. Excess sodium hydride was destroyed by the addition of water, and the mixture was diluted with ether and washed with water. The aqueous layer was extracted with ether (three times) and the combined extracts were dried (magnesium sulfate) and concentrated. The crude oily residue was flash chromatographed (silica gel/5-25% ethyl acetate:hexane) to obtain 1.67 g of the title compound as an oil.

### D) 1,3,4,5-Tetrahydro-3-(methoxycarbonyl)-1-(methoxymethyl)-4-(4-methoxyphenyl)-3-(2-propenyl)-7-(trifluoromethyl)-2H-1-benzazepin-2-one

To a suspension of sodium hydride (384 mg; 8 mmole; 50% oil dispersion) in dry dimethylformamide (35 ml), cooled in an ice water bath, was added a solution of 1,3,4,5-tetrahydro-3-(methoxycarbonyl)-1-(methoxymethyl)-4-(-4-methoxyphenyl)-7-(trifluoromethyl)-2H-1-benzazepin-2-one (917 mg; 21 mmole) in dimethylformamide (8 ml) with stirring. After stirring at 0-5°C for 30 minutes, allyl bromide (1.5 ml) was added in one portion, the mixture was allowed to stand at 0-5°C for three additional hours, whereupon excess hydride was destroyed by the addition of water. The mixture was diluted with ether and washed with water. The aqueous layer was extracted with ether (three times), and the combined ether extracts were dried (magnesium sulfate) and concentrated. The crude residue was flash chromatographed (silica gel/5-20% ethyl acetate:hexane) to obtain 905 mg of the title compound in crystalline form.

### E) 1,3,4,5-Tetrahydro-3-(methoxycarbonyl)-4-(4-methoxyphenyl)-3-(2-propenyl)-7-(trifluoromethyl)-2H-1-benzazepin-2-one

Concentrated sulfuric acid (8 ml) and anhydrous lithium bromide (720 mg; 8 mmole) were added to a suspension of 1,3,4,5-tetrahydro-3-(methoxycarbonyl)-1-(methoxymethyl)-4-(4-methoxyphenyl)-3-(2-propenyl)-7-(trifluoromethyl)-2H-1-benzazepin-2-one (905 mg; 1.9 mmole) in methanol (40 ml) with stirring. The reaction mixture was heated under reflux (bath temperature = 80-85°C) for nine hours, and then allowed to stand overnight at room temperature. The acid was carefully neutralized by the addition of saturated sodium bicarbonate solution and extracted with ethyl acetate (three times). The combined organic layers were dried (magnesium sulfate) and concentrated yielding 858 mg of the title compound as a solid.

### F) 1,3,4,5-Tetrahydro-4-(4-methoxyphenyl)-3-(2-propenyl)-7-(trifluoromethyl)-2H-1-benzazepin-2-one

Lithium iodide (1.08 g; 8.04 mmole) was added to 1,3,4,5-tetrahydro-3-(methoxycarbonyl)-4-(4-methoxyphenyl)-3-(2-propenyl)-7-(trifluoromethyl)-2H-1-benzazepin-2-one (870 mg; 2.01 mmole) in pyridine (14 ml), three drops of water were added, and the mixture was refluxed with stirring for 6.5 hours. The solution was dissolved into ethyl acetate and washed with 1N hydrochloric acid (three times). The organic layer was dried (magnesium sulfate) and concentrated giving 0.79 g of solid. The crude material was used "as is" in the next step.

### G) (cis)-1-[2-(Dimethylamino)ethyl]-1,3,4,5-tetrahydro-4-(4-methoxyphenyl)-3-(2-propenyl)-7-trifluoromethyl)-2H-1-benzazepin-2-one, monohydrochloride

To a slurry of washed (hexane) sodium hydride (∼50% in mineral oil) (0.10 g; 2.11 mmole) in dimethylformamide (13 ml) was added 1,3,4,5-tetrahydro-4-(4-methoxyphenyl)-3-(2-propenyl)-7-(trifluoromethyl)-2H-1-benzazepin-2-one (660 mg; 1.76 mmole) with stirring. After one hour at 25°C, 1.7N N,N-dimethyl-2-chloroethylamine (in toluene) (5.2 ml; 8.80 mmole) was added, and the mixture was heated to 80°C with stirring overnight. The mixture was quenched with 1N hydrochloric acid, made basic with 50% sodium hydroxide solution, extracted with ethyl acetate (two times) and dried (magnesium sulfate). The concentrated crude mixture weighed 0.77 g. The solid was flash chromatographed (silica gel/1% methanol:dichloromethane, followed by 2% methanol:dichloromethane, followed by 3% methanol:dichloromethane). Appropriate fractions contained the desired cis free amine product, which was collected (rotary evaporated fractions) and dissolved in ether. Some ether insoluble material (presumably silica gel) was filtered off and the filtrate was treated with hydrogen chloride saturated ether. The resulting precipitate was collected by suction-filtration and rinsed with ether. The title compound weighed 190 mg. A mixture of cis/trans product as well as pure trans product were also obtained from the flash chromatography.

| Analysis Calc'd for C₂₅H₂₉F₃N₂O₂·HCl·0.44H₂O: | | | | | |
|---|---|---|---|---|---|
| | C, 61.17; | H, 6.34; | N, 5.71; | Cl, 7.22; | F, 11.61 |
| Found: | C, 61.17; | H, 6.07; | N, 5.58; | Cl, 6.98; | F, 11.32 |

### Method II

### A) α-(2-Propenyl)-[2-(2-nitro-5-trifluoromethylphenyl)-1-(4-methoxyphenyl)ethyl]propanedioic acid, dimethyl ester

[2-(2-Nitro-5-trifluoromethylphenyl)-1-(4-methoxyphenyl)ethyl]propanedioic acid, dimethyl ester (46.54 g; 0.102 mole; see Example 4A) was dissolved in dry dimethylformamide (290 ml) under argon. Fifty percent sodium hydride (5.89 g; 0.123 mole; prewashed-hexane) was added with stirring which was continued for 20 minutes before allyl bromide (44 ml; 61.7 g; 0.510 mmole; d=1.398; 5 eq.) was added dropwise. After six hours, 40 minutes, the reaction was quenched with 1N hydrochloric acid. The solution was extracted with ethyl acetate (two times) and the extract was washed with 1N hydrochloric acid (two times), saturated potassium carbonate (two times), saturated sodium chloride, and dried (magnesium sulfate). The concentrated solution (viscous oil) was flash chromatographed (silica gel/9:1-hexane: ethyl acetate, followed by 8:2-hexane:ethyl acetate) in two portions. After drying in vacuo overnight, the yellow viscous oil product weighed 54.77 g. The product was used "as is" in the next step.

### B) α-(2-Propenyl)-[2-(2-amino-5-trifluoromethylphenyl)-1-(4-methoxyphenyl)ethyl]propanedioic acid, dimethyl ester

α-(2-Propenyl)-[2-(2-nitro-5-trifluoromethylphenyl)-1-(4-methoxyphenyl)ethyl]propanedioic acid, dimethyl ester (50.49 g; 0.102 mole) was dissolved in methanol (350 ml) under argon at room temperature. Powdered stannous chloride dihydrate (119.67 g; 0.53 mole; 5.2 eq.) was added followed by concentrated hydrochloric acid (155 ml) with stirring. Celite®, ethyl acetate, and saturated potassium carbonate solution were added with stirring (the potassium carbonate was added portionwise). The suspension was filtered through a Celite® pad (rinsed three times with ethyl acetate), rotary evaporated, and suction-filtered. The collected white solid was rinsed with 10% methanol:water and dried giving 51.97 g of the title compound.

### C) 1,3,4,5-Tetrahydro-3-(methoxycarbonyl)-4-(4-methoxyphenyl)-3-(2-propenyl)-7-(trifluoromethyl)-2H-1-benzazepin-2-one

α-(2-Propenyl)-[2-(2-amino-5-trifluoromethylphenyl)-1-(4-methoxyphenyl)ethyl]propanedioic acid, dimethyl ester (47.47 g; 0.102 mole) and a 25% (by weight) solution of sodium methoxide in methanol (107 ml; 0.469 mole; d=0.945; 4.6 eq.) in methanol (200 ml) and dry dimethylformamide (200 ml) was refluxed (∼95°C) overnight. 1N Hydrochloric acid was added with stirring, producing a precipitate that was collected by suction-filtration and triturated with water (two times). The solid was slurried in carbon tetrachloride and rotary evaporated followed by vacuum drying to give 42.99 g of crude product.

### D) 1,3,4,5-Tetrahydro-4-(4-methoxyphenyl)-3-(2-propenyl)-7-(trifluoromethyl)-2H-1-benzazepin-2-one

Lithium iodide (13.75 g; 102.6 mmole) was added to 1,3,4,5-tetrahydro-3-(methoxycarbonyl)-4-(4-methoxyphenyl)-3-(2-propenyl)-7-(trifluoromethyl)-2H-1-benzazepin-2-one (42.94 g; 99 mmole) in pyridine (300 ml), a few drops of water were added, and the mixture was refluxed with stirring for two days. Pyridine was vacuum distilled off and the nearly dry residue was dissolved in chloroform and washed with 1N hydrochloric acid (four times), saturated sodium chloride, and dried (magnesium sulfate). The organic solution was concentrated and vacuum dried overnight, yielding 35.12 g of reddish crude product. This material was triturated with methanol leaving 19.87 g of the title compound as a solid.

### E) (cis)-1-[2-(Dimethylamino)ethyl]-1,3,4,5-tetrahydro-4-(4-methoxyphenyl)-3-(2-propenyl)-7-trifluoromethyl)-2H-1-benzazepin-2-one, monohydrochloride

To a slurry of prewashed sodium hydride (320 mg of 60% sodium hydride in mineral oil) in 30 ml of dimethylformamide at 25°C was added 2.5 g of 1,3,4,5-tetrahydro-4-(4-methoxyphenyl)-3-(2-propenyl)-7-(trifluoromethyl)-2H-1-benzazepin-2-one. After stirring at 25°C for one hour, 4.65 ml of a 2.15 M solution of 2-dimethylaminoethyl chloride (9.99 mmole, 1.5 equiv.) in toluene was added. The reaction mixture was stirred for three hours at 80°C, then cooled to 25°C and quenched with 1N hydrochloric acid, basified with 1N sodium hydroxide, and extracted with ethyl acetate. The organic layer was dried (magnesium sulfate) and concentrated, and the residue was flash chromatographed on a prewashed silica gel column. The appropriate fractions were combined, concentrated, and vacuum dried overnight giving 1.92 g of free amine product. This material was dissolved in ether and hydrogen chloride saturated ether was added yielding 2.08 g of the title compound.

| Analysis Calc'd for C₂₅H₂₉F₃N₂O₂·HCl·0.22H₂O: | | | | | |
|---|---|---|---|---|---|
| | C, 61.66; | H, 6.30; | N, 5.75; | Cl, 7.28; | F, 11.70 |
| Found: | C, 61.66; | H, 6.15; | N, 5.73; | Cl, 7.17; | F, 11.46 |

### Example 6

### (cis)-1-[2-(Dimethylamino)ethyl]-1,3,4,5-tetrahydro-4-(4-methoxyphenyl)-3-propyl-7-(trifluoromethyl)-2H-1-benzazepin-2-one, monohydrochloride

### A) 1,3,4,5-Tetrahydro-4-(4-methoxyphenyl)-3-propyl-7-(trifluoromethyl)-2H-1-benzazepin-2-one

1,3,4,5-Tetrahydro-4-(4-methoxyphenyl)-3-(2-propenyl)-7-(trifluoromethyl)-2H-1-benzazepin-2-one (3.50 g; 9.32 mmole; see Example 5, Method II, part D) was dissolved in glacial acetic acid (100 ml) and trifluoroacetic acid (50 ml). Palladium on charcoal (0.71 g) was added to the degassed solution, and the mixture was placed on a Parr apparatus for four hours. The solution was then filtered through a pad of Celite® and the Celite® was rinsed several times with ethyl acetate. The concentrated mixture was vacuum dried yielding 3.53 g of the title compound as a solid.

### B) (cis)-1-[2-(Dimethylamino)ethyl]-1,3,4,5-tetrahydro-4-(4-methoxyphenyl)-3-propyl-7-(trifluoromethyl)-2H-1-benzazepin-2-one, monohydrochloride

Potassium bicarbonate (1.86 g; 18.5 mmole), 1,3,4,5-tetrahydro-4-(4-methoxyphenyl)-3-propyl-7-(trifluoromethyl)-2H-1-benzazepin-2-one (3.50 g; 9.27 mmole), and potassium iodide (catalytic amount) were suspended in methylethyl ketone (55 ml). 2-Dimethylaminoethyl chloride (5.5 ml of 2.15M solution in toluene; 11.9 mmole) was added with stirring, and the mixture was refluxed for one hour. An additional 5.5 ml of 2-dimethylaminoethyl chloride was added and reflux was continued for two hours. Dimethylformamide (10 ml) was added and after an additional four hours of reflux, the solution was rotary evaporated and dimethylformamide was removed using a high vacuum pump. The residue was partitioned between ethyl acetate/water, and the organic layer was dried (magnesium sulfate) and concentrated. The crude free amine of the title compound (4.29 g) was flash chromatographed using 0.1%-2.0% methanol:dichloromethane, yielding 2.28 g of product. This product was converted to the title hydrochloride salt using hydrogen chloride saturated ether; melting point 233.5-235°C.

| Analysis Calc'd for C₂₅H₃₁F₃N₂O₂·HCl: | | | | | |
|---|---|---|---|---|---|
| | C, 61.91; | H, 6.65; | N, 5.78; | Cl, 7.31; | F, 11.75 |
| Found: | C, 61.79; | H, 6.31; | N, 5.73; | Cl, 7.05; | F, 11.71 |

### Example 7

### (cis)-1-[2-(Dimethylamino)ethyl]-1,3,4,5-tetrahydro-4-(4-methoxyphenyl)-3-(2-propenyl)-6-(trifluoromethyl)-2H-1-benzazepin-2-one

### A) [2-(2-Nitro-6-trifluoromethylphenyl)-1-(4-methoxyphenyl)ethyl]propanedioic acid, dimethyl ester

To a dry two liter three neck flask was added 52.7 g (0.21 mol) of p-methoxybenzylidene malonate and 350 ml of dimethylformamide. This solution was stirred (under nitrogen), treated with 11.0 g (0.27 mol) of 60% sodium hydride dispersion and this slurry was treated dropwise with a solution of 43.0 g (0.21 mol) of 2-nitro-6-(trifluoromethyl)toluene in 50 ml of dimethylformamide over a period of 30 minutes while maintaining the temperature at 28-30°C. The mixture was stirred at room temperature for six hours, allowed to stand overnight at room temperature, cooled and treated portionwise with 20 ml of acetic acid. The slurry was poured onto two liters of ice water and extracted with 500 ml of dichioromethane. The aqueous layer was extracted with 250 ml of dichioromethane and then with 100 ml of dichloromethane (two times). The organic phases were combined, extracted with 500 ml of water (three times), dried (magnesium sulfate), filtered and the solvent evaporated to give 99.1 g of a granular solid. This was digested with 150 ml of hot methanol. This suspension was allowed to cool to room temperature, cooled overnight, filtered, washed with cold methanol and dried to give 78.3 g of solid, melting point 117-119°C.

### B) α-(2-Propenyl)-[2-(2-nitro-6-trifluoromethylphenyl)-1-(4-methoxyphenyl)ethyl]propanedioic acid, dimethyl ester

[2-(2-Nitro-6-trifluoromethylphenyl)-1-(4-methoxyphenyl)ethyl]propanedioic acid, dimethyl ester (14.55 g; 31.96 mmole) was dissolved in dry dimethylformamide (90 ml) under argon. Prewashed 50% sodium hydride (1.85 g; 38.5 mmole) was added with stirring and stirring was continued for 20 minutes before allyl bromide (19.33 g; 159.8 mmole; 13.8 ml; d=1.398; 5 eq.) was added dropwise. The mixture was quenched (1N hydrochloric acid) after a few minutes of reaction time, and extracted with ethyl acetate (two times). The organic extract was washed with saturated potassium carbonate (two times), saturated sodium chloride, and dried (magnesium sulfate). The solution was concentrated on a high vacuum pump leaving 23.0 g. This material was flash chromatographed (silica gel/9:1-hexane:ethyl acetate) and the appropriate fractions were combined and concentrated giving 15.45 g of the title compound as an oil.

### C) α-(2-Propenyl)-[2-(2-amino-6-trifluoromethylphenyl)-1-(4-methoxyphenyl)ethyl]propanedioic acid, dimethyl ester

α-(2-Propenyl)-[2-(2-nitro-6-trifluoromethylphenyl)-1-(4-methoxyphenyl)ethyl]propanedioic acid, dimethyl ester (15.42 g; 31.12 mmole) was dissolved in methanol (110 ml) under argon at room temperature. Powdered stannous chloride dihydrate (36.52 g; 161.8 mmol; 5.2 eq.) was added followed by concentrated hydrochloric acid (50 ml) with stirring. After ∼1.5 hours, Celite®, ethyl acetate, and saturated potassium carbonate solution were added with stirring (the potassium carbonate solution was added portionwise). The suspension was filtered, and the solid rinsed with ethyl acetate (three times). The filtrate was concentrated, and the residue was washed with 10% methanol/water. The washings were concentrated and dried in vacuo giving ∼16 g of a yellow oil. The solids (tin salts, Celite®, potassium bicarbonate, etc.) that had been filtered from the reaction mixture were triturated in acetone (several times) and suction filtered through a pad of Celite®. The acetone filtrate was concentrated, and the remaining residue was partitioned between chloroform and water. The chloroform layer was dried (magnesium sulfate) and concentrated, leaving 9.06 g of the title compound as a solid. The remaining product was assumed to be in the aforementioned ∼16 g of yellow oil. The yellow oil was used "as is" in the next step, as was the 9.06 g of solid.

### D) 1,3,4,5-Tetrahydro-3-(methoxycarbonyl)-4-(4-methoxyphenyl)-3-(2-propenyl)-6-(trifluoromethyl)-2H-1-benzazepin-2-one

α-(2-Propenyl)-[2-(2-amino-6-trifluoromethylphenyl)-1-(4-methoxyphenyl)ethyl]propanedioic acid, dimethyl ester (9.03 g; 19.4 mmole) and a 25% (by weight) sodium methoxide in methanol solution (20.5 ml; 89.5 mmole; d=0.945; 4.6 eq.) in methanol (100 ml) and dry dimethylformamide (100 ml) were refluxed (∼95°C for four hours. 1N Hydrochloric acid was added with stirring, producing a precipitate that was collected by suction-filtration, washed with water (three times) and dried in vacuo giving ∼10 g of the title compound as a solid. The "crude" material was used "as is".

### E) 1,3,4,5-Tetrahydro-4-(4-methoxyphenyl)-3-(2-propenyl)-6-(trifluoromethyl)-2H-1-benzazepin-2-one

Lithium iodide (11.98 g; 89.44 mmole) was added to 1,3,4,5-tetrahydro-3-(methoxycarbonyl)-4-(4-methoxyphenyl)-3-(2-propenyl)-6-(trifluoromethyl)-2H-1-benzazepin-2-one (9.69 g; 22.36 mmole) in pyridine (40 ml) and water (a few drops). The mixture was refluxed with stirring for 11 hours. The pyridine was vacuum distilled off and the residue was dissolved into methanol and washed with 1N hydrochloric acid (three times), and saturated sodium chloride, and dried (magnesium sulfate). The concentrated residue was black indicating some decomposition occurred. The 9.67 g of this black residue was flash chromatographed (silica gel/3:1-hexane:ethyl acetate, followed by 1:1-hexane/ethyl acetate). Appropriate fractions were combined and concentrated giving 5.87 g of the title compound as a solid.

### F) (cis)-1-[2-(Dimethylamino)ethyl]-1,3,4,5-tetrahydro-4-(4-methoxyphenyl)-3-(2-propenyl)-6-(trifluoromethyl)-2H-1-benzazepin-2-one

To 1,3,4,5-tetrahydro-4-(4-methoxyphenyl)-3-(2-propenyl)-6-(trifluoromethyl)-2H-1-benzazepin-2-one (3.40 g; 9.05 mmole), potassium bicarbonate (1.81 g; 18.1 mmole), and potassium iodide (catalytic amount) suspended in methylethyl ketone (55 ml) was added a 2.15M solution of 2-dimethylaminoethyl chloride (5.1 ml; 10.9 mmole) in toluene, with stirring. After refluxing for ∼30 minutes, an additional 5.1 ml of the amine was added, and an additional 1.81 g of potassium bicarbonate was added after 1.5 hours of reflux. After six hours of reflux, the mixture was rotary evaporated and the residue was dissolved into ethyl acetate, and washed with water. The organic layer was dried (magnesium sulfate) and concentrated leaving 4.07 g of viscous, oily free amine of the title compound. This material was flash chromatographed (silica gel/0.5%-3% gradient methanol:dichloromethane) and the cis containing fractions were combined and concentrated, dissolved in ether, washed with 1N sodium bicarbonate, dried (magnesium sulfate), and acidified with hydrogen chloride saturated ether. The mixture was concentrated and the white solid triturated in ether. The solid product was collected by suction-filtration, yielding 1.39 g of the title compound; melting point 226-228°C.

| Analysis Calc'd. for C₂₅H₃₀N₂ClF₃O₂·0.23H₂O: | | | | | |
|---|---|---|---|---|---|
| | C, 61.63; | H, 6.30; | N, 5.75; | F, 11.70; | Cl, 7.28 |
| Found: | C, 61.63; | H, 6.26; | N, 5.62; | F, 11.60; | Cl, 7.53 |

### Example 8

### (cis)-1-[2-(Dimethylamino)ethyl]-1,3,4,5-tetrahydro-4-(4-methoxyphenyl)-3-propyl-6-(trifluoromethyl)-2H-1-benzazepin-2-one, monohydrochloride

### A) 1,3,4,5-Tetrahydro-4-(4-methoxyphenyl)-3-propyl-6-(trifluoromethyl)-2H-1-benzazepin-2-one

(cis)-1-[2-(Dimethylamino)ethyl]-1,3,4,5-tetrahydro-4-(4-methoxyphenyl)-3-(2-propenyl)-6-(trifluoromethyl)-2H-1-benzazepin-2-one (4.25 g; 11.3 mmole) was dissolved in ethyl acetate (65 ml). Palladium on charcoal (0.86 g) was added to the degassed solution, and the mixture was placed on a Parr apparatus for four hours. The material was filtered through a pad of Celite® and concentrated giving 4.3 g of crystalline solid.

### B) (cis)-1-[2-(Dimethylamino)ethyl]-1,3,4,5-tetrahydro-4-(4-methoxyphenyl)-3-propyl-6-(trifluoromethyl)-2H-1-benzazepin-2-one, monohydrochloride

To 1,3,4,5-tetrahydro-4-(4-methoxyphenyl)-3-propyl-6-(trifluoromethyl)-2H-1-benzazepin-2-one (4.23 g; 11.21 mmole), potassium bicarbonate (4.49 g; 44.8 mmole; 4 eq.), and potassium iodide (catalytic amount) suspended in methylethyl ketone (70 ml) was added a 2.15M solution of 2-dimethylaminoethyl chloride (12.6 ml; 27.0 mmole; 2.4 eq.) in toluene with stirring. The mixture was refluxed for 10.5 hours, then concentrated. The residue as dissolved into ethyl acetate and washed with water. The organic layer was dried (magnesium sulfate) and concentrated. The residue was flash chromatographed (silica gel column/0.5% methanol:dichloromethane followed by 2.0% methanol: dichloromethane) and appropriate fractions were combined and concentrated. The residue was dissolved in ether and hydrogen chloride saturated ether was added. The mixture was concentrated, and coevaporated with ether several times. The solid was partitioned between ether and 1N sodium bicarbonate. The ether layer was dried (magnesium sulfate) and concentrated. After standing overnight, the solid residue was much less soluble in ether. The solid was triturated in ether and the mixture was centrifuged and a solid was obtained. Conversion to the hydrochloride salt using hydrogen chloride saturated ether yielded the title compound, melting point 180.5-182.5°C.

| Analysis Calc'd. for C₂₅H₃₁F₃N₂O₂·HCl·0.4H₂O: | | | | | |
|---|---|---|---|---|---|
| | C, 60.94; | H, 6.71; | N, 5.69; | Cl, 7.20; | F, 11.57 |
| Found: | C, 60.94; | H, 6.58; | N, 5.65; | Cl, 7.38; | F, 11.32 |

### Example 9

### (d-cis)-1-[2-(Dimethylamino)ethyl]-1,3,4,5-tetrahydro-4-(4-methoxyphenyl)-3-(2-propenyl)-6-(trifluoromethyl)-2H-1-benzazepin-2-one, monohydrochloride

### A) [2-(2-Amino-6-trifluoromethylphenyl)-1-(4-methoxyphenyl)ethyl]propanedioic acid, dimethyl ester

A suspension of 40.4 g (0.088 mol) of [2-(2-nitro-6-trifluoromethylphenyl)-1-(4-methoxyphenyl)ethyl]propanedioic acid, dimethyl ester (see Example 7A) in methanol was treated with a cold suspension of 5% palladium on charcoal in methanol (under nitrogen) and placed on the Parr apparatus at 58 psi of hydrogen. The mixture was heated at 50-55°C for 1 hour to assure that all of the starting material had dissolved. The mixture was removed from the Parr apparatus and allowed to stand at room temperature overnight. The flask was heated to dissolve the crystallized product, and the hot solution was filtered through Celite® (under nitrogen) and washed with hot methanol. The colorless filtrate was concentrated on a rotary evaporator to yield 36.9 g of the title compound, melting point 111-113°C. A sample crystallized from methanol melted at 112-114°C.

### B) 1,3,4,5-Tetrahydro-3-(methoxycarbonyl)-4-(4-methoxyphenyl)-6-(trifluoromethyl)-2H-1-benzazepin-2-one

To a dry two liter three-neck flask was added 34.5 g (0.081 mol) of [2-(2-amino-6-trifluoromethylphenyl)-1-(4-methoxyphenyl)ethyl]-propanedioic acid, dimethyl ester and 350 ml of methanol. The suspension was heated to 45°C and the resulting solution was cooled to 30°C and treated with 23 ml of a 25% solution of sodium methoxide in methanol. This mixture was heated and refluxed for 1 hour. The slurry was cooled to 15°C and treated with a solution of 30 ml of 6N hydrochloric acid in 350 ml of water. After stirring in an ice bath for 2 hours, the pale gray solid was filtered and dried; yield 30.8 g, melting point 214-216°C. A sample crystallized from methanol melted at 218-220°C.

### C) 3-Carboxy-1,3,4,5-tetrahydro-4-(4-methoxyphenyl)-6-(trifluoromethyl)-2H-1-benzazepin-2-one

To a stirred warm solution of 58.0 g (0.88 mol) of potassium hydroxide (85%) in 500 ml of methanol was added portionwise 81.7 g (0.21 mol) of 1,3,4,5-tetrahydro-3-(methoxycarbonyl)-4-(4-methoxyphenyl)-6-(trifluoromethyl)-2H-1-benzazepin-2-one - most of the solid dissolved. The mixture was diluted with 100 ml of dioxane and the resulting solution was refluxed for 6 hours. After standing overnight at room temperature, about 50% of the solvent was removed on a rotary evaporator and the residue was diluted with 4 liters of cold water. The insoluble material was filtered and dried (10 g) and the filtrate was cooled and treated portionwise with 270 ml of acetic acid to give a colorless granular solid. The latter was filtered, washed with cold water and dried in a desiccator; yield 69.0 g, melting point 179-181°C (s. 128°C).

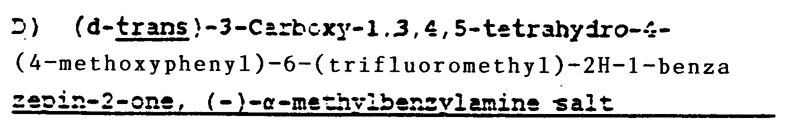

A mixture of 67.0 g (0.176 mol) of 3-carboxy-1,3,4,5-tetrahydro-4-(4-methoxyphenyl)-6-(trifluoromethyl)-2H-1-benzazepin-2-one and 1 liter of ethanol was warmed and the resulting solution (52°C) was treated with a solution of 21.4 g (0.176 mol) of (-)-α-methylbenzylamine in 100 ml of ethanol. This solution was seeded and allowed to stand undisturbed for 24 hours at room temperature. The product separated as well-formed crystals on the walls of the flask. The mother liquor was decanted from the solid and the latter was suspended in 70 ml of ethanol, filtered and washed with fresh ethanol to give 34.6 g of a colorless solid, melting point 156°C (dec.); [α]_{D}-10.3° (c, 1% methanol).

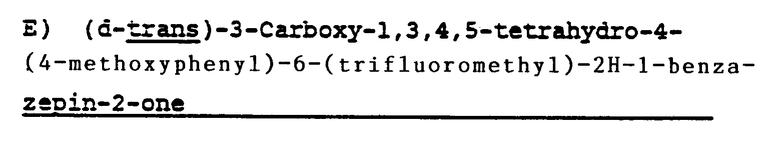

(d-trans)-3-Carboxy-1,3,4,5-tetrahydro-4-(4-methoxyphenyl)-6-(trifluoromethyl)-2H-1-benzazepin-2-one, (-)-α-methylbenzylamine salt (34.0 g, 67.9 mmole) was stirred in dichloromethane (780 ml) and water (390 ml), and treated with 1N hydrochloric acid (78 ml). Methanol (195 ml) was added in increments to expedite solvation. After 2 clear layers were obtained (15-20 minutes), the organic layer was separated. The aqueous layer was extracted with dichloromethane (twice) and the combined organic layers were washed with water (200 ml). The organic layer was dried (magnesium sulfate) and concentrated. The residue was co-evaporated with acetone (three times). Crude yield = 27.1 g.

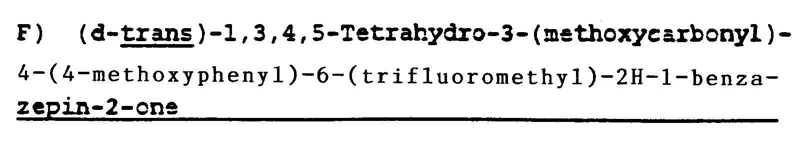

(d-trans)-3-Carboxy-1,3,4,5-tetrahydro-4-(4-methoxyphenyl)-6-(trifluoromethyl)-2H-1-benzazepin-2-one (25.75 g; 67.87 mmole) was dissolved in acetone (200 ml), and 1,8-diazabicyclo[5.4.0]undec-7-ene (10.54 g; 10.4 ml; 69.22 mmole; 1.01 eq; d=1.018) was added with stirring at room temperature. (After less than one minute, a white precipitate formed). Methyl iodide (43 ml; 96.3 g; sp. gr. 2.24-2.26; 678.7 mmole; 10 eq) was added and the mixture was heated at ∼45°C. (After ∼1 minute, the solution turned homogeneous and yellow). The mixture was allowed to stir for 15 minutes before concentrating it and partitioning the residue between chloroform and saturated potassium bisulfate. The aqueous phase was extracted with chloroform (four times), and the combined organics were dried (magnesium sulfate) and concentrated giving 51.74 g of a yellow viscous oil. A slightly yellow solid was obtained by co-evaporating the residue with ether. This material was preabsorbed onto 60-200 mesh silica gel and layered onto a silica gel packed (10 cm high) column. 1:1 Hexane:ethyl acetate was used to wash the product out of the column leaving baseline contamination at the origin. The filtrate was dried (magnesium sulfate), concentrated,co-evaporated with ether (twice) and vacuum dried yielding 24.78 g of the title compound.

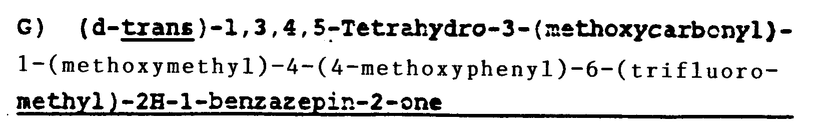

Following the procedure of Example 5, Method I, part C, but utilizing (d-trans)-1,3,4,5-tetrahydro-3-(methoxycarbonyl)-4-(4-methoxyphenyl)-6-(trifluoromethyl)-2H-1-benzazepin-2-one (10.87 g; 27.63 mmole), freshly distilled methoxymethyl bromide (2.5 ml; 3.8 g; 30.39 mmole; d=1.531; 1.1 eq.), prewashed (ether) sodium hydride (0.86 g; 35.92 mmole; 1.3 eq.), and dimethylformamide (110 ml) yielded the crude title compound (15.83 g). The crude material was flashed using a gradient of 10% ethyl acetate/hexane to 20% ethyl acetate/hexane. The yield of pure product was 4.23 g. An additional 4.44 g of starting material product mixture, and 1.98 g of pure recovered starting material were also obtained.

### H) (d)-1,3,4,5-Tetrahydro-3-(methoxycarbonyl)-1-(methoxymethyl)-4-(4-methoxyphenyl)-3-(2-propenyl)-6-(trifluoromethyl)-2H-1-benzazepin-2-one

Following the procedure of Example 5, Method I, part D, but utilizing (d-trans)-1,3,4,5-tetrahydro-3-(methoxycarbonyl)-1-(methoxymethyl)-4-(4-methoxyphenyl)-6-(trifluoromethyl)-2H-1-benzazepin-2-one (10.39 g; 23.7 mmole), allyl bromide (4.1 ml; 5.75 g; 47.5 mmole; d=1.398; 2 eq.), 50% sodium hydride (2.28 g; 47.5 mmole; 2 eq.) and dry dimethylformamide (110 ml) yielded the crude title compound (17.84 g) as a yellow oil. The oil solidified on coevaporation with ether.

### I) (d)-1,3 4,5-Tetrahydro-3-(methoxycarbonyl)-4-(4-methoxyphenyl)-3-(2-propenyl)-6-(trifluoromethyl)-2H-1-benzazepin-2-one

Following the procedure of Example 5, Method I, part E, but utilizing (d)-1,3,4,5-tetrahydro-3-(methoxycarbonyl)-1-(methoxymethyl)-4-(4-methoxyphenyl)-3-(2-propenyl)-6-(trifluoromethyl)-2H-1-benzazepin-2-one (11.27 g; 23.62 mmole), methanol (240 ml), sulfuric acid (40 ml), dry lithium bromide (8.65 g; 99.6 mmole) yielded the crude title compound as a yellow oily residue. The residue was dissolved in ethyl acetate and suction filtered through a pad of silica gel. The clear, yellow filtrate was concentrated forming a light yellow semi-solid.

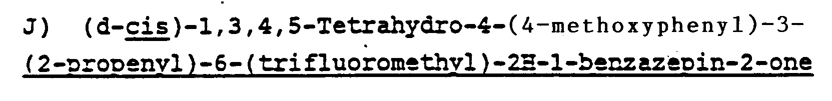

Following the procedure of Example 5, Method I, part F, but utilizing (d)-1,3,4,5-tetrahydro-3-(methoxycarbonyl)-4-(4-methoxyphenyl)-3-(2-propenyl)-6-(trifluoromethyl)-2H-1-benzazepin-2-one (10.20 g; 23.53 mmole), lithium iodide (12.60 g; 94.12 mmole; 4 eq.), pyridine (210 ml) and water (4 ml) yielded 8.95 g of the crude title compound.

### K) (cis)-1-[2-(Dimethylamino)ethyl]-1,3,4,5-tetrahydro-4-(4-methoxyphenyl)-3-propyl-6-(trifluoromethyl)-2H-1-benzazepin-2-one, monohydrochloride

Following the procedure of Example 5, Method I, part G, but utilizing (d-cis)-1,3,4,5-tetrahydro-4-(4-methoxyphenyl)-3-(2-propenyl)-6-(trifluoromethyl)-2H-1-benzazepin-2-one (8.79 g; 23.42 mmole), potassium iodide (catalytic amount), potassium bicarbonate (9.47 g; 93.68 mmole), methylethyl ketone (105 ml), 2.15M N,N-dimethyl-2-chloroethylamine in toluene (27.2 ml; 58.55 mmole) yielded the crude title compound. Recrystallization (hexane) of the crude product followed by hexane trituration of the precipitate gave pure cis material which was dissolved in ether and treated with hydrochloric acid. The resulting hydrochloride salt was recrystallized from ethyl acetate to yield 3.53 g of the title compound, melting point 225-227°C (dec.), [α]_{D}+96.7°

| Analysis Calc'd. for C₂₅H₂₉F₃N₂O₂·HCl·1.8H₂O: | | | | | |
|---|---|---|---|---|---|
| | C, 58.28; | H, 6.57; | N, 5.43; | Cl, 6.88; | F, 11.06 |
| Found: | C, 58.25; | H, 6.15; | N, 5.39; | Cl, 6.70; | F, 11.00 |

### Example 10

### (d-cis)-1-[2-(Dimethylamino)ethyl]-1,3,4,5-tetrahydro-4-(4-methoxyphenyl)-3-ethyl-6-(trifluoromethyl)-2H-1-benzazepin-2-one, monohydrochloride

### A) (d)-1,3,4,5-Tetrahydro-3-(methoxycarbonyl)-1-(methoxymethyl)-4-(4-methoxyphenyl)-3-ethyl-6-(trifluoromethyl)-2H-1-benzazepin-2-one

To a suspension of 1.2 g of sodium hydride (25 mmole, 50% oil dispersion which was prewashed with ether) in 50 ml of dimethylformamide, cooled at 0-5°C in an ice water bath was added with stirring 5.2 g crude solid (d-trans)-1,3,4,5-tetrahydro-3-(methoxycarbonyl)-1-(methoxymethyl)-4-(4-methoxyphenyl)-6-(trifluoromethyl)-2H-1-benzazepin-2-one (see Example 9G) in small portions. The reaction mixture was stirred at 0-5° for 20 minutes, whereupon 2 ml of iodoethane was added dropwise (25 mmole, 2 equivalents). The reaction mixture was allowed to stand at 0°C to room temperature for 2 hours, whereupon excess hydride was destroyed by the careful addition of water. The reaction mixture was diluted with ether and washed with water. The combined aqueous layer was extracted once with ether. The ether layer was dried over anhydrous magnesium sulfate and concentrated under reduced pressure. The crude yellow residue was chromatographed on a silica gel column and eluted with 5-20% ethyl acetate in hexane to obtain 5.01 g of the title compound as an oil.

### B) (d)-1,3,4,5-Tetrahydro-3-(methoxycarbonyl)-4-(4-methoxyphenyl)-3-ethyl-6-(trifluoromethyl)-2H-1-benzazepin-2-one

To a solution of 4.85 g (10.7 mmole) of (d)-1,3,4,5-tetrahydro-3-(methoxycarbonyl)-1-(methoxymethyl)-4-(4-methoxyphenyl)-3-ethyl-6-(trifluoromethyl)-2H-1-benzazepin-2-one in 100 ml of methanol, cooled in an ice water bath was added dropwise 20 ml of concentrated sulfuric acid. To this solution was added 2.7 g of anhydrous lithium bromide. The cooling bath was removed and the reaction mixture was heated under reflux (bath temperature 80°C). Heating was continued for 2½ hours, whereupon the reaction mixture was cooled (ice water bath) and diluted with water. A white precipitate was observed. Acid was carefully neutralized by the addition of solid sodium bicarbonate. The reaction mixture was extracted thoroughly with ethyl acetate (4 times). The combined ethyl acetate extract was washed with saturated brine solution, dried over anhydrous magnesium sulfate and concentrated under reduced pressure. The crude residue was chromatographed on a silica gel column and eluted with 20-50% ethyl acetate in hexane to obtain 4.36 g of white crystalline product; melting point 77-81°C.

### C) (d-cis)-1,3,4,5-Tetrarydro-4-(4-methoxyphenyl)-3-ethyl-6-(trifluoromethyl)-2H-1-benzazepin-1-one

Lithium iodide (3.96 g, 29.6 mmole) was added to (d)-1,3,4,5-tetrahydro-3-(methoxycarbonyl)-4-(4-methoxyphenyl)-3-ethyl-6-(trifluoromethyl)-2H-1-benzazepin-2-one (3.12 g, 7.4 mmole) in 40 ml of pyridine and 3 ml of water, and the mixture was refluxed at 133°C for 4½ hours. Additional lithium iodide (1.00 g ) and dimethylformamide (10 ml) were added and the reflux was continued overnight. The mixture was cooled and partitioned between ether and 1N hydrochloric acid. The aqueous layer was extracted several times with ether, and the combined organic layers were dried (magnesium sulfate) and concentrated. The crude, dark oily residue was flash chromatographed (silica gel; 1%-10% pyridine:hexane) giving 2.65 g of crude (d-cis)-1,3,4,5-tetrahydro-4-(methoxyphenyl)-3-ethyl-6-(trifluoromethyl)-2H-1-benzazepin-1-one (an approximately 80:20-cis:trans mixture was obtained).

### D) (d-cis)-1-[2-(Dimethylamino)ethyl]-1,3,4,5-tetrahydro-4-(4-methoxyphenyl)-3-ethyl-6-(trifluoromethyl)-2H-1-benzazepin-2-one, monohydrochloride

Prewashed (hexane) sodium hydride (0.26 g, 10.7 mmole) and (d-cis)-1,3,4,5-tetrahydro-4-(4-methoxyphenyl)-3-ethyl-6-(trifluoromethyl)-2H-1-benzazepin-1-one (2.60 g, 7.15 mmole) were stirred in 50 ml of dry dimethylformamide for 35 minutes. A 2.15M toluene solution of N N-dimethyl-2-chloroethylamine (13.3 ml, 28.6 mmole) was then added, and the mixture was heated at 80°C for 1½ hours with stirring. The reaction was quenched with 1N hydrochloric acid, made basic with 50% sodium hydroxide solution, and partitioned between ethyl acetate/water. The aqueous layer was extracted with ethyl acetate, and the combined organic layers were dried (magnesium sulfate) and concentrated. The free amine was recrystallized from hot hexane (3 times), dissolved into ethyl acetate and washed with 1N sodium bicarbonate. The ethyl acetate layer was dried (magnesium sulfate) and then treated with saturated hydrochloric acid in ether. The concentrated mixture was vacuum dried overnight giving 0.52 g of white solid product, melting point 162-164°C, [α]_{D}+112° (The mother liquor contained additional product).

| Analysis Calc'd. for C₂₄H₂₉N₂O₂F₃·HCl·0.95H₂O: | | | | | |
|---|---|---|---|---|---|
| | C, 59.07; | H, 6.59; | N, 5.74; | F, 11.67; | Cl, 7.26 |
| Found: | C, 59.07; | H, 6.71; | N, 5.76; | F, 11.89; | Cl, 7.51 |

### Example 11

### (d-cis)-1-[2-(Dimethylamino)ethyl]-1,3,4,5-tetrahydro-4-(4-methoxyphenyl)-3-methyl-6-(trifluoromethyl)-2H-1-benzazepin-2-one, monohydrochloride

### A) (d)-1,3,4,5-Tetrahydro-3-(methoxycarbonyl)-1-(methoxymethyl)-4-(4-methoxyphenyl)-3-methyl-6-(trifluoromethyl)-2H-1-benzazepin-2-one

To a suspension of 1.2 g of sodium hydride (25 mmole) in 50 ml of dry dimethylformamide, cooled in an ice water bath was added with stirring 5.2 g of (d-trans)-1,3,4,5-tetrahydro-3-(methoxycarbonyl)-1-(methoxymethyl)-4- (4-methoxyphenyl)-6-(trifluoromethyl)-2H-1-benzazepin-2-one (12 mmole, crude; see Example 9G) in small portions. After stirring for 20 minutes at 0-5°C, 1.6 ml of iodomethane (25 mmole) was added rapidly. The reaction mixture was allowed to stand at 0°C to room temperature for 3 hours, whereupon excess hydride was destroyed by careful addition of water. The reaction mixture was diluted with ether and washed with water. The aqueous layer was extracted with ether. The combined ether extract was dried over anhydrous magnesium sulfate and concentrated under reduced pressure. The crude yellow oil was chromatographed on a silica gel column and eluted with 5-20% ethyl acetate in hexane to obtain 4.68 g of the title compound.

### B) (d)-1,3,4,5-Tetrahydro-3-(methoxycarbonyl)-4-(4-methoxyphenyl)-3-methyl-6-(trifluoromethyl)-2H-1-benzazepin-2-one

To a solution of 4.37 g of (d)-1,3,4,5-tetrahydro-3-(methoxycarbonyl)-1-(methoxymethyl)-4-(4-methoxyphenyl)-3-methyl-6-(trifluoromethyl)-2H-1-benzazepin-2-one (9.7 mmole) in 100 ml of methanol, cooled in an ice water bath was added 2.7 g of anhydrous lithium bromide, followed by 20 ml of concentrated sulfuric acid dropwise. The cooling bath was removed and the reaction mixture was heated under reflux (bath temperature 80-85°C) for 4 hours. The reaction mixture was cooled and diluted with ice cold water. Acid was neutralized by the careful addition of solid sodium bicarbonate followed by extraction with ethyl acetate. The combined organic extract was washed once with brine and dried over anhydrous magnesium sulfate. Concentration under reduced pressure left a yellow oily residue which was chromatographed on a silica gel column. Elution with 5-25% ethyl acetate in hexane afforded 1.6 g of the desired white crystalline product, melting point 71-76°C.

### C) (d-cis)-1,3,4,5-Tetrahydro-4-(4-methoxyphenyl)-3-methyl-6-(trifluoromethyl-2H-1-benzazepin-2-one

To a solution of 1.5 g (d)-1,3,4,5-tetrahydro-3-(methoxycarbonyl)-4-(4-methoxyphenyl)-3-methyl-6-(trifluoromethyl)-2H-1-benzazepin-2-one (4 mmole) in 12 ml of dimethylformamlde was added with stirring 2 g of anhydrous lithium bromide and 920 mg of p-aminothiophenol (∼8 mmole). The reaction mixture was placed on an oil bath and heated to 135°C under an argon atmosphere for 5 hours. It was cooled and diluted with ice water. The reaction mixture was extracted with ether (3 times) and the combined ether extract was washed with water, 1N aqueous hydrochloric acid solution (2 times) and finally with saturated sodium chloride solution. The ether layer was dried over anhydrous magnesium sulfate and concentrated under reduced pressure to obtain 1.3 g of a light yellow viscous oil. NMR spectrum of the crude product indicated the cis/trans ratio to be approximately 20:1.

### D) (d-cis)-1-[2-(Dimethylamino)ethyl]-1,3,4,5-tetrahydro-4-(4-methoxyphenyl)-3-methyl-6-(trifluoromethyl)-2H-1-benzazepin-2-one, monohydrochloride

To a slurry of washed (ether) sodium hydride (0.13 g; 5.58 mmole) in dry dimethylformamide (15 ml) was added (d-cis)-1,3,4,5-tetrahydro-4-(4-methoxyphenyl)-3-methyl-6-(trifluoromethyl)-2H-1-benzazepin-2-one (1.27 g; 3.64 mmole). The mixture was stirred at room temperature for 35 minutes before a 2.15M toluene solution of N,N-dimethyl-2-chloroethylamine (5.2 ml; 11.2 mmole) was added, and the solution was heated at 80°C for 6 hours. The reaction was quenched with 1N hydrochloric acid, made basic with 50% sodium hydroxide solution, extracted with ethyl acetate (3 times), and dried over magnesium sulfate. The concentrated residue was coevaporated with hexane (3 times) and placed under vacuum overnight. The crude material was purified by preparative plate chromatography (silica gel, 5% methanol:dichloromethane developed 3 times), dissolved in ethyl acetate, washed with 1N sodium bicarbonate, and dried (magnesium sulfate). The solution was treated with ethereal hydrochloric acid solution, concentrated, coevaporated with ether (4 times), and dried in vacuo giving 870 mg of white solid product, melting point 146-148°C, [α]_{D}+100.4° (methanol).

| Analysis Calc'd. for C₂₃H₂₇N₂O₂F₃·HCl·1.91H₂O: | | | | | |
|---|---|---|---|---|---|
| | C, 56.23; | H, 6.53; | N, 5.70; | Cl, 11.60; | F, 7.22 |
| Found: | C, 56.44; | H, 6.69; | N, 5.83; | Cl, 11.62; | F, 7.04 |

Additional compounds falling within the scope of this invention are:
(cis)-7-Chloro-1-[2-(dimethylamino)ethyl]-1,3,4,5-tetrahydro-4-(4-methoxyphenyl)-3-isopropyl-2H-1-benzazepin-2-one
(cis)-7-Chloro-1-[2-(dimethylamino)ethyl]-1,3,4,5-tetrahydro-4-(4-methoxyphenyl)-3-butyl-2H-1-benzazepin-2-one
(cis)-7-Chloro-1-[2-(dimethylamino)ethyl]-1,3,4,5-tetrahydro-4-(4-methoxyphenyl)-3-isobutyl-2H-1-benzazepin-2-one
(cis)-7-Chloro-1-[2-(dimethylamino)ethyl]-1,3,4,5-tetrahydro-4-(4-methoxyphenyl)-3-cyclohexylmethyl-2H-1-benzazepin-2-one
(cis)-7-Chloro-1-[2-(dimethylamino)ethyl]-1,3,4,5-tetrahydro-4-(4-methoxyphenyl)-3-cyclopentylmethyl-2H-1-benzazepin-2-one
(cis)-7-Chloro-1-[2-(dimethylamino)ethyl]-1,3,4,5-tetrahydro-4-(4-methoxyphenyl)-3-phenylmethyl-2H-1-benzazepin-2-one
(cis)-7-Chloro-1-[2-(dimethylamino)ethyl]-1,3,4,5-tetrahydro-4-(4-methoxyphenyl)-3-allyl-2H-1-benzazepin-2-one
(cis)-7-Chloro-1-[2-(dimethylamino)ethyl]-1,3,4,5-tetrahydro-4-(4-methoxyphenyl)-3-(2-butenyl)-2H-1-benzazepin-2-one
(cis)-7-Chloro-1-[2-(dimethylamino)ethyl]-1,3,4,5-tetrahydro-4-(4-methoxyphenyl)-3-(2-propynyl)-2H-1-benzazepin-2-one
(cis)-7-Chloro-1-[2-(dimethylamino)ethyl]-1,3,4,5-tetrahydro-4-(4-methoxyphenyl)-3-[(2-pyrrolyl)methyl]-2H-1-benzazepin-2-one
(cis)-7-Methoxy-1-[2-(dimethylamino)ethyl]-1,3,4,5-tetrahydro-4-(4-methoxyphenyl)-3-methyl-2H-1-benzazepin-2-one
(cis)-7-(Trifluoromethyl)-1-[2-(dimethylamino)ethyl]-1,3,4,5-tetrahydro-4-(4-methoxyphenyl)-3-isopropyl-2H-1-benzazepin-2-one
(cis)-7-(Trifluoromethyl)-1-[2-(dimethylamino)ethyl]-1,3,4,5-tetrahydro-4-(4-methoxyphenyl)-3-ethyl-2H-1-benzazepin-2-one
(cis)-6-(Trifluoromethyl)-1-[2-(dimethylamino)ethyl]-1,3,4,5-tetrahydro-4-(4-methoxyphenyl)-3-ethyl-2H-1-benzazepin-2-one
(cis)-7-(Trifluoromethyl)-1-[2-(dimethylamino)ethyl]-1,3,4,5-tetrahydro-4-(4-methoxyphenyl)-3-(phenylmethyl)-2H-1-benzazepin-2-one
(cis)-6-(Trifluoromethyl)-1-[2-(dimethylamino)ethyl]-1,3,4,5-tetrahydro-4-(4-methoxyphenyl)-3-(phenylmethyl)-2H-1-benzazepin-2-one
(cis)-7-(Trifluoromethyl)-1-[2-(dimethylamino)ethyl]-1,3,4,5-tetrahydro-4-(4-methoxyphenyl)-3-(2-butenyl)-2H-1-benzazepin-2-one
(cis)-6-(Trifluoromethyl)-1-[2-(dimethylamino)ethyl]-1,3,4,5-tetrahydro-4-(4-methoxyphenyl)-3-[(2-furanyl)methyl)-2H-1-benzazepin-2-one
(cis)-6-(Trifluoromethyl)-1-[2-(dimethylamino)ethyl]-1,3,4,5-tetrahydro-4-(4-methoxyphenyl)-3-(2-propynyl)-2H-1-benzazepin-2-one
(cis)-7-(Trifluoromethyl)-1-[2-(dimethylamino)ethyl]-1,3,4,5-tetrahydro-4-(4-methoxyphenyl)-3-(2-propynyl)-2H-1-benzazepin-2-one

## Claims (Claims for the following Contracting State(s): BE, CH, DE, FR, GB, IT, LI, LU, NL, SE)

1. A compound having the formula 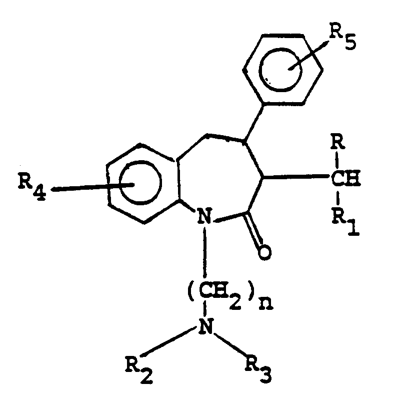 or a pharmaceutically acceptable salt thereof wherein R and R₁ are each hydrogen or C₁₋₁₀ alkyl, R is hydrogen and R₁ is C₁₋₁₀-alkyl, C₂₋₁₀-alkenyl, C₂₋₁₀-alkynyl, aryl, or C₃₋₇-cycloalkyl,
R₂ and R₃ are each independently C₁₋₁₀-alkyl or C₃₋₇-cycloalkyl or R₂ and R₃ together with the nitrogen atom to which they are attached are pyrrolidinyl, piperidinyl, or morpholinyl;
R₄ and R₅ are each independently hydrogen, halogen, C₁₋₁₀-alkyl, C₁₋₁₀-alkoxy, aryloxy, aryl-C₁₋₁₀-alkoxy, aryl-C₁₋₁₀-alkyl, hydroxy, C₂₋₁₁-alkanoyloxy, 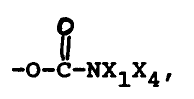 difluoromethoxy, trifluoromethyl, -NX₃X₄, -S(O)ₘC1-10-alkyl, or -S(O)ₘaryl;
n is 2 or 3;
m is 0, 1 or 2;
X₁ and X₂ are each indpendently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl;
with the provisio that if R₄ is a 7-alkyl group, it must have a tertiary carbon atom bonded to the ring;
wherein the term "aryl" refers to phenyl and phenyl substituted with 1, 2 or 3 amino, alkylamino, dialkylamino, nitro, halogen, hydroxyl, trifluoromethyl, alkyl of 1 to 4 carbon atoms, alkoxy of 1 to 4 carbon atoms, alkanoyloxy, carbamoyl, or carboxyl groups;
the term "heteroaryl" refers to pyridinyl, pyrrolyl, imidazolyl, furyl, thienyl, or thiazolyl; and
the term "nitrogen containing heteroaryl" refers to pyridinyl, pyrrolyl, imidazolyl or thiazolyl.

2. A compound in accordance with claim 1 wherein R and R₁ are hydrogen.

3. A compound in accordance with claim 1 wherein R is hydrogen and R₁ is C₁₋₁₀-alkyl or R and R₁ are each C₁₋₁₀-alkyl.

4. A compound in accordance with claim 1 wherein R is hydrogen and R₁ is C₂₋₁₀-alkenyl.

5. A compound in accordance with claim 4 wherein R is hydrogen and R₁ is vinyl.

6. A compound in accordance with claim 1 wherein R is hydrogen and R₁ is C₂₋₁₀-alkynyl.

7. A compound in accordance with claim 1 wherein R is hydrogen and R₁ is aryl.

8. A compound in accordance with claim 1 wherein R is hyrogen and R₁ is C₃₋₇-cycloalkyl.

9. A compound in accordance with claim 1 wherein R₂ and R₃ are each C₁₋₁₀-alkyl.

10. A compound in accordance with claim 9 wherein R₂ and R₃ are each methyl.

11. A compound in accordance with claim 1 wherein R₂ and R₃ are each C₃₋₇-cycloalkyl.

12. A compound in accordance with claim 1 whrein R₂ and R₃ together with the nitrogen atom to which they are attached are pyrrolidinyl, piperidinyl or morpholinyl.

13. A compound in accordance with claim 1 wherein R₄ is hydrogen.

14. A compound in accordance with claim 1 wherein R₄ is halogen or trifluoromethyl.

15. A compound in accordance with claim 14 wherein R₄ is chlorine or trifluoromethyl and is located in the 7-position of the benzazepine nucleus.

16. A compound in accordance with claim 1 wherein R₅ is C₁₋₁₀-alkoxy.

17. A compound in accordance with claim 1 wherein R₅ is methoxy and is located in the 4-position of the phenyl ring to which it is attached.

18. A compound in accordance with claim 1 wherein n is 2.

19. A compound in accordance with claim 1 wherein n is 3.

20. The d-cis enantiomer of a compound of claim 1.

21. The compound in accordance with claim 1, (cis)-7-chloro-1-[2-(dimethylamino)ethyl]-1,3,4,5-tetrahydro-4-(4-methoxyphenyl)-3-methyl-2H-1-benzazepin-2-one or a pharmaceutically acceptable salt thereof.

22. The compound in accordance with claim 1, (cis)-7-chloro-1-[2-(dimethylamino)ethyl]-3-ethyl-1,3,4,5-tetrahydro-4-(4-methoxyphenyl)-2H-1-benzazepin-2-one or a pharmaceutically acceptable salt thereof.

23. The compound in accordance with claim 1, (cis)-7-chloro-1-[2-(dimethylamino)ethyl]-1,3,4,5-tetrahydro-4-(4-methoxyphenyl)-3-(2-propenyl)-2H-1-benzazepin-2-one or a pharmaceutically acceptable salt thereof.

24. The compound in accordance with claim 1, (cis)-1-[2-(dimethylamino)ethyl]-1,3,4,5-tetrahydro-4-4-methoxyphenyl)-3-methyl-7-(trifluoromethyl)-2H-1-benzazepin-2-one or a pharmaceutically acceptable salt thereof.

25. The compound in accordance with claim 1, (cis)-1-[2-(dimethylamino)ethyl]-1,3,4,5-tetrahydro-4-(4-methoxyphenyl)-3-(2-propenyl)-7-(trifluoromethyl)-2H-1-benzazepin-2-one or a pharmaceutically acceptable salt thereof.

26. The compound in accordance with claim 1, (cis)-1-[2-(dimethylamino)ethyl]-1,3,4,5-tetrahydro-4-(4-methoxyphenyl)-3-propyl-7-(trifluoromethyl)-2H-1-benzazepin-2-one or a pharmaceutically acceptable salt thereof.

27. The compound in accordance with claim 1, (cis)-1-[2-(dimethylamino)ethyl]-1,3,4,5-tetrahydro-4-(4-methoxyphenyl)-3-(2-propenyl)-6-(trifluoromethyl)-2H-1-benzazepin-2-one or a pharmaceutically acceptable salt thereof.

28. The compound in accordance with claim 1, (cis)-1-[2-(dimethylamino)ethyl]-1,3,4,5-tetrahydro-4-(4-methoxyphenyl)-3-propyl-6-(trifluoromethyl)-2H-1-benzazepin-2-one or a pharmaceutically acceptable salt thereof.

29. The compound in accordance with claim 20, (d-cis)-1-[2-(dimethylamino)ethyl]-1,3,4,5-tetrahydro-4-(4-methoxyphenyl)-3-(2-propenyl)-6-(trifluoromethyl)-2H-1-benzazepin-2-one or a pharmaceutically acceptable salt thereof.

30. The compound in accordance with claim 20, (d-cis)-1-[2-(dimethylamino)ethyl]-1,3,4,5-tetrahydro-4-(4-methoxyphenyl)-3-ethyl-6-(trifluoromethyl)-2H-1-benzazepin-2-one or a pharmaceutically acceptable salt thereof.

31. The compound in accordance with claim 20, (d-cis)-1-[2-(dimethylamino)ethyl]-1,3,4,5-tetrahydro-4-(4-methoxyphenyl)-3-methyl-6-(trifluoromethyl)-2H-1-benzazepin-2-one or a pharmaceutically acceptable salt thereof.

32. The d-cis enantiomer of a compound having the formula 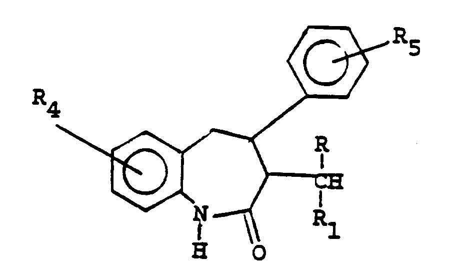 wherein R and R₁ are each hydrogen or C₁₋₁₀ alkyl, R is hydrogen and R₁ is C₁₋₁₀-alkyl-, C₂₋₁₀-alkenyl, C₂₋₁₀-alkynyl, aryl, or C₃ ₇-cycloalkyl,
R₄ and R₅ are each independently hydrogen, halogen, C₁₋₁₀-alkyl, C₂₋₁₀-alkoxy, aryloxy, aryl-C₁₋₁₀-alkoxy, aryl-C₁₋₁₀-alkyl, hydroxy, C₂₋₁₁-alkanoyloxy, 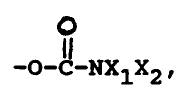 difluoromethoxy, trifluoromethyl, -NX₃X₄, -S(O)ₘC₁₋₁₀-alkyl, or -S(O)ₘaryl;
m is 0, 1 or 2;
X₁ and X₂ are each independently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl.

33. A process for preparing a compound of any of the claims 1 to 31 which comprises reacting a compound having the formula 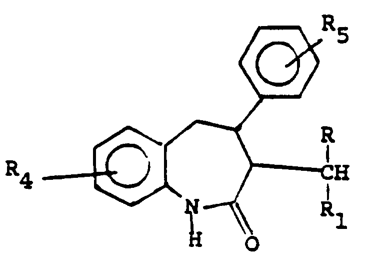 with a compound having the formula
halogen-(CH₂)ₙ-NR₂R₃
and optionally converting the product to a pharmaceutically acceptable salt.

34. A process in accordance with claim 33 wherein the benzazepine reactant and product are each d-cis enantiomers.

35. A process for preparing a compound of claim 32 which comprises the decarboxylation of the d-cis enantiomer of a compound having the formula 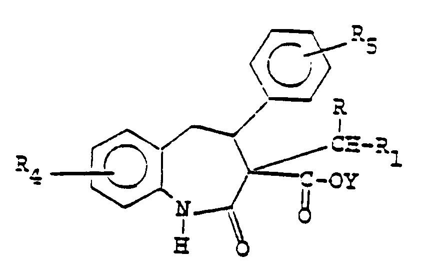 wherein Y is C₁₋₁₀-alkyl.

36. A pharmaceutical preparation containing a compound of any of the claims 1 to 31 and a pharmaceutically acceptable carrier.

## Claims (Claims for the following Contracting State(s): AT)

1. A process for preparing a compound having the formula 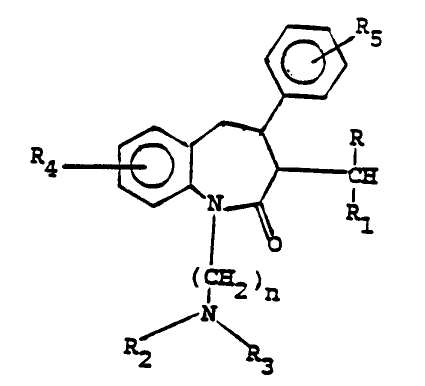 or a pharmaceutically acceptable salt thereof wherein R and R₁ are each hydrogen or C₁₋₁₀ alkyl, R is hydrogen and R₁ is C₁₋₁₀-alkyl, C₂₋₁₀-alkenyl, C₂₋₁₀-alkynyl, aryl, or C₃₋₇-cycloalkyl,
R₂ and R₃ are each independently C₁₋₁₀-alkyl or C₃₋₇-cycloalkyl or R₂ and R₃ together with the nitrogen atom to which they are attached are pyrrolidinyl, piperidinyl, or morpholinyl;
R₄ and R₅ are each independently hydrogen, halogen, C₁₋₁₀-alkyl, C₁₋₁₀-alkoxy, aryloxy, aryl-C₁₋₁₀-alkoxy, aryl-C₁₋₁₀-alkyl, hydroxy, C₂₋₁₁-alkanoyloxy, 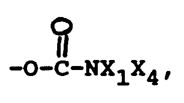 difluoromethoxy, trifluoromethyl, -NX₃X₄, -S(O)ₘC1-10-alkyl, or -S(O)ₘaryl;
n is 2 or 3;
m is 0, 1 or 2;
X₁ and X₂ are each indpendently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl;
with the provisio that if R₄ is a 7-alkyl group, it must have a tertiary carbon atom bonded to the ring;
wherein the term "aryl" refers to phenyl and phenyl substituted with 1, 2 or 3 amino, alkylamino, dialkylamino, nitro, halogen, hydroxyl, trifluoromethyl, alkyl of 1 to 4 carbon atoms, alkoxy of 1 to 4 carbon atoms, alkanoyloxy, carbamoyl, or carboxyl groups;
the term "heteroaryl" refers to pyridinyl, pyrrolyl, imidazolyl, furyl, thienyl, or thiazolyl; and
the term "nitrogen containing heteroaryl" refers to pyridinyl, pyrrolyl, imidazolyl or thiazolyl, which comprises reacting a compound having the formula 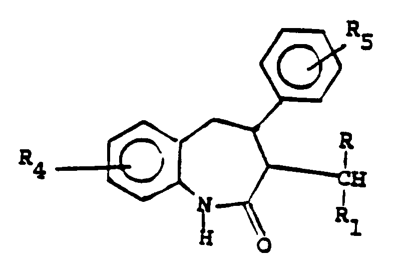 with a compound having the formula
halogen-(CH₂)ₙ-NR₂R₃
and optionally converting the product to a pharmaceutically acceptable salt.

2. A process according to claim 1 for preparing a compound in accordance with claim 1 wherein R and R₁ are hydrogen.

3. A process according to claim 1 for preparing a compound in accordance with claim 1 wherein R is hydrogen and R₁ is C₁₋₁₀-alkyl or R and R₁ are each C₁₋₁₀-alkyl.

4. A process according to claim 1 for preparing a compound in accordance with claim 1 wherein R is hydrogen and R₁ is C₂₋₁₀-alkenyl.

5. A process according to claim 4 for preparing a compound in accordance with claim 4 wherein R is hydrogen and R₁ is vinyl.

6. A process according to claim 1 for preparing a compound in accordance with claim 1 wherein R is hydrogen and R₁ is C₂₋₁₀-alkynyl.

7. A process according to claim 1 for preparing a compound in accordance with claim 1 wherein R is hydrogen and R₁ is aryl.

8. A process according to claim 1 for preparing a compound in accordance with claim 1 wherein R is hyrogen and R₁ is C₃₋₇-cycloalkyl.

9. A process according to claim 1 for preparing a compound in accordance with claim 1 wherein R₂ and R₃ are each C₁₋₁₀-alkyl.

10. A process according to claim 9 for preparing a compound in accordance with claim 9 wherein R₂ and R₃ are each methyl.

11. A process according to claim 1 for preparing a compound in accordance with claim 1 wherein R₂ and R₃ are each C₃₋₇-cycloalkyl.

12. A process according to claim 1 for preparing a compound in accordance with claim 1 whrein R₂ and R₃ together with the nitrogen atom to which they are attached are pyrrolidinyl, piperidinyl or morpholinyl.

13. A process according to claim 1 for preparing a compound in accordance with claim 1 wherein R₄ is hydrogen.

14. A process according to claim 1 for preparing a compound in accordance with claim 1 wherein R₄ is halogen or trifluoromethyl.

15. A process according to claim 14 for preparing a compound in accordance with claim 14 wherein R₄ is chlorine or trifluoromethyl and is located in the 7-position of the benzazepine nucleus.

16. A process according to claim 1 for preparing a compound in accordance with claim 1 wherein R₅ is C₁₋₁₀-alkoxy.

17. A process according to claim 1 for preparing a compound in accordance with claim 1 wherein R₅ is methoxy and is located in the 4-position of the phenyl ring to which it is attached.

18. A process according to claim 1 for preparing a compound in accordance with claim 1 wherein n is 2.

19. A process according to claim 1 for preparing a compound in accordance with claim 1 wherein n is 3.

20. A process according to claim 1 for preparing the d-cis enantiomer of a compound of claim 1.

21. A process according to claim 1 for preparing (cis)-7-chloro-1-[2-(dimethylamino)ethyl]-1,3,4,5-tetrahydro-4-(4-methoxyphenyl)-3-methyl-2H-1-benzazepin-2-one or a pharmaceutically acceptable salt thereof.

22. A process according to claim 1 for preparing (cis)-7-chloro-1-[2-(dimethylamino)ethyl]-3-ethyl-1,3,4,5- tetrahydro-4-(4-methoxyphenyl)-2H-1-benzazepin-2-one or a pharmaceutically acceptable salt thereof.

23. A process according to claim 1 for preparing (cis)-7-chloro-1-[2-(dimethylamino)ethyl]-1,3,4,5-tetrahydro-4-(4-methoxyphenyl)-3-(2-propenyl)-2H-1-benzazepin-2-one or a pharmaceutically acceptable salt thereof.

24. A process according to claim 1 for preparing (cis)-1-[2-(dimethylamino)ethyl]-1,3,4,5-tetrahydro-4-4-methoxy-phenyl)-3-methyl-7-(trifluoromethyl)-2H-1-benzazepin-2-one or a pharmaceutically acceptable salt thereof.

25. A process according to claim 1 for preparing (cis)-1-[2-(dimethylamino)ethyl]-1,3,4,5-tetrahydro-4-(4-methoxy-phenyl)-3-(2-propenyl)-7-(trifluoromethyl)-2H-1-benza-zepin-2-one or a pharmaceutically acceptable salt thereof.

26. A process according to claim 1 for preparing (cis)-1-[2-(dimethylamino)ethyl]-1,3,4,5-tetrahydro-4-(4-methoxy-phenyl)-3-propyl-7-(trifluoromethyl)-2H-1-benzazepin-2-one or a pharmaceutically acceptable salt thereof.

27. A process according to claim 1 for preparing (cis)-1-[2-(dimethylamino)ethyl]-1,3,4,5-tetrahydro-4-(4-methoxy-phenyl)-3-(2-propenyl)-6-(trifluoromethyl)-2H-1-benza-zepin-2-one or a pharmaceutically acceptable salt thereof.

28. A process according to claim 1 for preparing (cis)-1-[2-(dimethylamino)ethyl]-1,3,4,5-tetrahydro-4-(4-methoxy-phenyl)-3-propyl-6-(trifluoromethyl)-2H-1-benzazepin-2-one or a pharmaceutically acceptable salt thereof.

29. A process according to claim 20 for preparing (d-cis)-1-[2-(dimethylamino)ethyl]-1,3,4,5-tetrahydro-4-(4-methoxy-phenyl)-3-(2-propenyl)-6-(trifluoromethyl)-2H-1-benzazepin-2-one or a pharmaceutically acceptable salt thereof.

30. A process according to claim 20 for preparing (d-cis)-1-[2-(dimethylamino)ethyl]-1,3,4,5-tetrahydro-4-(4-methoxy-phenyl)-3-ethyl-6-(trifluoromethyl)-2H-1-benzazepin-2-one or a pharmaceutically acceptable salt thereof.

31. A process according to claim 20 for preparing the (d-cis)-1-[2-(dimethylamino)ethyl]-1,3,4,5-tetrahydro-4-(4-methoxy-phenyl)-3-methyl-6-(trifluoromethyl)-2H-1-benzazepin-2-one or a pharmaceutically acceptable salt thereof.

32. A process according to claim 1 for preparing d-cis enantiomer of a compound having the formula 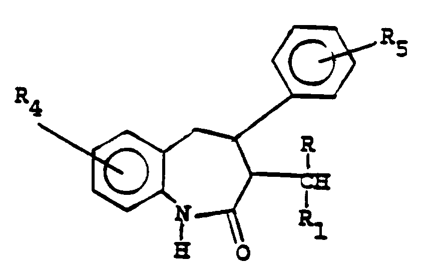 wherein R and R₁ are each hydrogen or alkyl, R is hydrogen and R₁ is C₁₋₁₀-alkyl-, C₂₋₁₀-alkenyl, C₂₋₁₀-alkynyl, aryl, or C₃₋₇-cycloalkyl,
R₄ and R₅ are each independently hydrogen, halogen, C₁₋₁₀-alkyl, C₂₋₁₀-alkoxy, aryloxy, aryl-C₁₋₁₀-alkoxy, aryl-C₁₋₁₀-alkyl, hydroxy, C₂₋₁₁-alkanoyloxy, 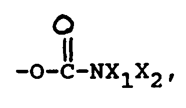 difluoromethoxy, trifluoromethyl, -NX₃X₄, -S(O)ₘC₁₋₁₀-alkyl, or -S(O)ₘaryl;
m is 0, 1 or 2;
X1 and X2 are each independently hydrogen, C1-10-alkyl, aryl or heteroaryl, or X1 and X2 together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl.

33. A process in accordance with claim 1 wherein the benzazepine reactant and product are each d-cis enantiomers.

34. A process for preparing a compound of claim 32 which comprises the decarboxylation of the d-cis enantiomer of a compound having the formula 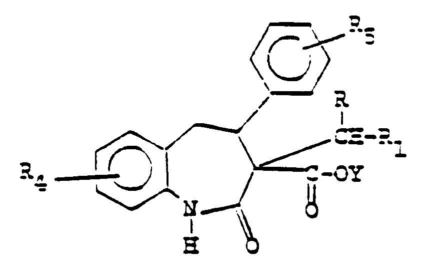 wherein Y is C₁₋₁₀-alkyl.

35. A process for preparing a pharmaceutical preparation which comprises the mixing of a compound obtainable by the process of any of the claims 1-34 and a pharmaceutically acceptable carrier.

## Claims (Claims for the following Contracting State(s): ES)

1. A process for preparing a compound having the formula 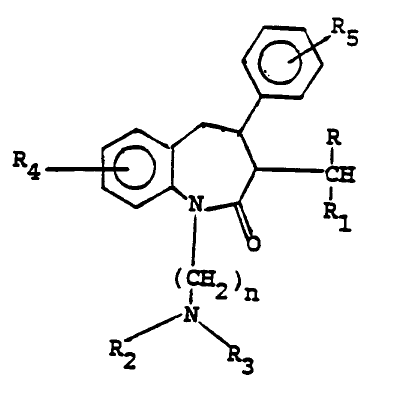 or a pharmaceutically acceptable salt thereof wherein R and R₁ are each hydrogen or C₁₋₁₀ alkyl, R is hydrogen and R₁ is C₁₋₁₀-alkyl, C₂₋₁₀-alkenyl, C₂₋₁₀-alkynyl, aryl, or C₃₋₇-cycloalkyl,
R₂ and R₃ are each independently C₁₋₁₀-alkyl or C₃₋₇-cycloalkyl or R₂ and R₃ together with the nitrogen atom to which they are attached are pyrrolidinyl, piperidinyl, or morpholinyl;
R₄ and R₅ are each independently hydrogen, halogen, C₁₋₁₀-alkyl, C₁₋₁₀-alkoxy, aryloxy, aryl-C₁₋₁₀-alkoxy, aryl-C₁₋₁₀-alkyl, hydroxy, C₂₋₁₁-alkanoyloxy, 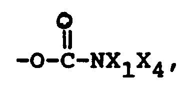 difluoromethoxy, trifluoromethyl, -NX₃X₄, -S(O)ₘC1-10-alkyl, or -S(O)ₘaryl;
n is 2 or 3;
m is 0, 1 or 2;
X₁ and X₂ are each indpendently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl;
with the provisio that if R₄ is a 7-alkyl group, it must have a tertiary carbon atom bonded to the ring;
wherein the term "aryl" refers to phenyl and phenyl substituted with 1, 2 or 3 amino, alkylamino, dialkylamino, nitro, halogen, hydroxyl, trifluoromethyl, alryl of 1 to 4 carbon atoms, alkoxy of 1 to 4 carbon atoms, alkanoyloxy, carbamoyl, or carboxyl groups;
the term "heteroaryl" refers to pyridinyl, pyrrolyl, imidazolyl, furyl, thienyl, or thiazolyl; and
the term "nitrogen containing heteroaryl" refers to pyridinyl, pyrrolyl, imidazolyl or thiazolyl, which comprises a process reacting a compound having the formula 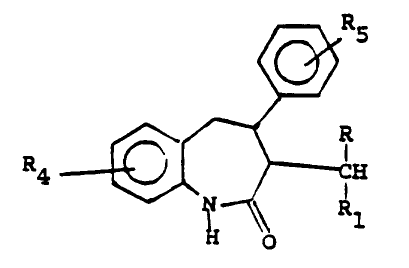 with a compound having the formula
halogen-(CH₂)ₙ-NR₂R₃
and optionally converting the product to a pharmaceutically acceptable salt.

2. A process according to claim 1 for preparing a compound in accordance with claim 1 wherein R and R₁ are hydrogen.

3. A process according to claim 1 for preparing a compound in accordance with claim 1 wherein R is hydrogen and R₁ is C₁₋₁₀-alkyl or R and R₁ are each C₁₋₁₀-alkyl.

4. A process according to claim 1 for preparing a compound in accordance with claim 1 wherein R is hydrogen and R₁ is C₂₋₁₀-alkenyl.

5. A process according to claim 4 for preparing a compound in accordance with claim 4 wherein R is hydrogen and R₁ is vinyl.

6. A process according to claim 1 for preparing a compound in accordance with claim 1 wherein R is hydrogen and R₁ is C₂₋₁₀-alkynyl.

7. A process according to claim 1 for preparing a compound in accordance with claim 1 wherein R is hydrogen and R₁ is aryl.

8. A process according to claim 1 for preparing a compound in accordance with claim 1 wherein R is hyrogen and R₁ is C₃₋₇-cycloalkyl.

9. A process according to claim 1 for preparing a compound in accordance with claim 1 wherein R₂ and R₃ are each C₁₋₁₀-alkyl.

10. A process according to claim 9 for preparing a compound in accordance with claim 9 wherein R₂ and R₃ are each methyl.

11. A process according to claim 1 for preparing a compound in accordance with claim 1 wherein R₂ and R₃ are each C₃₋₇-cycloalkyl.

12. A process according to claim 1 for preparing a compound in accordance with claim 1 whrein R₂ and R₃ together with the nitrogen atom to which they are attached are pyrrolidinyl, piperidinyl or morpholinyl.

13. A process according to claim 1 for preparing a compound in accordance with claim 1 wherein R₄ is hydrogen.

14. A process according to claim 1 for preparing a compound in accordance with claim 1 wherein R₄ is halogen or trifluoromethyl.

15. A process according to claim 14 for preparing a compound in accordance with claim 14 wherein R₄ is chlorine or trifluoromethyl and is located in the 7-position of the benzazepine nucleus.

16. A process according to claim 1 for preparing a compound in accordance with claim 1 wherein R₅ is C₁₋₁₀-alkoxy.

17. A process according to claim 1 for preparing a compound in accordance with claim 1 wherein R₅ is methoxy and is located in the 4-position of the phenyl ring to which it is attached.

18. A process according to claim 1 for preparing a compound in accordance with claim 1 wherein n is 2.

19. A process according to claim 1 for preparing a compound in accordance with claim 1 wherein n is 3.

20. A process according to claim 1 for preparing the d-cis enantiomer of a compound of claim 1.

21. A process according to claim 1 for preparing (cis)-7-chloro-1-[2-(dimethylamino)ethyl]-1,3,4,5-tetrahydro-4-(4-methoxyphenyl)-3-methyl-2H-1-benzazepin-2-one or a pharmaceutically acceptable salt thereof.

22. A process according to claim 1 for preparing (cis)-7-chloro-1-[2-(dimethylamino)ethyl]-3-ethyl-1,3,4,5- tetrahydro-4-(4-methoxyphenyl)-2H-1-benzazepin-2-one or a pharmaceutically acceptable salt thereof.

23. A process according to claim 1 for preparing (cis)-7-chloro-1-[2-(dimethylamino)ethyl]-1,3,4,5-tetrahydro-4-(4-methoxyphenyl)-3-(2-propenyl)-2H-1-benzazepin-2-one or a pharmaceutically acceptable salt thereof.

24. A process according to claim 1 for preparing (cis)-1-[2-(dimethylamino)ethyl]-1,3,4,5-tetrahydro-4-4-methoxy-phenyl)-3-methyl-7-(trifluoromethyl)-2H-1-benzazepin-2-one or a pharmaceutically acceptable salt thereof.

25. A process according to claim 1 for preparing (cis)-1-[2-(dimethylamino)ethyl]-1,3,4,5-tetrahydro-4-(4-methoxy-phenyl)-3-(2-propenyl)-7-(trifluoromethyl)-2H-1-benza-zepin-2-one or a pharmaceutically acceptable salt thereof.

26. A process according to claim 1 for preparing (cis)-1-[2-(dimethylamino)ethyl]-1,3,4,5-tetrahydro-4-(4-methoxy-phenyl)-3-propyl-7-(trifluoromethyl)-2H-1-benzazepin-2-one or a pharmaceutically acceptable salt thereof.

27. A process according to claim 1 for preparing (cis)-1-[2-(dimethylamino)ethyl]-1,3,4,5-tetrahydro-4-(4-methoxy-phenyl)-3-(2-propenyl)-6-(trifluoromethyl)-2H-1-benza-zepin-2-one or a pharmaceutically acceptable salt thereof.

28. A process according to claim 1 for preparing (cis)-1-[2-(dimethylamino)ethyl]-1,3,4,5-tetrahydro-4-(4-methoxy-phenyl)-3-propyl-6-(trifluoromethyl)-2H-1-benzazepin-2-one or a pharmaceutically acceptable salt thereof.

29. A process according to claim 20 for preparing (d-cis)-1-[2-(dimethylamino)ethyl]-1,3,4,5-tetrahydro-4-(4-methoxy-phenyl)-3-(2-propenyl)-6-(trifluoromethyl)-2H-1-benzazepin-2-one or a pharmaceutically acceptable salt thereof.

30. A process according to claim 20 for preparing (d-cis)-1-[2-(dimethylamino)ethyl]-1,3,4,5-tetrahydro-4-(4-methoxy-phenyl)-3-ethyl-6-(trifluoromethyl)-2H-1-benzazepin-2-one or a pharmaceutically acceptable salt thereof.

31. A process according to claim 20 for preparing the (d-cis)-1-[2-(dimethylamino)ethyl]-1,3,4,5-tetrahydro-4-(4-methoxy-phenyl)-3-methyl-6-(trifluoromethyl)-2H-1-benzazepin-2-one or a pharmaceutically acceptable salt thereof.

32. A process according to claim 1 for preparing d-cis enantiomer of a compound having the formula 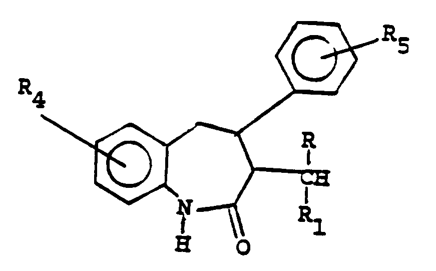 wherein R and R₁ are each hydrogen or alkyl, R is hydrogen and R₁ is C₁₋₁₀-alkyl-, C₂₋₁₀-alkenyl, C₂₋₁₀-alkynyl, aryl, or C₃₋₇-cycloalkyl,
R₄ and R₅ are each independently hydrogen, halogen, C₁₋₁₀-alkyl, C₂₋₁₀-alkoxy, aryloxy, aryl-C₁₋₁₀-alkoxy, aryl-C₁₋₁₀-alkyl, hydroxy, C₂₋₁₁-alkanoyloxy, 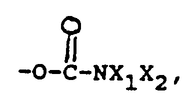 difluoromethoxy, trifluoromethyl, -NX₃X₄, -S(O)ₘC₁₋₁₀-alkyl, or -S(O)ₘaryl;
m is O, 1 or 2;
X1 and X2 are each independently hydrogen, C1-10-alkyl, aryl or heteroaryl, or X1 and X2 together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl.

33. A process in accordance with claim 1 wherein the benzazepine reactant and product are each d-cis enantiomers.

34. A process for preparing a compound of claim 32 which comprises the decarboxylation of the d-cis enantiomer of a compound having the formula 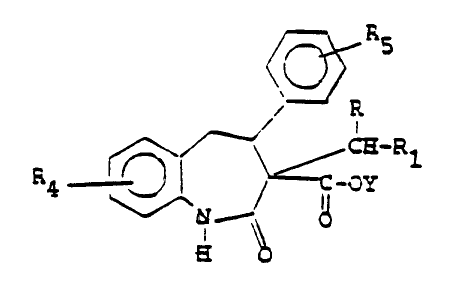 wherein Y is C₁₋₁₀-alkyl.

## Claims (Claims for the following Contracting State(s): GR)

1. A compound having the formula 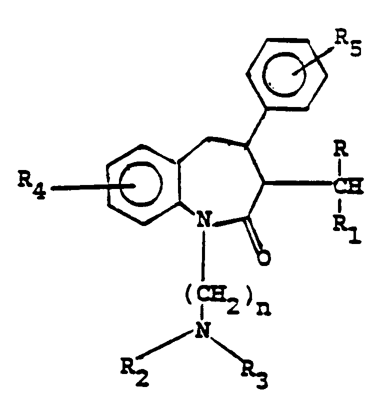 or a pharmaceutically acceptable salt thereof wherein R and R₁ are each hydrogen or C₁₋₁₀ alkyl, R is hydrogen and R₁ is C₁₋₁₀-alkyl, C₂₋₁₀-alkenyl, C₂₋₁₀-alkynyl, aryl, or C₃₋₇-cycloalkyl,
R₂ and R₃ are each independently C₁₋₁₀-alkyl or C₃₋₇-cycloalkyl or R₂ and R₃ together with the nitrogen atom to which they are attached are pyrrolidinyl, piperidinyl, or morpholinyl;
R₄ and R₅ are each independently hydrogen, halogen, C₁₋₁₀-alkyl, C₁₋₁₀-alkoxy, aryloxy, aryl-C₁₋₁₀-alkoxy, aryl-C₁₋₁₀-alkyl, hydroxy, C₂₋₁₁-alkanoyloxy, 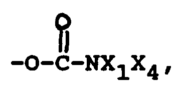 difluoromethoxy, trifluoromethyl, -NX₃X₄, -S(O)ₘC1-10-alkyl, or -S(O)ₘaryl;
n is 2 or 3;
m is 0, 1 or 2;
X₁ and X₂ are each indpendently hydrogen, C₁-₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl;
with the provisio that if R₄ is a 7-alkyl group, it must have a tertiary carbon atom bonded to the ring;
wherein the term "aryl" refers to phenyl and phenyl substituted with 1, 2 or 3 amino, alkylamino, dialkylamino, nitro, halogen, hydroxyl, trifluoromethyl, alkyl of 1 to 4 carbon atoms, alkoxy of 1 to 4 carbon atoms, alkanoyloxy, carbamoyl, or carboxyl groups;
the term "heteroaryl" refers to pyridinyl, pyrrolyl, imidazolyl, furyl, thienyl, or thiazolyl; and
the term "nitrogen containing heteroaryl" refers to pyridinyl, pyrrolyl, imidazolyl or thiazolyl.

2. A compound in accordance with claim 1 wherein R and R₁ are hydrogen.

3. A compound in accordance with claim 1 wherein R is hydrogen and R₁ is C₁₋₁₀-alkyl or R and R₁ are each C₁₋₁₀-alkyl.

4. A compound in accordance with claim 1 wherein R is hydrogen and R₁ is C₂₋₁₀-alkenyl.

5. A compound in accordance with claim 4 wherein R is hydrogen and R₁ is vinyl.

6. A compound in accordance with claim 1 wherein R is hydrogen and R₁ is C₂₋₁₀-alkynyl.

7. A compound in accordance with claim 1 wherein R is hydrogen and R₁ is aryl.

8. A compound in accordance with claim 1 wherein R is hyrogen and R₁ is C₃₋₇-cycloalkyl.

9. A compound in accordance with claim 1 wherein R₂ and R₃ are each C₁₋₁₀-alkyl.

10. A compound in accordance with claim 9 wherein R₂ and R₃ are each methyl.

11. A compound in accordance with claim 1 wherein R₂ and R₃ are each C₃₋₇-cycloalkyl.

12. A compound in accordance with claim 1 whrein R₂ and R₃ together with the nitrogen atom to which they are attached are pyrrolidinyl, piperidinyl or morpholinyl.

13. A compound in accordance with claim 1 wherein R₄ is hydrogen.

14. A compound in accordance with claim 1 wherein R₄ is halogen or trifluoromethyl.

15. A compound in accordance with claim 14 wherein R₄ is chlorine or trifluoromethyl and is located in the 7-position of the benzazepine nucleus.

16. A compound in accordance with claim 1 wherein R₅ is C₁₋₁₀-alkoxy.

17. A compound in accordance with claim 1 wherein R₅ is methoxy and is located in the 4-position of the phenyl ring to which it is attached.

18. A compound in accordance with claim 1 wherein n is 2.

19. A compound in accordance with claim 1 wherein n is 3.

20. The d-cis enantiomer of a compound of claim 1.

21. The compound in accordance with claim 1, (cis)-7-chloro-1-[2-(dimethylamino)ethyl]-1,3,4,5-tetrahydro-4-(4-methoxyphenyl)-3-methyl-2H-1-benzazepin-2-one or a pharmaceutically acceptable salt thereof.

22. The compound in accordance with claim 1, (cis)-7-chloro-1-[2-(dimethylamino)ethyl]-3-ethyl-1,3,4,5-tetrahydro-4-(4-methoxyphenyl)-2H-1-benzazepin-2-one or a pharmaceutically acceptable salt thereof.

23. The compound in accordance with claim 1, (cis)-7-chloro-1-[2-(dimethylamino)ethyl]-1,3,4,5-tetrahydro-4-(4-methoxyphenyl)-3-(2-propenyl)-2H-1-benzazepin-2-one or a pharmaceutically acceptable salt thereof.

24. The compound in accordance with claim 1, (cis)-1-[2-(dimethylamino)ethyl]-1,3,4,5-tetrahydro-4-4-methoxyphenyl)-3-methyl-7-(trifluoromethyl)-2H-1-benzazepin-2-one or a pharmaceutically acceptable salt thereof.

25. The compound in accordance with claim 1, (cis)-1-[2-(dimethylamino)ethyl]-1,3,4,5-tetrahydro-4-(4-methoxyphenyl)-3-(2-propenyl)-7-(trifluoromethyl)-2H-1-benzazepin-2-one or a pharmaceutically acceptable salt thereof.

26. The compound in accordance with claim 1, (cis)-1-[2-(dimethylamino)ethyl]-1,3,4,5-tetrahydro-4-(4-methoxyphenyl)-3-propyl-7-(trifluoromethyl)-2H-1-benzazepin-2-one or a pharmaceutically acceptable salt thereof.

27. The compound in accordance with claim 1, (cis)-1-[2-(dimethylamino)ethyl]-1,3,4,5-tetrahydro-4-(4-methoxyphenyl)-3-(2-propenyl)-6-(trifluoromethyl)-2H-1-benzazepin-2-one or a pharmaceutically acceptable salt thereof.

28. The compound in accordance with claim 1, (cis)-1-[2-(dimethylamino)ethyl]-1,3,4,5-tetrahydro-4-(4-methoxyphenyl)-3-propyl-6-(trifluoromethyl)-2H-1-benzazepin-2-one or a pharmaceutically acceptable salt thereof.

29. The compound in accordance with claim 20, (d-cis)-1-[2-(dimethylamino)ethyl]-1,3,4,5-tetrahydro-4-(4-methoxyphenyl)-3-(2-propenyl)-6-(trifluoromethyl)-2H-1-benzazepin-2-one or a pharmaceutically acceptable salt thereof.

30. The compound in accordance with claim 20, (d-cis)-1-[2-(dimethylamino)ethyl]-1,3,4,5-tetrahydro-4-(4-methoxyphenyl)-3-ethyl-6-(trifluoromethyl)-2H-1-benzazepin-2-one or a pharmaceutically acceptable salt thereof.

31. The compound in accordance with claim 20, (d-cis)-1-[2-(dimethylamino)ethyl]-1,3,4,5-tetrahydro-4-(4-methoxyphenyl)-3-methyl-6-(trifluoromethyl)-2H-1-benzazepin-2-one or a pharmaceutically acceptable salt thereof.

32. The d-cis enantiomer of a compound having the formula 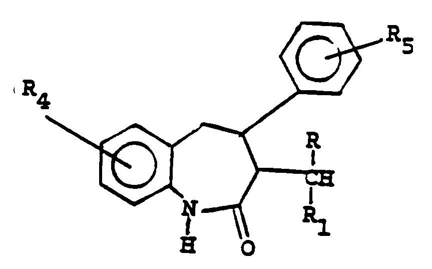 wherein R and R₁ are each hydrogen or alkyl, R is hydrogen and R₁ is C₁₋₁₀-alkyl-, C₂₋₁₀-alkenyl, C₂₋₁₀-alkynyl, aryl, or C₃₋₇-cycloalkyl,
R₄ and R₅ are each independently hydrogen, halogen, C₁₋₁₀-alkyl, C₂₋₁₀-alkoxy, aryloxy, aryl-C₁₋₁₀-alkoxy, aryl-C₁₋₁₀-alkyl, hydroxy, C₂₋₁₁-alkanoyloxy, 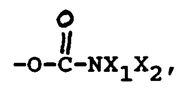 difluoromethoxy, trifluoromethyl, -NX₃X₄, -S(O)ₘC₁₋₁₀-alkyl, or -S(O)ₘaryl;
m is O, 1 or 2;
X₁ and X₂ are each independently hydrogen, C₁₋₁₀-alkyl, aryl or heteroaryl, or X₁ and X₂ together with the nitrogen atom to which they are attached are a nitrogen containing heteroaryl;
X₃ and X₄ are each independently hydrogen, C₁₋₁₀-alkyl, C₂₋₁₁-alkanoyl, arylcarbonyl, heteroarylcarbonyl, or carbamoyl.

33. A process for preparing a compound of any of the claims 1 to 31 which comprises reacting a compound having the formula 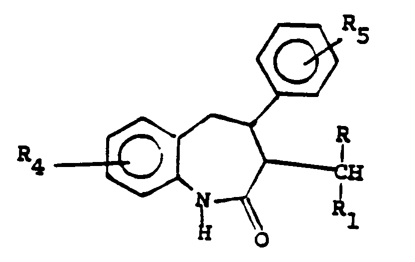 with a compound having the formula
halogen-(CH₂)ₙ-NR₂R₃
and optionally converting the product to a pharmaceutically acceptable salt.

34. A process in accordance with claim 33 wherein the benzazepine reactant and product are each d-cis enantiomers.

35. A process for preparing a compound of claim 32 which comprises the decarboxylation of the d-cis enantiomer of a compound having the formula 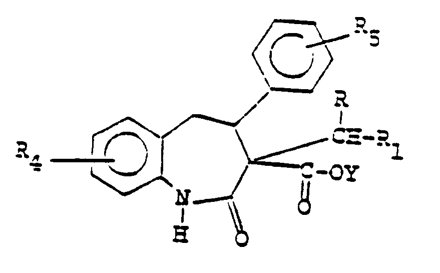 wherein Y is C₁₋₁₀-alkyl.

36. A process for preparing a pharmaceutical preparation which comprises mixing a compound obtainable by the process of of any of the claims 1 to 35 and a pharmaceutically acceptable carrier.

## Patentansprüche (Patentansprüche für folgende(n) Vertragsstaat(en): BE, CH, DE, FR, GB, IT, LI, LU, NL, SE)

1. Verbindung der Formel 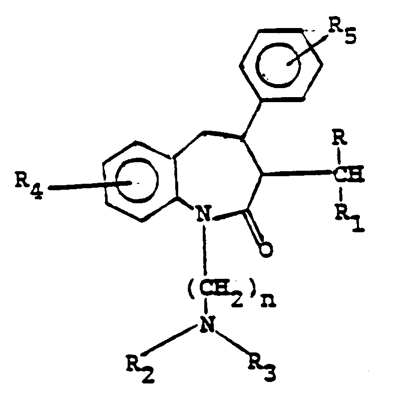 oder ein pharmazeutisch verträgliches Salz davon, wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder
C₃₋₇-Cycloalkylrest ist, R₂ und R₃ jeweils unabhängige C₁₋₁₀-Alkyl- oder C₃₋₇-Cycloalkylreste sind oder R₂ und R₃ zusammen mit dem an sie gebundenen Stickstoffatom Pyrrolidinyl-, Piperidinyl- oder Morpholinylreste bilden;
R₄ und R₅ jeweils unabhängige Wasserstoff-, Halogenatome, C₁₋₁₀-Alkyl-, C₁₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 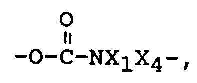 Difluoromethoxy-, Trifluormethyl-, -NH₃X₄, -S(O)ₘC₁₋₁₀-Alkyl-, oder -S(O)ₘ-Arylreste sind;
n der Wert 2 oder 3 ist;
m der Wert 0, 1 oder 2 ist;
X₁ und X₂ jeweils unabhängige Waaserstoffatome, C₁₋₁₀-Alkyl-, Aryl- oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl oder Carbamoylreste sind;
mit der Maßgabe, daß wenn R₄ ein 7-Alkylrest ist, er ein am Ring gebundenes tertiäres Kohlenstoffatom aufweisen muß;
wobei der Begriff "Aryl" sich auf eine Phenylgruppe und einen mit 1, 2 oder 3 Amino-, Alkylamino-, Dialkylamino-, Nitro-, Halogen-, Hydroxyl-, Trifluoromethylresten, Alkylresten mit 1 bis 4 Kohlenstoffatomen, Alkoxyresten mit 1 bis 4 Kohlenstoffatomen, Alkanoyloxy-, Carbamoyl- oder Carbonsäuregruppen substituierten Phenylrest bezieht;
der Begriff "Heteroaryl" sich auf eine Pyridinyl-, Pyrrolyl-, Imidazolyl-, Furyl-, Thienyl- oder Thiazolylgruppe und der Begriff "stickstoffhaltiger Heteroarylrest" sich auf eine Pyridinyl-, Pyrrolyl-, Imidazolyl- oder Thiazolylgruppe bezieht.

2. Verbindung nach Anspruch 1, wobei R und R₁ Wasserstoffatome sind.

3. Verbindung nach Anspruch 1, wobei R ein Wasserstoffatom und R₁ ein C₁₋₁₀-Alkylrest ist oder R und R₁ jeweils C₁₋₁₀-Alkylreste sind.

4. Verbindung nach Anspruch 1, wobei R ein Wasserstoffatom und R₁ ein C₂₋₁₀-Alkenylrest ist.

5. Verbindung nach Anspruch 4, wobei R ein Wasserstoffatom und R₁ eine Vinylgruppe ist.

6. Verbindung nach Anspruch 1, wobei R ein Wasserstoffatom und R₁ ein C₂₋₁₀-Alkinylrest ist.

7. Verbindung nach Anspruch 1, wobei R ein Wasserstoffatom und R₁ ein Arylrest ist.

8. Verbindung nach Anspruch 1, wobei R ein Wasserstoffatom und R₁ ein C₃₋₇-Cycloalkylrest ist.

9. Verbindung nach Anspruch 1, wobei R₂ und R₃ jeweils C₁₋₁₀- Alkylreste sind.

10. Verbindung nach Anspruch 9, wobei R₂ und R₃ jeweils Methylreste sind.

11. Verbindung nach Anspruch 1, wobei R₂ und R₃ jeweils C₃₋₇-Cycloalkylreste sind.

12. Verbindung nach Anspruch 1, wobei R₂ und R₃ zusammen mit dem an sie gebundenen Stickstoffatom Pyrrolidinyl-, Piperidinyl- oder Morpholinylgruppen sind.

13. Verbindung nach Anspruch 1, wobei R₄ ein Wasserstoffatom ist.

14. Verbindung nach Anspruch 1, wobei R₄ ein Halogenatom oder eine Trifluormethylgruppe ist.

15. Verbindung nach Anspruch 14, wobei R₄ ein Chloratom oder eine Trifluomethylgruppe ist und sich in 7-Stellung des Benzazepinkerns befindet.

16. Verbindung nach Anspruch 1, wobei R₅ ein C₁₋₁₀-Alkoxyrest ist.

17. Verbindung nach Anspruch 1, wobei R₅ eine Methoxygruppe ist und sich in 4-Stellung des an sie gebundenen Phenylrings befindet.

18. Verbidung nach Anspruch 1, wobei n den Wert 2 hat.

19. Verbindung nach Anspruch 1, wobei n den Wert 3 hat.

20. Das d-cis-Enantiomer einer Verbindung nach Anspruch 1.

21. Verbindung nach Anspruch 1, nämlich (cis)-7-Chlor-1-[2-(dimethylamino)ethyl]-1,3,4,5-tetrahydro-4-(4-methoxyphenyl)-3-methyl-2H-1-benzazepin-2-on oder ein pharmazeutisch verträgliches Salz davon.

22. Verbindung nach Anspruch 1, nämlich (cis)-7-Chlor-1-[2-(dimethylamino)ethyl]-3-ethyl-1,3,4,5-tetrahydro-4-(4-methoxyphenyl)-2H-1-benzazepin-2-on oder ein pharmazeutisch verträgliches Salz davon.

23. Verbindung nach Anspruch 1, nämlich (cis)-7-Chlor-1-[2-(dimethylamino)ethyl]-1,3,4,5-tetrahydro-4-(4-methoxyphenyl)-3-(2-propenyl)-2H-1-benzazepin-2-on oder ein pharmazeutisch verträgliches Salz davon.

24. Verbindung nach Anspruch 1, nämlich (cis)-1-[2-(dimethylamino)ethyl]-1,3,4,5-tetrahydro-4-(4-methoxyphenyl)-3-methyl-7-(trifluormethyl)-2H-1-benzazepin-2-on oder ein pharmazeutisch verträgliches Salz davon.

25. Verbindung nach Anspruch 1, nämlich (cis)-1-[2-(dimethylamino)ethyl]-1,3,4,5-tetrahydro-4-(4-methoxyphenyl)3-(2-propenyl)-7-(trifluormethyl)-2H-1-benzazepin-2-on oder ein pharmazeutisch verträgliches Salz davon.

26. Verbindung nach Anspruch 1, nämlich (cis)-1-[2-(dimethylamino)ethyl]-1,3,4,5-tetrahydro-4-(4- methoxyphenyl)-3-propyl-7-(trifluormethyl)-2H-1-benzazepin-2-on oder ein pharmazeutisch verträgliches Salz davon.

27. Verbindung nach Anspruch 1, nämlich (cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

28. Verbindung nach Anspruch 1, nämlich (cis)-1-[2-(dimethylamino)ethyl]-1,3,4,5-tetrahydro-4-(4-methoxyphenyl)-3-propyl-6-(trifluormethyl)-2H-1-benzazepin-2-on oder ein pharmazeutisch verträgliches Salz davon.

29. Verbindung nach Anspruch 20, nämlich (d-cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

30. Verbindung nach Anspruch 20, nämlich (d-cis)-1-[2-(dimethylamino)ethyl]-1,3,4,5-tetrahydro-4-(4-methoxyphenyl)3-ethyl-6-(trifluormethyl)-2H-1-benzazepin-2-on oder ein pharmazeutisch verträgliches Salz davon.

31. Verbindung nach Anspruch 20, nämlich (d-cis)-1-[2-(dimethylamino)ethyl]-1,3,4,5-tetrahydro-4-(4-methoxyphenyl)-3-methyl-6-(trifluormethyl)-2H-1-benzazepin-2-on oder ein pharmazeutisch verträgliches Salz davon.

32. Das d-cis-Enantiomer einer Verbindung der Formel 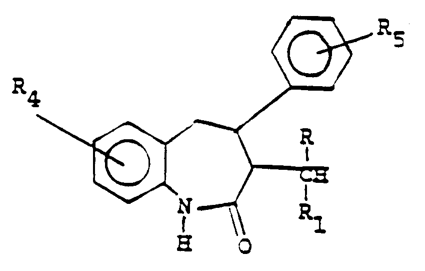 wobei R und R₁ jeweils ein Wasserstoffatom oder ein C₁₋ ₁₀-Alkylrest ist, R ein Wasserstoffatom und R₁ ein C₁₋ ₁₀-Alkyl-, C₂₋₁₀-Alkenyl-, C₂₋₁₀-Alkinyl-, Aryl- oder C₃₋₇-Cycloalkylrest ist, R₄ und R₅ jeweils unabhängige Wasserstoff-, Halogenatome, C₁₋₁₀-Alkyl-, C₂₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 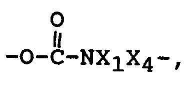 Difluoromethoxy-, Trifluormethyl-, -NH₃X₄, -S(O)ₘC₁₋₁₀-Alkyl-, oder -S(O)ₘ-Arylreste sind;
m der Wert 0, 1 oder 2 ist;
X₁ und X₂ jeweils unabhängige Wasserstoffatome, C₁₋₁₀-Alkyl-, Aryl-oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind.

33. Verfahren zur Herstellung einer Verbindung nach einem der Ansprüche 1 bis 31, umfassend die Umsetzung einer Verbindung der Formel 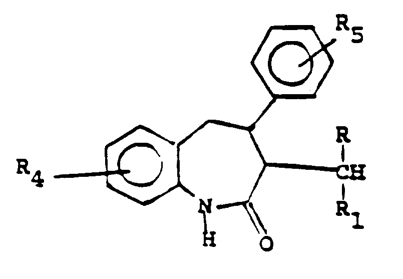 mit einer Verbindung der Formel
Halogen-(CH₂)ₙ-NR₂R₃
und gegebenenfalls die Umwandlung des Produktes zu einem pharmazeutisch verträglichen Salz.

34. Verfahren nach Anspruch 33, wobei der Benzazepin-Reaktant und das Produkt jeweils d-cis-Enantiomere sind.

35. Verfahren zur Herstellung einer Verbindung nach Anspruch 32, umfassend die Decarboxylierung des d-cis-Enantiomers einer Verbindung der Formel 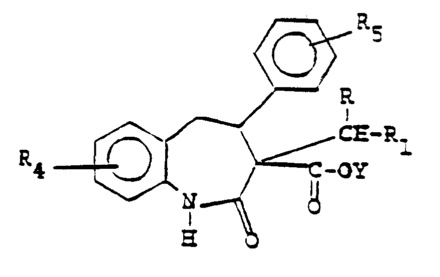 wobei Y ein C₁₋₁₀-Alkylrest ist.

36. Arzneimittel, enthaltend eine Verbindung nach einem der Ansprüche 1 bis 31 und einen pharmazeutisch verträglichen Träger.

## Patentansprüche (Patentansprüche für folgende(n) Vertragsstaat(en): AT)

1. Verfahren zur Herstellung einer Verbindung der Formel 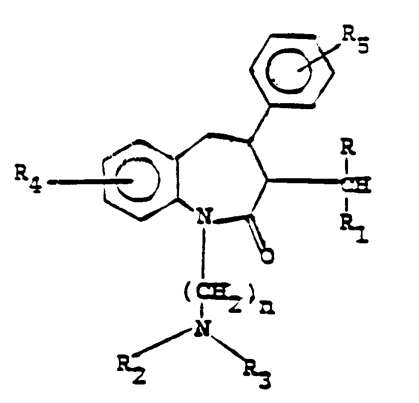 oder ein pharmazeutisch verträgliches Salz davon, wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder C₃₋₇-Cycloalkylrest ist, R₂ und R₃ jeweils unabhängige C₁₋₁₀-Alkyl- oder C₃₋₇-Cycloalkylreste sind oder R₂ und R₃ zusammen mit dem an sie gebundenen Stickstoffatom Pyrrolidinyl-, Piperidinyl- oder Morpholinylreste bilden;
R₄ und R₅ jeweils unabhängige Wasserstoff-, Halogenatome, C₁₋₁₀-Alkyl-, C₁₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 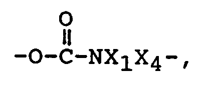 Difluoromethoxy-, Trifluormethyl-, -NH₃X₄, -S(O)ₘC₁₋₁₀-Alkyl-, oder -S(O)ₘ-Arylreste sind;
n der Wert 2 oder 3 ist;
m der Wert 0, 1 oder 2 ist;
X₁ und X₂ jeweils unabhängige Waaserstoffatome, C₁₋₁₀-Alkyl-, Aryl- oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind;
mit der Maßgabe, daß wenn R₄ ein 7-Alkylrest ist, er ein am Ring gebundenes tertiäres Kohlenstoffatom aufweisen muß;
wobei der Begriff "Aryl" sich auf eine Phenylgruppe und einen mit 1, 2 oder 3 Amino-, Alkylamino-, Dialkylamino-, Nitro-, Halogen-, Hydroxyl-, Trifluoromethylresten, Alkylresten mit 1 bis 4 Kohlenstoffatomen, Alkoxyresten mit 1 bis 4 Kohlenstoffatomen, Alkanoyloxy-, Carbamoyl- oder Carbonsäuregruppen substituierten Phenylrest bezieht; der Begriff "Heteroaryl" sich auf eine Pyridinyl-, Pyrrolyl-Imidazolyl-, Furyl-, Thienyl- oder Thiazolylgruppe und der Begriff "stickstoffhaltiger Heteroarylrest" sich auf eine Pyridinyl-, Pyrrolyl-, Imidazolyl- oder Thiazolylgruppe bezieht, umfassend die Umsetzung einer Verbindung der Formel 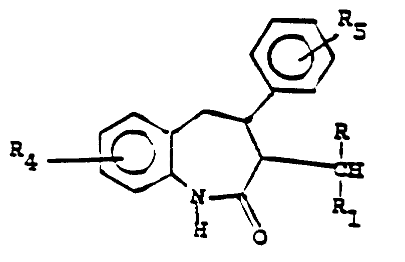 mit einer Verbindung der Formel
Halogen-(CH₂)ₙ-NR₂R₃
und gegebenenfalls die Umwandlung des Produkts zu einem pharmazeutisch verträglichen Salz.

2. Verfahren nach Anspruch 1 zur Herstellung einer Verbindung nach Anspruch 1 , wobei R und R₁ Wasserstoffatome sind.

3. Verfahren nach Anspruch 1 zur Herstellung einer Verbindung nach Anspruch 1, wobei R ein Wasserstoffatom und R₁ ein C₁₋₁₀-Alkylrest ist oder R und R₁ jeweils C₁₋ ₁₀-Alkylreste sind.

4. Verfahren nach Anspruch 1 zur Herstellung einer Verbindung nach Anspruch 1, wobei R ein Wasserstoffatom und R₁ ein C₂₋₁₀-Alkenylrest ist.

5. Verfahren nach Anspruch 4 zur Herstellung einer Verbindung nach Anspruch 4, wobei R ein Wasserstoffatom und R₁ eine Vinylgruppe ist.

6. Verfahren nach Anspruch 1 zur Herstellung einer Verbindung nach Anspruch 1, wobei R ein Wasserstoffatom und R₁ ein C₂₋₁₀-Alkinylrest ist.

7. Verfahren nach Anspruch 1 zur Herstellung einer Verbindung nach Anspruch 1, wobei R ein Wasserstoffatom und R₁ ein Arylrest ist.

8. Verfahren nach Anspruch 1 zur Herstellung einer Verbindung nach Anspruch 1, wobei R ein Wasserstoffatom und R₁ ein C₃₋₇-Cycloalkylrest ist.

9. Verfahren nach Anspruch 1 zur Herstellung einer Verbindung nach Anspruch 1, wobei R₂ und R₃ jeweils C₁₋₁₀- Alkylreste sind.

10. Verfahren nach Anspruch 9 zur Herstellung einer Verbindung nach Anspruch 9, wobei R₂ und R₃ jeweils Methylreste sind.

11. Verfahren nach Anspruch 1 zur Herstellung einer Verbindung nach Anspruch 1, wobei R₂ und R₃ jeweils C₃₋₇-Cycloalkylreste sind.

12. Verfahren nach Anspruch 1 zur Herstellung einer Verbindung nach Anspruch 1, wobei R₂ und R₃ zusammen mit dem an sie gebundenen Stickstoffatom Pyrrolidinyl-, Piperidinyl- oder Morpholinylgruppen sind.

13. Verfahren nach Anspruch 1 zur Herstellung einer Verbindung nach Anspruch 1, wobei R₄ ein Wasserstoffatom ist.

14. Verfahren nach Anspruch 1 zur Herstellung einer Verfahren zur Herstellung einer Verbindung nach Anspruch 1, wobei R₄ ein Halogenatom oder eine Trifluormethylgruppe ist.

15. Verfahren nach Anspruch 14 zur Herstellung einer Verbindung nach Anspruch 14, wobei R₄ ein Chloratom oder eine Trifluomethylgruppe ist und sich in 7-Stellung des Benzazepinkerns befindet.

16. Verfahren nach Anspruch 1 zur Herstellung einer Verbindung nach Anspruch 1, wobei R₅ ein C₁₋₁₀-Alkoxyrest ist.

17. Verfahren nach Anspruch 1 zur Herstellung einer Verbindung nach Anspruch 1, wobei R₅ eine Methoxygruppe ist und sich in 4-Stellung des an sie gebundenen Phenylrings befindet.

18. Verfahren nach Anspruch 1 zur Herstellung einer Verbidung nach Anspruch 1, wobei n den Wert 2 hat.

19. Verfahren nach Anspruch 1 zur Herstellung einer Verbindung nach Anspruch 1, wobei n den Wert 3 hat.

20. Verfahren nach Anspruch 1 zur Herstellung des d-cis-Enantiomer einer Verbindung von Anspruch 1.

21. Verfahren nach Anspruch 1 zur Herstellung von (cis)-7-Chlor-1-[2-(dimethylamino)ethyl]-1,3,4,5-tetrahydro-4-(4-methoxyphenyl)-3-methyl-2H-1-benzazepin-2-on oder ein pharmazeutisch verträgliches Salz davon.

22. Verfahren nach Anspruch 1 zur Herstellung von (cis)-7-Chlor-1-[2-(dimethylamino)ethyl]-3-ethyl-1,3,4,5-tetrahydro-4-(4-methoxyphenyl)-2H-1-benzazepin-2-on oder ein pharmazeutisch verträgliches Salz davon.

23. Verfahren nach Anspruch 1 zur Herstellung von (cis)-7-Chlor-1-[2-(dimethylamino)ethyl]-1,3,4,5-tetrahydro-4-(4-methoxyphenyl)-3-(2-propenyl)-2H-1-benzazepin-2-on oder ein pharmazeutisch verträgliches Salz davon.

24. Verfahren nach Anspruch 1 zur Herstellung von (cis)-1-[2-(dimethylamino)ethyl]-1,3,4,5-tetrahydro-4-(4-methoxyphenyl)-3-methyl-7-(trifluormethyl)-2H-1-benzazepin-2-on oder ein pharmazeutisch verträgliches Salz davon.

25. Verfahren nach Anspruch 1 zur Herstellung von (cis)-1-[2-(dimethylamino)ethyl]-1,3,4,5-tetrahydro-4-(4-methoxyphenyl)3-(2-propenyl)-7-(trifluormethyl)-2H-1-benzazepin-2-on oder ein pharmazeutisch verträgliches Salz davon.

26. Verfahren nach Anspruch 1 zur Herstellung von (cis)-1-[2-(dimethylamino)ethyl]-1,3,4,5-tetrahydro-4-(4-methoxyphenyl)-3-propyl-7-(trifluormethyl)-2H-1-benzazepin-2-on oder ein pharmazeutisch verträgliches Salz davon.

27. Verfahren nach Anspruch 1 zur Herstellung von (cis)-1-[2-(dimethylamino)ethyl]-1,3,4,5-tetrahydro-4-(4- methoxyphenyl)-3-(2-propenyl)-6-(trifluormethyl)-2H-1-benzazepin-2-on oder ein pharmazeutisch verträgliches Salz davon.

28. Verfahren nach Anspruch 1 zur Herstellung von (cis)-1-[2-(dimethylamino)ethyl]-1,3,4,5-tetrahydro-4-(4-methoxyphenyl)-3-propyl-6-(trifluormethyl)-2H-1-benzazepin-2-on oder ein pharmazeutisch verträgliches Salz davon.

29. Verfahren nach Anspruch 20 zur Herstellung von (d-cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

30. Verfahren nach Anspruch 20 zur Herstellung von (d-cis)-1-[2-(dimethylamino)ethyl]-1,3,4,5-tetrahydro-4-(4-methoxyphenyl)3-ethyl-6-(trifluormethyl)-2H-1-benzazepin-2-on oder ein pharmazeutisch verträgliches Salz davon.

31. Verfahren nach Anspruch 20 zur Herstellung von (d-cis)-1-[2-(dimethylamino)ethyl]-1,3,4,5-tetrahydro-4-(4-methoxyphenyl)-3-methyl-6-(trifluormethyl)-2H-1-benzazepin-2-on oder ein pharmazeutisch verträgliches Salz davon.

32. Verfahren nach Anspruch 1 zur Herstellung des d-cis-Enantiomers einer Verbindung der Formel 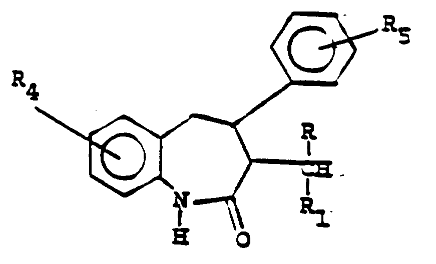 wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder C₃₋₇-Cycloalkylrest ist, R₄ und R₅ jeweils unabhängige Wasserstoff-, Halogenatome, C₁₋₁₀-Alkyl-, C₂₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 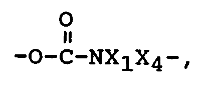 Difluoromethoxy-, Trifluormethyl-, -NH₃X₄, -S(O)ₘC₁₋₁₀-Alkyl-, oder -S(O)ₘ-Arylreste sind;
m der Wert 0, 1 oder 2 ist;
X₁ und X₂ jeweils unabhängige Wasserstoffatome, C₁₋₁₀-Alkyl-, Aryl-oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind.

33. Verfahren nach Anspruch 1, wobei der Benzazepin-Reaktant und das Produkt jeweils d-cis-Enantiomere sind.

34. Verfahren zur Herstellung einer Verbindung nach Anspruch 32, umfassend die Decarboxylierung des d-cis-Enantiomers einer Verbindung der Formel 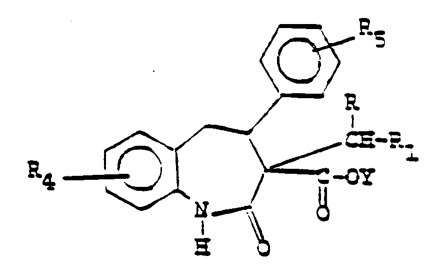 wobei Y ein C₁₋₁₀-Alkylrest ist.

35. Verfahren zur Herstellung eines Arzneimittels, umfassend das Vermischen einer Verbindung erhältlich nach dem Verfahren eines der Ansprüche 1 bis 34 mit einem pharmazeutisch verträglichen Träger.

## Patentansprüche (Patentansprüche für folgende(n) Vertragsstaat(en): ES)

1. Verfahren zur Herstellung einer Verbindung der Formel 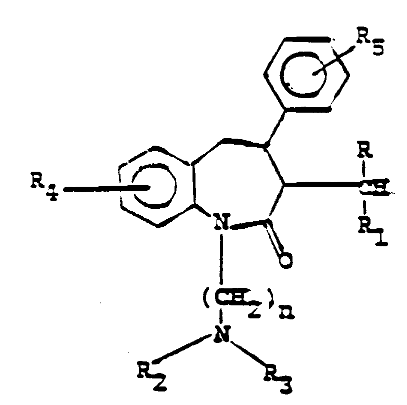 oder ein pharmazeutisch verträgliches Salz davon, wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder C₃₋₇-Cycloalkylrest ist, R₂ und R₃ jeweils unabhängige C₁₋₁₀-Alkyl- oder C₃₋₇-Cycloalkylreste sind oder R₂ und R₃ zusammen mit dem an sie gebundenen Stickstoffatom Pyrrolidinyl-, Piperidinyl- oder Morpholinylreste bilden;
R₄ und R₅ jeweils unabhängige Wasserstoff-, Halogenatome, C₁₋₁₀-Alkyl-, C₁₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 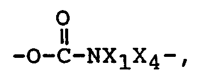 Difluoromethoxy-, Trifluormethyl-, -NH₃X₄, -S(O)ₘC₁₋₁₀-Alkyl-, oder -S(O)ₘ-Arylreste sind;
n der Wert 2 oder 3 ist;
m der Wert 0, 1 oder 2 ist;
X₁ und X₂ jeweils unabhängige Waaserstoffatome, C₁₋₁₀-Alkyl-, Aryl- oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind;
mit der Maßgabe, daß wenn R₄ ein 7-Alkylrest ist, er ein am Ring gebundenes tertiäres Kohlenstoffatom aufweisen muß;
wobei der Begriff "Aryl" sich auf eine Phenylgruppe und einen mit 1, 2 oder 3 Amino-, Alkylamino-, Dialkylamino-, Nitro-, Halogen-, Hydroxyl-, Trifluoromethylresten, Alkylresten mit 1 bis 4 Kohlenstoffatomen, Alkoxyresten mit 1 bis 4 Kohlenstoffatomen, Alkanoyloxy-, Carbamoyl- oder Carbonsäuregruppen substituierten Phenylrest bezieht; der Begriff "Heteroaryl" sich auf eine Pyridinyl-, Pyrrolyl-Imidazolyl, Furyl-, Thienyl- oder Thiazolylgruppe und der Begriff "stickstoffhaltiger Heteroarylrest" sich auf eine Pyridinyl-, Pyrrolyl-, Imidazolyl- oder Thiazolylgruppe bezieht, umfassend die Umsetzung einer Verbindung der Formel 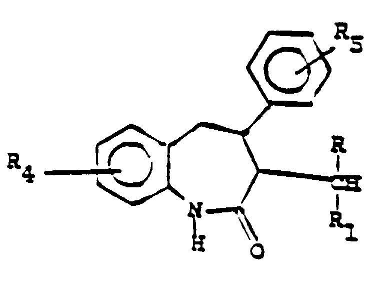 mit einer Verbindung der Formel
Halogen-(CH₂)ₙ-NR₂R₃
und gegebenenfalls die Umwandlung des Produkts zu einem pharmazeutisch verträglichen Salz.

2. Verfahren nach Anspruch 1 zur Herstellung einer Verbindung nach Anspruch 1 , wobei R und R₁ Wasserstoffatome sind.

3. Verfahren nach Anspruch 1 zur Herstellung einer Verbindung nach Anspruch 1, wobei R ein Wasserstoffatom und R₁ ein C₁₋₁₀-Alkylrest ist oder R und R₁ jeweils C₁₋ ₁₀-Alkylreste sind.

4. Verfahren nach Anspruch 1 zur Herstellung einer Verbindung nach Anspruch 1, wobei R ein Wasserstoffatom und R₁ ein C₂₋₁₀-Alkenylrest ist.

5. Verfahren nach Anspruch 4 zur Herstellung einer Verbindung nach Anspruch 4, wobei R ein Wasserstoffatom und R₁ eine Vinylgruppe ist.

6. Verfahren nach Anspruch 1 zur Herstellung einer Verbindung nach Anspruch 1, wobei R ein Wasserstoffatom und R₁ ein C₂₋₁₀-Alkinylrest ist.

7. Verfahren nach Anspruch 1 zur Herstellung einer Verbindung nach Anspruch 1, wobei R ein Wasserstoffatom und R₁ ein Arylrest ist.

8. Verfahren nach Anspruch 1 zur Herstellung einer Verbindung nach Anspruch 1, wobei R ein Wasserstoffatom und R₁ ein C₃₋₇-Cycloalkylrest ist.

9. Verfahren nach Anspruch 1 zur Herstellung einer Verbindung nach Anspruch 1, wobei R₂ und R₃ jeweils C₁₋₁₀- Alkylreste sind.

10. Verfahren nach Anspruch 9 zur Herstellung einer Verbindung nach Anspruch 9, wobei R₂ und R₃ jeweils Methylreste sind.

11. Verfahren nach Anspruch 1 zur Herstellung einer Verbindung nach Anspruch 1, wobei R₂ und R₃ jeweils C₃₋₇-Cycloalkylreste sind.

12. Verfahren nach Anspruch 1 zur Herstellung einer Verbindung nach Anspruch 1, wobei R₂ und R₃ zusammen mit dem an sie gebundenen Stickstoffatom Pyrrolidinyl-, Piperidinyl- oder Morpholinylgruppen sind.

13. Verfahren nach Anspruch 1 zur Herstellung einer Verbindung nach Anspruch 1, wobei R₄ ein Wasserstoffatom ist.

14. Verfahren nach Anspruch 1 zur Herstellung einer Verfahren zur Herstellung einer Verbindung nach Anspruch 1, wobei R₄ ein Halogenatom oder eine Trifluormethylgruppe ist.

15. Verfahren nach Anspruch 14 zur Herstellung einer Verbindung nach Anspruch 14, wobei R₄ ein Chloratom oder eine Trifluomethylgruppe ist und sich in 7-Stellung des Benzazepinkerns befindet.

16. Verfahren nach Anspruch 1 zur Herstellung einer Verbindung nach Anspruch 1, wobei R₅ ein C₁₋₁₀-Alkoxyrest ist.

17. Verfahren nach Anspruch 1 zur Herstellung einer Verbindung nach Anspruch 1, wobei R₅ eine Methoxygruppe ist und sich in 4-Stellung des an sie gebundenen Phenylrings befindet.

18. Verfahren nach Anspruch 1 zur Herstellung einer Verbidung nach Anspruch 1, wobei n den Wert 2 hat.

19. Verfahren nach Anspruch 1 zur Herstellung einer Verbindung nach Anspruch 1, wobei n den Wert 3 hat.

20. Verfahren nach Anspruch 1 zur Herstellung des d-cis-Enantiomer einer Verbindung von Anspruch 1.

21. Verfahren nach Anspruch 1 zur Herstellung von (cis)-7-Chlor-1-[2-(dimethylamino)ethyl]-1,3,4,5-tetrahydro-4-(4-methoxyphenyl)-3-methyl-2H-1-benzazepin-2-on oder ein pharmazeutisch verträgliches Salz davon.

22. Verfahren nach Anspruch 1 zur Herstellung von (cis)-7-Chlor-1-[2-(dimethylamino)ethyl]-3-ethyl-1,3,4,5-tetrahydro-4-(4-methoxyphenyl)-2H-1-benzazepin-2-on oder ein pharmazeutisch verträgliches Salz davon.

23. Verfahren nach Anspruch 1 zur Herstellung von (cis)-7-Chlor-1-[2-(dimethylamino)ethyl]-1,3,4,5-tetrahydro-4-(4-methoxyphenyl)-3-(2-propenyl)-2H-1-benzazepin-2-on oder ein pharmazeutisch verträgliches Salz davon.

24. Verfahren nach Anspruch 1 zur Herstellung von (cis)-1-[2-(dimethylamino)ethyl]-1,3,4,5-tetrahydro-4-(4-methoxyphenyl)-3-methyl-7-(trifluormethyl)-2H-1-benzazepin-2-on oder ein pharmazeutisch verträgliches Salz davon.

25. Verfahren nach Anspruch 1 zur Herstellung von (cis)-1-[2-(dimethylamino)ethyl]-1,3,4,5-tetrahydro-4-(4-methoxyphenyl)3-(2-propenyl)-7-(trifluormethyl)-2H-1-benzazepin-2-on oder ein pharmazeutisch verträgliches Salz davon.

26. Verfahren nach Anspruch 1 zur Herstellung von (cis)-1-[2-(dimethylamino)ethyl]-1,3,4,5-tetrahydro-4-(4-methoxyphenyl)-3-propyl-7-(trifluormethyl)-2H-1-benzazepin-2-on oder ein pharmazeutisch verträgliches Salz davon.

27. Verfahren nach Anspruch 1 zur Herstellung von (cis)-1-[2-(dimethylamino)ethyl]-1,3,4,5-tetrahydro-4-(4- methoxyphenyl)-3-(2-propenyl)-6-(trifluormethyl)-2H-1-benzazepin-2-on oder ein pharmazeutisch verträgliches Salz davon.

28. Verfahren nach Anspruch 1 zur Herstellung von (cis)-1-[2-(dimethylamino)ethyl]-1,3,4,5-tetrahydro-4-(4-methoxyphenyl)-3-propyl-6-(trifluormethyl)-2H-1-benzazepin-2-on oder ein pharmazeutisch verträgliches Salz davon.

29. Verfahren nach Anspruch 20 zur Herstellung von (d-cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

30. Verfahren nach Anspruch 20 zur Herstellung von (d-cis)-1-[2-(dimethylamino)ethyl]-1,3,4,5-tetrahydro-4-(4-methoxyphenyl)3-ethyl-6-(trifluormethyl)-2H-1-benzazepin-2-on oder ein pharmazeutisch verträgliches Salz davon.

31. Verfahren nach Anspruch 20 zur Herstellung von (d-cis)-1-[2-(dimethylamino)ethyl]-1,3,4,5-tetrahydro-4-(4-methoxyphenyl)-3-methyl-6-(trifluormethyl)-2H-1-benzazepin-2-on oder ein pharmazeutisch verträgliches Salz davon.

32. Verfahren nach Anspruch 1 zur Herstellung des d-cis-Enantiomers einer Verbindung der Formel 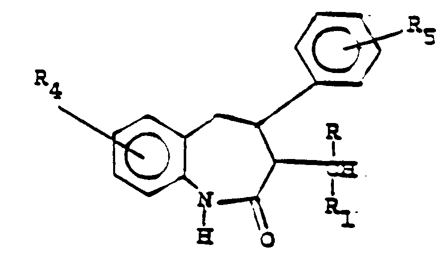 wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder C₃₋₇-Cycloalkylrest ist, R₄ und R₅ jeweils unabhängige Wasserstoff-, Halogenatome, C₁₋₁₀-Alkyl-, C₂₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 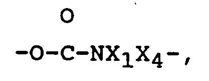 Difluoromethoxy-, Trifluormethyl-, -NH₃X₄, -S(O)ₘC₁₋₁₀-Alkyl-, oder -S(O)ₘ-Arylreste sind;
m der Wert 0, 1 oder 2 ist; X₁ und X₂ jeweils unabhängige Wasserstoffatome,
C₁₋₁₀-Alkyl-, Aryl-oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind.

33. Verfahren nach Anspruch 1, wobei der Benzazepin-Reaktant und das Produkt jeweils d-cis-Enantiomere sind.

34. Verfahren zur Herstellung einer Verbindung nach Anspruch 32, umfassend die Decarboxylierung des d-cis-Enantiomers einer Verbindung der Formel 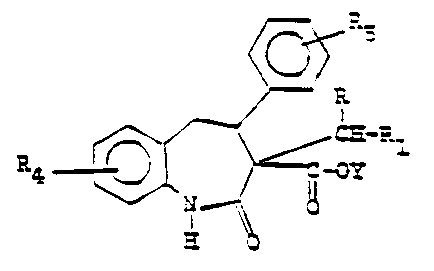 wobei Y ein C₁₋₁₀-Alkylrest ist.

## Patentansprüche (Patentansprüche für folgende(n) Vertragsstaat(en): GR)

1. Verbindung der Formel 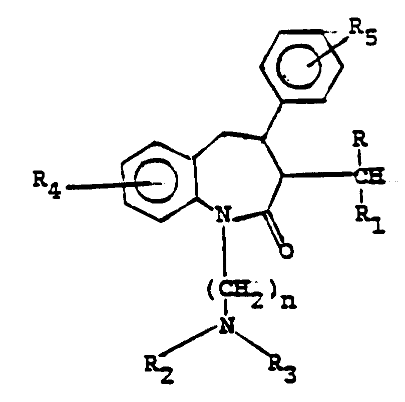 oder ein pharmazeutisch verträgliches Salz davon, wobei R und R₁ jeweils ein Wasserstoffatom oder ein C₁₋₁₀-Alkylrest ist, R ein Wasserstoffatom und R₁ ein C₁₋₁₀-Alkyl-, C₂₋₁₀-Alkenyl-, C₂₋₁₀-Alkinyl-, Aryl- oder
C₃₋₇-Cycloalkylrest ist, R₂ und R₃ jeweils unabhängige C₁₋₁₀-Alkyl- oder C₃₋₇-Cycloalkylreste sind oder R₂ und R₃ zusammen mit dem an sie gebundenen Stickstoffatom Pyrrolidinyl-, Piperidinyl- oder Morpholinylreste bilden;
R₄ und R₅ jeweils unabhängige Wasserstoff-, Halogenatome, C₁₋₁₀-Alkyl-, C₁₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 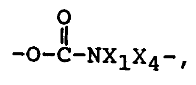 Difluoromethoxy, Trifluormethyl-, -NH₃X₄, -S(O)ₘC₁₋₁₀-Alkyl- oder -S(O)ₘ-Arylreste sind;
n der Wert 2 oder 3 ist;
m der Wert 0, 1 oder 2 ist;
X₁ und X₂ jeweils unabhängige Waaserstoffatome, C₁₋₁₀-Alkyl-, Aryl- oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl-, Arylcarbonyl-, Heteroarylcarbonyl- oder Carbamoylreste sind;
mit der Maßgabe, daß wenn R₄ ein 7-Alkylrest ist, er ein am Ring gebundenes tertiäres Kohlenstoffatom aufweisen muß;
wobei der Begriff "Aryl" sich auf eine Phenylgruppe und einen mit 1, 2 oder 3 Amino-, Alkylamino-, Dialkylamino-, Nitro-, Halogen-, Hydroxyl-, Trifluoromethylresten, Alkylresten mit 1 bis 4 Kohlenstoffatomen, Alkoxyresten mit 1 bis 4 Kohlenstoffatomen, Alkanoyloxy-, Carbamoyl- oder Carbonsäuregruppen substituierten Phenylrest bezieht;
der Begriff "Heteroaryl" sich auf eine Pyridinyl-, Pyrrolyl-, Imidazolyl-, Furyl-, Thienyl- oder Thiazolylgruppe und der Begriff "stickstoffhaltiger Heteroarylrest" sich auf eine Pyridinyl-, Pyrrolyl-, Imidazolyl- oder Thiazolylgruppe bezieht.

2. Verbindung nach Anspruch 1, wobei R und R₁ Wasserstoffatome sind.

3. Verbindung nach Anspruch 1, wobei R ein Wasserstoffatom und R₁ ein C₁₋₁₀-Alkylrest ist oder R und R₁ jeweils C₁₋₁₀-Alkylreste sind.

4. Verbindung nach Anspruch 1, wobei R ein Wasserstoffatom und R₁ ein C₂₋₁₀-Alkenylrest ist.

5. Verbindung nach Anspruch 4, wobei R ein Wasserstoffatom und R₁ eine Vinylgruppe ist.

6. Verbindung nach Anspruch 1, wobei R ein Wasserstoffatom und R₁ ein C₂₋₁₀-Alkinylrest ist.

7. Verbindung nach Anspruch 1, wobei R ein Wasserstoffatom und R₁ ein Arylrest ist.

8. Verbindung nach Anspruch 1, wobei R ein Wasserstoffatom und R₁ ein C₃₋₇-Cycloalkylrest ist.

9. Verbindung nach Anspruch 1, wobei R₂ und R₃ jeweils C₁₋₁₀- Alkylreste sind.

10. Verbindung nach Anspruch 9, wobei R₂ und R₃ jeweils Methylreste sind.

11. Verbindung nach Anspruch 1, wobei R₂ und R₃ jeweils C₃₋₇-Cycloalkylreste sind.

12. Verbindung nach Anspruch 1, wobei R₂ und R₃ zusammen mit dem an sie gebundenen Stickstoffatom Pyrrolidinyl-, Piperidinyl- oder Morpholinylgruppen sind.

13. Verbindung nach Anspruch 1, wobei R₄ ein Wasserstoffatom ist.

14. Verbindung nach Anspruch 1, wobei R₄ ein Halogenatom oder eine Trifluormethylgruppe ist.

15. Verbindung nach Anspruch 14, wobei R₄ ein Chloratom oder eine Trifluomethylgruppe ist und sich in 7-Stellung des Benzazepinkerns befindet.

16. Verbindung nach Anspruch 1, wobei R₅ ein C₁₋₁₀-Alkoxyrest ist.

17. Verbindung nach Anspruch 1, wobei R₅ eine Methoxygruppe ist und sich in 4-Stellung des an sie gebundenen Phenylrings befindet.

18. Verbidung nach Anspruch 1, wobei n den Wert 2 hat.

19. Verbindung nach Anspruch 1, wobei n den Wert 3 hat.

20. Das d-cis-Enantiomer einer Verbindung nach Anspruch 1.

21. Verbindung nach Anspruch 1, nämlich (cis)-7-Chlor-1-[2-(dimethylamino)ethyl]-1,3,4,5-tetrahydro-4-(4-methoxyphenyl)-3-methyl-2H-1-benzazepin-2-on oder ein pharmazeutisch verträgliches Salz davon.

22. Verbindung nach Anspruch 1, nämlich (cis)-7-Chlor-1-[2-(dimethylamino)ethyl]-3-ethyl-1,3,4,5-tetrahydro-4-(4-methoxyphenyl)-2H-1-benzazepin-2-on oder ein pharmazeutisch verträgliches Salz davon.

23. Verbindung nach Anspruch 1, nämlich (cis)-7-Chlor-1-[2-(dimethylamino)ethyl]-1,3,4,5-tetrahydro-4-(4-methoxyphenyl)-3-(2-propenyl)-2H-1-benzazepin-2-on oder ein pharmazeutisch verträgliches Salz davon.

24. Verbindung nach Anspruch 1, nämlich (cis)-1-[2-(dimethylamino)ethyl]-1,3,4,5-tetrahydro-4-(4-methoxyphenyl)-3-methyl-7-(trifluormethyl)-2H-1-benzazepin-2-on oder ein pharmazeutisch verträgliches Salz davon.

25. Verbindung nach Anspruch 1, nämlich (cis)-1-[2-(dimethylamino)ethyl]-1,3,4,5-tetrahydro-4-(4-methoxyphenyl)3-(2-propenyl)-7-(trifluormethyl)-2H-1-benzazepin-2-on oder ein pharmazeutisch verträgliches Salz davon.

26. Verbindung nach Anspruch 1, nämlich (cis)-1-[2-(dimethylamino)ethyl]-1,3,4,5-tetrahydro-4-(4- methoxyphenyl)-3-propyl-7-(trifluormethyl)-2H-1-benzazepin-2-on oder ein pharmazeutisch verträgliches Salz davon.

27. Verbindung nach Anspruch 1, nämlich (cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

28. Verbindung nach Anspruch 1, nämlich (cis)-1-[2-(dimethylamino)ethyl]-1,3,4,5-tetrahydro-4-(4-methoxyphenyl)-3-propyl-6-(trifluormethyl)-2H-1-benzazepin-2-on oder ein pharmazeutisch verträgliches Salz davon.

29. Verbindung nach Anspruch 20, nämlich (d-cis)-1-[2-(dimethylamino)ethyl]-1,3,4,5-tetrahydro-4-(4-methoxyphenyl)-3-(2-propenyl)-6-(trifluormethyl)-2H-1-benzazepin-2-on oder ein pharmazeutisch verträgliches Salz davon.

30. Verbindung nach Anspruch 20, nämlich (d-cis)-1-[2-(dimethylamino)ethyl]-1,3,4,5-tetrahydro-4-(4-methoxyphenyl)3-ethyl-6-(trifluormethyl)-2H-1-benzazepin-2-on oder ein pharmazeutisch verträgliches Salz davon.

31. Verbindung nach Anspruch 20, nämlich (d-cis)-1-[2-(dimethylamino)ethyl]-1,3,4,5-tetrahydro-4-(4-methoxyphenyl)-3-methyl-6-(trifluormethyl)-2H-1-benzazepin-2-on oder ein pharmazeutisch verträgliches Salz davon.

32. Das d-cis-Enantiomer einer Verbindung der Formel 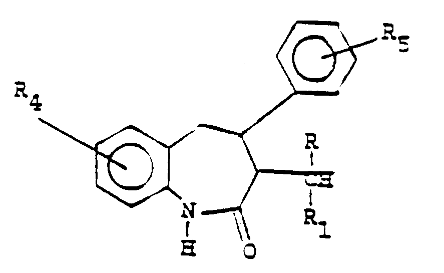 wobei R und R₁ jeweils ein Wasserstoffatom oder ein C₁₋ ₁₀-Alkylrest ist, R ein Wasserstoffatom und R₁ ein C₁₋ ₁₀-Alkyl-, C₂₋₁₀-Alkenyl-, C₂₋₁₀-Alkinyl-, Aryl- oder C₃₋₇-Cycloalkylrest ist, R₄ und R₅ jeweils unabhängige Wasserstoff-, Halogenatome, C₁₋₁₀-Alkyl-, C₂₋₁₀-Alkoxy-, Aryloxy-, Aryl-C₁₋₁₀-Alkoxy-, Aryl-C₁₋₁₀-Alkyl-, Hydroxy-, C₂₋₁₁-Alkanoyloxy-, 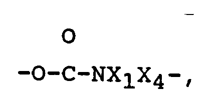 Difluoromethoxy-, Trifluormethyl-, -NH₃X₄, -S(O)ₘC₁₋₁₀-Alkyl-, oder -S(O)ₘ-Arylreste sind;
m der Wert 0, 1 oder 2 ist;
X₁ und X₂ jeweils unabhängige Wasserstoffatome, C₁₋₁₀-Alkyl-, Aryl-oder Heteroarylreste sind, oder X₁ und X₂ zusammen mit dem an sie gebundenen Stickstoffatom einen stickstoffhaltigen Arylrest bilden;
X₃ und X₄ jeweils unabhängige Wasserstoffatome, C₁₋₁₀-Alkyl-, C₂₋₁₁-Alkanoyl, Arylcarbonyl-, Heteroarylcarbonyl- oder Carbamoylreste sind.

33. Verfahren zur Herstellung einer Verbindung nach einem der Ansprüche 1 bis 31, umfassend die Umsetzung einer Verbindung der Formel 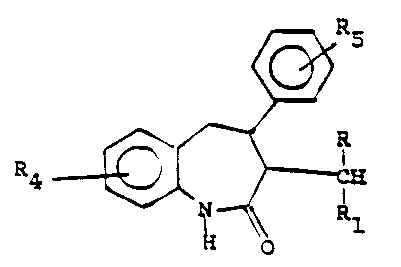 mit einer Verbindung der Formel
Halogen-(CH₂)ₙ-NR₂R₃
und gegebenenfalls die Umwandlung des Produktes zu einem pharmazeutisch verträglichen Salz.

34. Verfahren nach Anspruch 33, wobei der Benzazepin-Reaktant und das Produkt jeweils d-cis-Enantiomere sind.

35. Verfahren zur Herstellung einer Verbindung nach Anspruch 32, umfassend die Decarboxylierung des d-cis-Enantiomers einer Verbindung der Formel 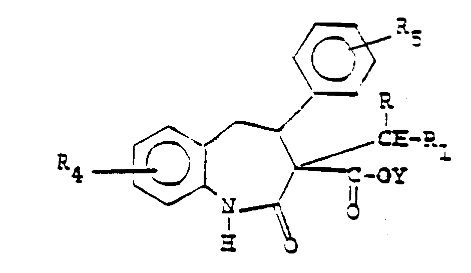 wobei Y ein C₁₋₁₀-Alkylrest ist.

36. Verfahren zur Herstellung eines Arzneimittels, umfassend das Vermischen einer Verbindung erhältlich nach dem Verfahren eines der Ansprüche 1 bis 35 mit einem pharmazeutisch verträglichen Träger.

## Revendications (Revendications pour l'(les) Etat(s) contractant(s) suivant(s): BE, CH, DE, FR, GB, IT, LI, LU, NL, SE)

1. Composé ayant pour formule 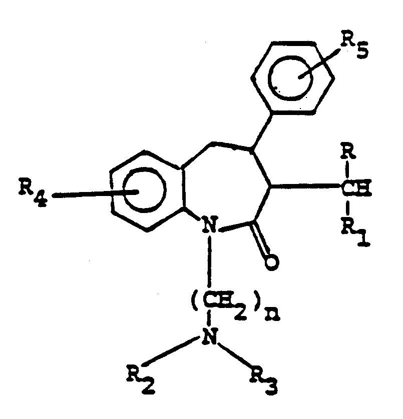 éventuellement sous la forme d'un sel pharmaceutiquement acceptable, formule dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₂ et R₃ sont chacun, indépendamment, un groupe alkyle en C₁₋₁₀ ou cycloalkyle en C₃₋₇, ou bien R₂ et R₃ forment, avec l'atome d'azote auquel ils sont fixés, un groupe pyrrolidinyle, pipéridinyle ou morpholinyle;
R₄ et R₅ sont chacun, indépendamment, l'hydrogène, un halogène, un groupe alkyle en C₁₋₁₀, alcoxy en C₁₋₁₀, aryloxy, aryl-alcoxy en C₁₋₁₀, aryl-alkyle en C₁₋₁₀, hydroxy, alcanoyloxy en C₂₋₁₁, 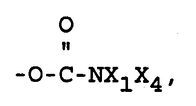 difluorométhoxy, trifluorométhyle, -NX₃X₄, -S(O)ₘalkyle en C₁₋₁₀ ou -S(O)ₘ aryle;
n est égal à 2 ou 3;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle;
avec cette réserve que, si R₄ est un groupe 7-alkyle, il doit avoir un atome de carbone tertiaire lié au cycle;
le terme "aryle" désignant un groupe phényle éventuellement substitué par 1, 2 ou 3 atomes ou groupes amine, alkylamine, dialkylamine, nitro, halogène, hydroxyle, trifluorométhyle, alkyle de 1 à 4 atomes de carbone, alcoxy de 1 à 4 atomes de carbone, alcanoyloxy, carbamoyle ou carboxyle;
le terme "hétéroaryle" désignant un groupe pyridinyle, pyrrolyle, imidazolyle, furyle, thiényle ou thiazolyle; et
le terme "groupe hétéroaryle azoté" désignant un groupe pyridinyle, pyrrolyle, imidazolyle ou thiazolyle.

2. Composé selon la revendication 1, dans lequel R et R₁ sont l'hydrogène.

3. Composé selon la revendication 1, dans lequel R est l'hydrogène et R₁ est un groupe alkyle en C₁₋₁₀, ou bien R et R₁ sont chacun un groupe alkyle en C₁₋₁₀.

4. Composé selon la revendication 1, dans lequel R est l'hydrogène et R₁ est un groupe alcényle en C₂₋₁₀.

5. Composé selon la revendication 4, dans lequel R est l'hydrogène et R₁ est le groupe vinyle.

6. Composé selon la revendication 1, dans lequel R est l'hydrogène et R₁ est un groupe alcynyle en C₂₋₁₀.

7. Composé selon la revendication 1, dans lequel R est l'hydrogène et R₁ est un groupe aryle.

8. Composé selon la revendication 1, dans lequel R est l'hydrogène et R₁ est un groupe cycloalkyle en C₃₋₇.

9. Composé selon la revendication 1, dans lequel R₂ et R₃ sont chacun un groupe alkyle en C₁₋₁₀.

10. Composé selon la revendication 9, dans lequel R₂ et R₃ sont chacun le groupe méthyle.

11. Composé selon la revendication 1, dans lequel R₂ et R₃ sont chacun un groupe cycloalkyle en C₃₋₇.

12. Composé selon la revendication 1, dans lequel R₂ et R₃ forment, avec l'atome d'azote auquel ils sont fixés, un groupe pyrrolidinyle, pipéridinyle ou morpholinyle.

13. Composé selon la revendication 1, dans lequel R₄ est l'hydrogène.

14. Composé selon la revendication 1, dans lequel R₄ est un halogène ou le groupe trifluorométhyle.

15. Composé selon la revendication 14, dans lequel R₄ est le chlore ou le groupe trifluorométhyle et occupe la position 7 du noyau benzazépine.

16. Composé selon la revendication 1, dans lequel R₅ est un groupe alcoxy en C₁₋₁₀.

17. Composé selon la revendication 1, dans lequel R₅ est le groupe méthoxy et occupe la position 4 du noyau phényle auquel il est fixé.

18. Composé selon la revendication 1, dans lequel n est égal à 2.

19. Composé selon la revendication 1, dans lequel n est égal à 3.

20. L'énantiomère d-cis d'un composé selon la revendication 1.

21. Composé selon la revendication 1, qui est la (cis)-7-chloro-1-[2-(diméthylamino)éthyl]-1,3,4,5-tétrahydro-4- (4-méthoxyphényl)-3-méthyl-2H-1-benzazépin-2-one ou un sel pharmaceutiquement acceptable de celle-ci.

22. Composé selon la revendication 1, qui est la (cis)-7-chloro-1-[2-(diméthylamino)éthyl]-3-éthyl-1,3,4,5-tétrahydro-4-(4-méthoxyphényl)-2H-1-benzazépin-2-one ou un sel pharmaceutiquement acceptable de celle-ci.

23. Composé selon la revendication 1, qui est la (cis)-7-chloro-1-[2-(diméthylamino)éthyl]-1,3,4,5-tétrahydro-4-(4-méthoxyphényl)-3-(2-propényl)-2H-1-benzazépin-2-one ou un sel pharmaceutiquement acceptable de celle-ci.

24. Composé selon la revendication 1, qui est la (cis)-1-[2-(diméthylamino)éthyl]-1,3,4,5-tétrahydro-4-(4-méthoxyphényl)-3-méthyl-7- (trifluorométhyl)-2H-1-benzazépin-2-one ou un sel pharmaceutiquement acceptable de celle-ci.

25. Composé selon la revendication 1, qui est la (cis)-1-[2-(diméthylamino)éthyl]-1,3,4,5-tétrahydro-4-(4-méthoxyphényl)-3-(2-propényl)-7-(trifluorométhyl)-2H -1-benzazépin-2-one ou un sel pharmaceutiquement acceptable de celle-ci.

26. Composé selon la revendication 1, qui est la (cis)-1-[2-(diméthylamino)éthyl]-1,3,4,5-tétrahydro-4-(4-méthoxyphényl)-3-propyl-7-(trifluorométhyl)-2H-1-benzazépin-2-one ou un sel pharmaceutiquement acceptable de celle-ci.

27. Composé selon la revendication 1, qui est la (cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou un sel pharmaceutiquement acceptable de celle-ci.

28. Composé selon la revendication 1, qui est la (cis)-1-[2-(diméthylamino)éthyl]-1,3,4,5-tétrahydro-4-(4-méthoxyphényl)-3-propyl-6- (trifluorométhyl)-2H-1-benzazépin-2-one ou un sel pharmaceutiquement acceptable de celle-ci.

29. Composé selon la revendication 20, qui est la (d-cis)-1-[2-(diméthylamino)éthyl]-1,3,4,5-tétra-hydro-4-(4-méthoxyphényl)-3-(2-propényl)-6-(trifluorométhyl)-2H-1-benzazépin-2-one ou un sel pharmaceutiquement acceptable de celle-ci.

30. Composé selon la revendication 20, qui est la (d-cis)-1-[2-(diméthylamino)éthyl]-1,3,4,5-tétrahydro-4-(4-méthoxyphényl)-3-éthyl-6-(trifluorométhyl)-2H-1-benzazépin-2-one ou un sel pharmaceutiquement acceptable de celle-ci.

31. Composé selon la revendication 20, qui est la (d-cis)-1-[2-(diméthylamino)éthyl]-1,3,4,5-tétra-hydro-4-(4-méthoxyphényl)-3-méthyl-6-(trifluorométhyl)-2H-1-benzazépin-2-one ou un sel pharmaceutiquement acceptable de celle-ci.

32. L'énantiomère d-cis d'un composé ayant pour formule 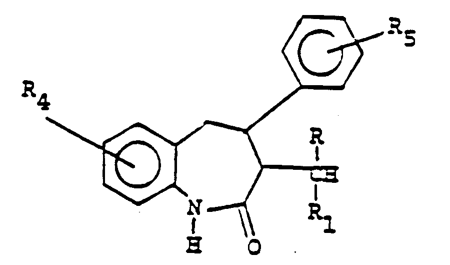 dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₄ et R₅ sont chacun, indépendamment, l'hydrogène, un halogène, ou un groupe alkyle en C₁₋₁₀, alcoxy en C₂₋₁₀, aryloxy, aryl-alcoxy en C₁₋₁₀, aryl-alkyle en C₁₋₁₀, hydroxy, alcanoyloxy en C₂₋₁₁, 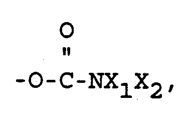 difluorométhoxy, trifluorométhyle, -NX₃X₄, -S(O)ₘ-alkyle en C₁₋₁₀, ou -S(O)ₘaryle;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle.

33. Procédé de préparation d'un composé selon l'une quelconque des revendications 1 à 31, qui consiste à faire réagir un composé de formule 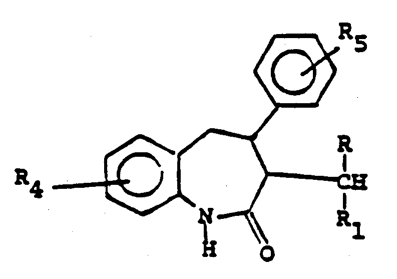 avec un composé de formule
halogène-(CH₂)ₙ-NR₂R₃
et à transformer facultativement ce produit en un sel pharmaceutiquement acceptable.

34. Procédé selon la revendication 33, dans lequel la benzazépine de départ et le produit obtenu sont chacun un énantiomère d-cis.

35. Procédé de préparation d'un composé selon la revendication 32, qui comprend la décarboxylation de l'énantiomère d-cis d'un composé de formule 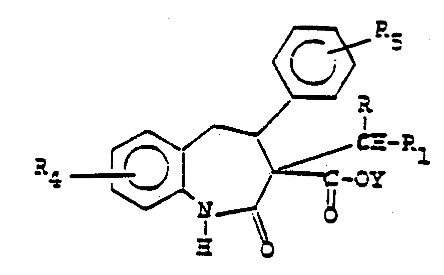 dans laquelle Y est un groupe alkyle en C₁₋₁₀.

36. Préparation pharmaceutique contenant un composé selon l'une quelconque des revendications 1 à 31 et un porteur pharmaceutiquement acceptable.

## Revendications (Revendications pour l'(les) Etat(s) contractant(s) suivant(s): AT)

1. Procédé de préparation d'un composé de formule 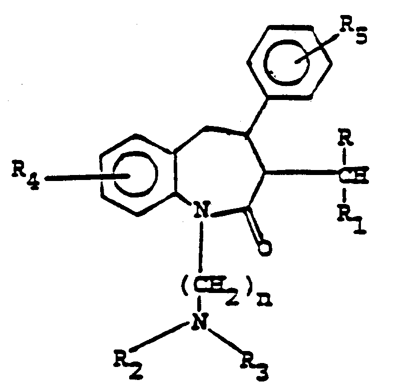 ou d'un sel pharmaceutiquement acceptable de celui-i, formule dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₂ et R₃ sont chacun, indépendamment, un groupe alkyle en C₁₋₁₀ ou cycloalkyle en C₃₋₇, ou bien R₂ et R₃ forment, avec l'atome d'azote auquel ils sont fixés, un groupe pyrrolidinyle, pipéridinyle ou morpholinyle;
R₄ et R₅ sont chacun, indépendamment, l'hydrogène, un halogène, ou un groupe alkyle en C₁₋₁₀, alcoxy en C₁₋₁₀, aryloxy, aryl-alcoxy en C₁₋₁₀, aryl-alkyle en C₁₋₁₀, hydroxy, alcanoyloxy en C₂₋₁₁, 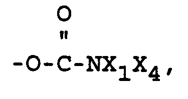 difluorométhoxy, trifluorométhyle, -NX₃X₄, -S(O)ₘ-alkyle en C₁₋₁₀ ou -S(O)ₘaryle;
n est égal à 2 ou 3;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle;
avec cette réserve que, si R₄ est un groupe 7-alkyle, il doit avoir un atome de carbone tertiaire lié au noyau;
le terme "aryle" désignant un groupe phényle éventuellement substitué par 1, 2 ou 3 atomes ou groupements amine, alkylamine, dialkylamine, nitro, halogène, hydroxyle, trifluorométhyle, alkyle de 1 à 4 atomes de carbone, alcoxy de 1 à 4 atomes de carbone, alcanoyloxy, carbamoyle ou carboxyle;
le terme "hétéroaryle" désignant un groupe pyridinyle, pyrrolyle, imidazolyle, furyle, thiényle ou thiazolyle; et
le terme "groupe hétéroaryle azoté" désignant un groupe pyridinyle, pyrrolyle, imidazolyle ou thiazolyle, qui consiste à faire réagir un composé de formule 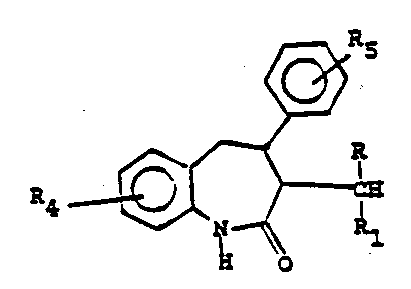 avec un composé de formule
halogène-(CH₂)ₙ-NR₂R₃
et à transformer facultativement le produit en un sel pharmaceutiquement acceptable.

2. Procédé selon la revendication 1 de préparation d'un composé selon la revendication 1, dans lequel R et R₁ sont l'hydrogène.

3. Procédé selon la revendication 1 de préparation d'un composé selon la revendication 1, dans lequel R est l'hydrogène et R₁ est un groupe alkyle en C₁₋₁₀, ou bien R et R₁ sont chacun un groupe alkyle en C₁₋₁₀.

4. Procédé selon la revendication 1 de préparation d'un composé selon la revendication 1, dans lequel R est l'hydrogène et R₁ est un groupe alcényle en C₂₋₁₀.

5. Procédé selon la revendication 4 de préparation d'un composé selon la revendication 4, dans lequel R est l'hydrogène et R₁ est le groupe vinyle.

6. Procédé selon la revendication 1 de préparation d'un composé selon la revendication 1, dans lequel R est l'hydrogène et R₁ est un groupe alcynyle en C₂₋₁₀.

7. Procédé selon la revendication 1 de préparation d'un composé selon la revendication 1, dans lequel R est l'hydrogène et R₁ est un groupe aryle.

8. Procédé selon la revendication 1 de préparation d'un composé selon la revendication 1, dans lequel R est l'hydrogène et R₁ est un groupe cycloalkyle en C₃₋₇.

9. Procédé selon la revendication 1 de préparation d'un composé selon la revendication 1, dans lequel R₂ et R₃ sont chacun un groupe alkyle en C₁₋₁₀.

10. Procédé selon la revendication 9 de préparation d'un composé selon la revendication 9, dans lequel R₂ et R₃ sont chacun le groupe méthyle.

11. Procédé selon la revendication 1 de préparation d'un composé selon la revendication 1, dans lequel R₂ et R₃ sont chacun un groupe cycloalkyle en C₃₋₇.

12. Procédé selon la revendication 1 de préparation d'un composé selon la revendication 1, dans lequel R₂ et R₃ forment, avec l'atome d'azote auquel ils sont fixés, un groupe pyrrolidinyle, pipéridinyle ou morpholinyle.

13. Procédé selon la revendication 1 de préparation d'un composé selon la revendication 1 , dans lequel R₄ est l'hydrogène.

14. Procédé selon la revendication 1 de préparation d'un composé selon la revendication 1, dans lequel R₄ est un halogène ou le groupe trifluorométhyle.

15. Procédé selon la revendication 14 de préparation d'un composé selon la revendication 14, dans lequel R₄ est le chlore ou le groupe trifluorométhyle et occupe la position 7 du noyau benzazépine.

16. Procédé selon la revendication 1 de préparation d'un composé selon la revendication 1, dans lequel R₅ est un groupe alcoxy en C₁₋₁₀.

17. Procédé selon la revendication 1 de préparation d'un composé selon la revendication 1, dans lequel R₅ est le groupe méthoxy et occupe la position 4 du noyau phényle auquel il est fixé.

18. Procédé selon la revendication 1 de préparation d'un composé selon la revendication 1, dans lequel n est égal à 2.

19. Procédé selon la revendication 1 de préparation d'un composé selon la revendication 1, dans lequel n est égal à 3.

20. Procédé selon la revendication 1 de préparation de l'énantiomère d-cis d'un composé selon la revendication 1.

21. Procédé selon la revendication 1 de préparation de la (cis)-7-chloro-1-[2-(diméthylamino)-éthyl]-1,3,4,5-tétrahydro-4-(4-méthoxyphényl)-3-méthyl- 2H-1-benzazépin-2-one ou d'un sel pharmaceutiquement acceptable de celle-ci.

22. Procédé selon la revendication 1 de préparation de la (cis)-7-chloro-1-[2-(diméthylamino)-éthyl]-3-éthyl-1,3,4,5-tétrahydro-4-(4-méthoxyphényl)-2H-1-benzazépin-2-one ou d'un sel pharmaceutiquement acceptable de celle-ci.

23. Procédé selon la revendication 1 de préparation de la(cis)-7-chloro-1-[2-(diméthylamino)-éthyl]-1,3,4,5-tétrahydro-4-(4-méthoxyphényl)-3-(2-propényl)-2H-1-benzazépin-2-one ou un d'un sel pharmaceutiquement acceptable de celle-ci.

24. Procédé selon la revendication 1 de préparation de la (cis)-1-[2-(diméthylamino)éthyl]-1,3,4,5-tétrahydro-4-(4-méthoxyphényl)-3-méthyl-7-(trifluorométhyl)-2H-1-benzazépin-2-one ou d'un sel pharmaceutiquement acceptable de celle-ci.

25. Procédé selon la revendication 1 de préparation de la (cis)-1-[2-(diméthylamino)éthyl]-1,3,4,5-tétrahydro-4-(4-méthoxyphényl)-3-(2-propényl)-7-(trifluorométhyl)-2H -1-benzazépin-2-one ou d'un sel pharmaceutiquement acceptable de celle-ci.

26. Procédé selon la revendication 1 de préparation de la (cis)-1-[2-(diméthylamino)éthyl]-1,3,4,5-tétrahydro-4-(4-méthoxyphényl)-3-propyl-7-(trifluorométhyl)-2H-1-benzazépin-2-one ou d'un sel pharmaceutiquement acceptable de celle-ci.

27. Procédé selon la revendication 1 de préparation de la (cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou d'un sel pharmaceutiquement acceptable de celle-ci.

28. Procédé selon la revendication 1 de préparation de la(cis)-1-[2-(diméthylamino)éthyl]-1,3,4,5-tétrahydro-4-(4-méthoxyphényl)-3-propyl-6-(trifluorométhyl)-2H-1-benzazépin-2-one ou d'un sel pharmaceutiquement acceptable de celle-ci.

29. Procédé selon la revendication 20 de préparation de la (d-cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou d'un sel pharmaceutiquement acceptable de celle-ci.

30. Procédé selon la revendication 20 de préparation de la (d-cis)-1-[2-(diméthylamino)éthyl]-1,3,4,5-tétrahydro-4-(4-méthoxyphényl)-3-éthyl-6-(trifluorométhyl)-2H-1-benzazépin-2-one ou d'un sel pharmaceutiquement acceptable de celle-ci.

31. Procédé selon la revendication 20 de préparation de la (d-cis)-1-[2-(diméthylamino)éthyl]-1,3,4,5-tétrahydro-4-(4-méthoxyphényl)-3-méthyl-6-(trifluorométhyl)-2H-1-benzazépin-2-one ou d'un sel pharmaceutiquement acceptable de celle-ci.

32. Procédé selon la revendication 1 de préparation de l'énantiomère d-cis d'un composé de formule 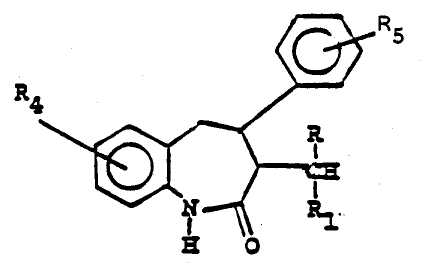 dans laquelle R et R₁ sont chacun l'hydrogène ou un groupe alkyle, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₄ et R₅ sont chacun, indépendamment, l'hydrogène, un halogène, ou un groupe alkyle en C₁₋₁₀, alcoxy en C₂₋₁₀, aryloxy, aryl-alcoxy en C₁₋₁₀, aryl-alkyle en C₁₋₁₀, hydroxy, alcanoyloxy en C₂₋₁₁, 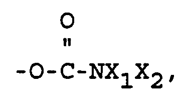 difluorométhoxy, trifluorométhyle, -NX₃X₄, -S(O)ₘ-alkyle en C₁₋₁₀ ou -S(O)ₘaryle;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle.

33. Procédé selon la revendication 1, dans lequel la benzazépine de départ et le produit sont chacun un énantiomère d-cis.

34. Procédé de préparation d'un composé selon la revendication 32, qui comprend la décarboxylation de l'énantiomère d-cis d'un composé de formule 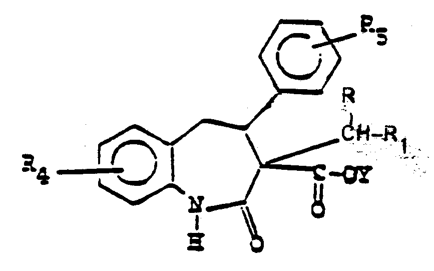 dans laquelle Y est un groupe alkyle en C₁₋₁₀.

35. Procédé de préparation d'un produit pharmaceutique qui consiste à mélanger un composé pouvant être obtenu par le procédé selon l'une quelconque des revendications 1 à 34, avec un porteur pharmaceutiquement acceptable.

## Revendications (Revendications pour l'(les) Etat(s) contractant(s) suivant(s): ES)

1. Procédé de préparation d'un composé de formule 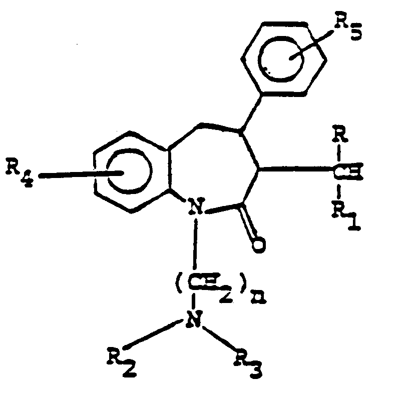 ou d'un sel pharmaceutiquement acceptable de celui-i, formule dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₂ et R₃ sont chacun, indépendamment, un groupe alkyle en C₁₋₁₀ ou cycloalkyle en C₃₋₇, ou bien R₂ et R₃ forment, avec l'atome d'azote auquel ils sont fixés, un groupe pyrrolidinyle, pipéridinyle ou morpholinyle;
R₄ et R₅ sont chacun, indépendamment, l'hydrogène, un halogène, ou un groupe alkyle en C₁₋₁₀, alcoxy en C₁₋₁₀, aryloxy, aryl-alcoxy en C₁₋₁₀, aryl-alkyle en C₁₋₁₀, hydroxy, alcanoyloxy en C₂₋₁₁, 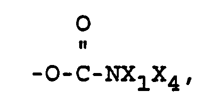 difluorométhoxy, trifluorométhyle, -NX₃X₄, -S(O)ₘ-alkyle en C₁₋₁₀ ou -S(O)ₘaryle;
n est égal à 2 ou 3;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle;
avec cette réserve que, si R₄ est un groupe 7-alkyle, il doit avoir un atome de carbone tertiaire lié au noyau;
le terme "aryle" désignant un groupe phényle éventuellement substitué par 1, 2 ou 3 atomes ou groupements amine, alkylamine, dialkylamine, nitro, halogène, hydroxyle, trifluorométhyle, alkyle de 1 à 4 atomes de carbone, alcoxy de 1 à 4 atomes de carbone, alcanoyloxy, carbamoyle ou carboxyle;
le terme "hétéroaryle" désignant un groupe pyridinyle, pyrrolyle, imidazolyle, furyle, thiényle ou thiazolyle; et
le terme "groupe hétéroaryle azoté" désignant un groupe pyridinyle, pyrrolyle, imidazolyle ou thiazolyle, qui consiste à faire réagir un composé de formule 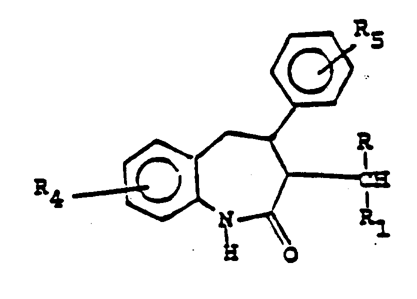 avec un composé de formule
halogène-(CH₂)ₙ-NR₂R₃
et à transformer facultativement le produit en un sel pharmaceutiquement acceptable.

2. Procédé selon la revendication 1 de préparation d'un composé selon la revendication 1, dans lequel R et R₁ sont l'hydrogène.

3. Procédé selon la revendication 1 de préparation d'un composé selon la revendication 1, dans lequel R est l'hydrogène et R₁ est un groupe alkyle en C₁₋₁₀, ou bien R et R₁ sont chacun un groupe alkyle en C₁₋₁₀.

4. Procédé selon la revendication 1 de préparation d'un composé selon la revendication 1, dans lequel R est l'hydrogène et R₁ est un groupe alcényle en C₂₋₁₀.

5. Procédé selon la revendication 4 de préparation d'un composé selon la revendication 4, dans lequel R est l'hydrogène et R₁ est le groupe vinyle.

6. Procédé selon la revendication 1 de préparation d'un composé selon la revendication 1, dans lequel R est l'hydrogène et R₁ est un groupe alcynyle en C₂₋₁₀.

7. Procédé selon la revendication 1 de préparation d'un composé selon la revendication 1, dans lequel R est l'hydrogène et R₁ est un groupe aryle.

8. Procédé selon la revendication 1 de préparation d'un composé selon la revendication 1, dans lequel R est l'hydrogène et R₁ est un groupe cycloalkyle en C₃₋₇.

9. Procédé selon la revendication 1 de préparation d'un composé selon la revendication 1, dans lequel R₂ et R₃ sont chacun un groupe alkyle en C₁₋₁₀.

10. Procédé selon la revendication 9 de préparation d'un composé selon la revendication 9, dans lequel R₂ et R₃ sont chacun le groupe méthyle.

11. Procédé selon la revendication 1 de préparation d'un composé selon la revendication 1, dans lequel R₂ et R₃ sont chacun un groupe cycloalkyle en C₃₋₇.

12. Procédé selon la revendication 1 de préparation d'un composé selon la revendication 1, dans lequel R₂ et R₃ forment, avec l'atome d'azote auquel ils sont fixés, un groupe pyrrolidinyle, pipéridinyle ou morpholinyle.

13. Procédé selon la revendication 1 de préparation d'un composé selon la revendication 1, dans lequel R₄ est l'hydrogène.

14. Procédé selon la revendication 1 de préparation d'un composé selon la revendication 1, dans lequel R₄ est un halogène ou le groupe trifluorométhyle.

15. Procédé selon la revendication 14 de préparation d'un composé selon la revendication 14, dans lequel R₄ est le chlore ou le groupe trifluorométhyle et occupe la position 7 du noyau benzazépine.

16. Procédé selon la revendication 1 de préparation d'un composé selon la revendication 1, dans lequel R₅ est un groupe alcoxy en C₁₋₁₀.

17. Procédé selon la revendication 1 de préparation d'un composé selon la revendication 1, dans lequel R₅ est le groupe méthoxy et occupe la position 4 du noyau phényle auquel il est fixé.

18. Procédé selon la revendication 1 de préparation d'un composé selon la revendication 1, dans lequel n est égal à 2.

19. Procédé selon la revendication 1 de préparation d'un composé selon la revendication 1, dans lequel n est égal à 3.

20. Procédé selon la revendication 1 de préparation de l'énantiomère d-cis d'un composé selon la revendication 1.

21. Procédé selon la revendication 1 de préparation de la (cis)-7-chloro-1-[2-(diméthylamino)-éthyl]-1,3,4,5-tétrahydro-4-(4-méthoxyphényl)-3-méthyl- 2H-1-benzazépin-2-one ou d'un sel pharmaceutiquement acceptable de celle-ci.

22. Procédé selon la revendication 1 de préparation de la (cis)-7-chloro-1-[2-(diméthylamino)-éthyl]-3-éthyl-1,3,4,5-tétrahydro-4-(4-méthoxyphényl)-2H-1-benzazépin-2-one ou d'un sel pharmaceutiquement acceptable de celle-ci.

23. Procédé selon la revendication 1 de préparation de la(cis)-7-chloro-1-[2-(diméthylamino)-éthyl]-1,3,4,5-tétrahydro-4-(4-méthoxyphényl)-3-(2-propényl)-2H-1-benzazépin-2-one ou un d'un sel pharmaceutiquement acceptable de celle-ci.

24. Procédé selon la revendication 1 de préparation de la (cis)-1-[2-(diméthylamino)éthyl]-1,3,4,5-tétrahydro-4-(4-méthoxyphényl)-3-méthyl-7-(trifluorométhyl)-2H-1-benzazépin-2-one ou d'un sel pharmaceutiquement acceptable de celle-ci.

25. Procédé selon la revendication 1 de préparation de la (cis)-1-[2-(diméthylamino)éthyl]-1,3,4,5-tétrahydro-4-(4-méthoxyphényl)-3-(2-propényl)-7-(trifluorométhyl)-2H -1-benzazépin-2-one ou d'un sel pharmaceutiquement acceptable de celle-ci.

26. Procédé selon la revendication 1 de préparation de la (cis)-1-[2-(diméthylamino)éthyl]-1,3,4,5-tétrahydro-4-(4-méthoxyphényl)-3-propyl-7-(trifluorométhyl)-2H-1-benzazépin-2-one ou d'un sel pharmaceutiquement acceptable de celle-ci.

27. Procédé selon la revendication 1 de préparation de la (cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou d'un sel pharmaceutiquement acceptable de celle-ci.

28. Procédé selon la revendication 1 de préparation de la(cis)-1-[2-(diméthylamino)éthyl]-1,3,4,5-tétrahydro-4-(4-méthoxyphényl)-3-propyl-6-(trifluorométhyl)-2H-1-benzazépin-2-one ou d'un sel pharmaceutiquement acceptable de celle-ci.

29. Procédé selon la revendication 20 de préparation de la (d-cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou d'un sel pharmaceutiquement acceptable de celle-ci.

30. Procédé selon la revendication 20 de préparation de la (d-cis)-1-[2-(diméthylamino)éthyl]-1,3,4,5-tétrahydro-4-(4-méthoxyphényl)-3-éthyl-6-(trifluorométhyl)-2H-1-benzazépin-2-one ou d'un sel pharmaceutiquement acceptable de celle-ci.

31. Procédé selon la revendication 20 de préparation de la (d-cis)-1-[2-(diméthylamino)éthyl]-1,3,4,5-tétrahydro-4-(4-méthoxyphényl)-3-méthyl-6-(trifluorométhyl)-2H-1-benzazépin-2-one ou d'un sel pharmaceutiquement acceptable de celle-ci.

32. Procédé selon la revendication 1 de préparation de l'énantiomère d-cis d'un composé de formule 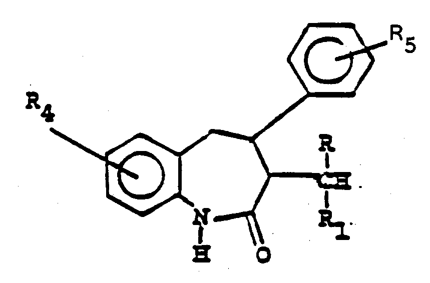 dans laquelle R et R₁ sont chacun l'hydrogène ou un groupe alkyle, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₄ et R₅ sont chacun, indépendamment, l'hydrogène, un halogène, ou un groupe alkyle en C₁₋₁₀, alcoxy en C₂₋₁₀, aryloxy, aryl-alcoxy en C₁₋₁₀, aryl-alkyle en C₁₋₁₀, hydroxy, alcanoyloxy en C₂₋₁₁, 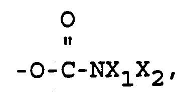 difluorométhoxy, trifluorométhyle, -NX₃X₄, -S(O)ₘ-alkyle en C₁₋₁₀ ou -S(O)ₘaryle;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle.

33. Procédé selon la revendication 1, dans lequel la benzazépine de départ et le produit sont chacun un énantiomère d-cis.

34. Procédé de préparation d'un composé selon la revendication 32, qui comprend la décarboxylation de l'énantiomère d-cis d'un composé de formule 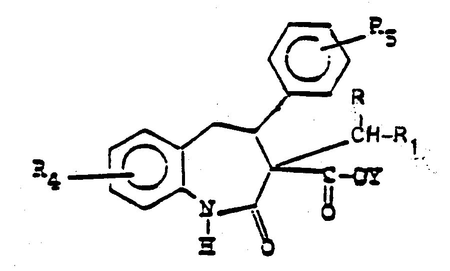 dans laquelle Y est un groupe alkyle en C₁₋₁₀.

## Revendications (Revendications pour l'(les) Etat(s) contractant(s) suivant(s): GR)

1. Composé ayant pour formule 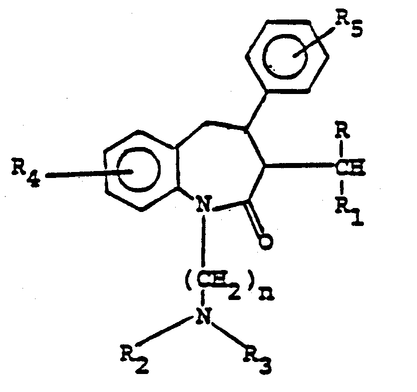 éventuellement sous la forme d'un sel pharmaceutiquement acceptable, formule dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₂ et R₃ sont chacun, indépendamment, un groupe alkyle en C₁₋₁₀ ou cycloalkyle en C₃₋₇, ou bien R₂ et R₃ forment, avec l'atome d'azote auquel ils sont fixés, un groupe pyrrolidinyle, pipéridinyle ou morpholinyle;
R₄ et R₅ sont chacun, indépendamment, l'hydrogène, un halogène, un groupe alkyle en C₁₋₁₀, alcoxy en C₁₋₁₀, aryloxy, aryl-alcoxy en C₁₋₁₀, aryl-alkyle en C₁₋₁₀, hydroxy, alcanoyloxy en C₂₋₁₁, 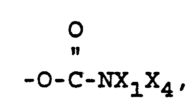 difluorométhoxy, trifluorométhyle, -NX₃X₄, -S(O)ₘalkyle en C₁₋₁₀ ou -S(O)ₘ aryle;
n est égal à 2 ou 3;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle;
avec cette réserve que, si R₄ est un groupe 7-alkyle, il doit avoir un atome de carbone tertiaire lié au cycle;
le terme "aryle" désignant un groupe phényle éventuellement substitué par 1, 2 ou 3 atomes ou groupes amine, alkylamine, dialkylamine, nitro, halogène, hydroxyle, trifluorométhyle, alkyle de 1 à 4 atomes de carbone, alcoxy de 1 à 4 atomes de carbone, alcanoyloxy, carbamoyle ou carboxyle;
le terme "hétéroaryle" désignant un groupe pyridinyle, pyrrolyle, imidazolyle, furyle, thiényle ou thiazolyle; et
le terme "groupe hétéroaryle azoté" désignant un groupe pyridinyle, pyrrolyle, imidazolyle ou thiazolyle.

2. Composé selon la revendication 1, dans lequel R et R₁ sont l'hydrogène.

3. Composé selon la revendication 1, dans lequel R est l'hydrogène et R₁ est un groupe alkyle en C₁₋₁₀, ou bien R et R₁ sont chacun un groupe alkyle en C₁₋₁₀.

4. Composé selon la revendication 1, dans lequel R est l'hydrogène et R₁ est un groupe alcényle en C₂₋₁₀.

5. Composé selon la revendication 4, dans lequel R est l'hydrogène et R₁ est le groupe vinyle.

6. Composé selon la revendication 1, dans lequel R est l'hydrogène et R₁ est un groupe alcynyle en C₂₋₁₀.

7. Composé selon la revendication 1, dans lequel R est l'hydrogène et R₁ est un groupe aryle.

8. Composé selon la revendication 1, dans lequel R est l'hydrogène et R₁ est un groupe cycloalkyle en C₃₋₇.

9. Composé selon la revendication 1, dans lequel R₂ et R₃ sont chacun un groupe alkyle en C₁₋₁₀.

10. Composé selon la revendication 9, dans lequel R₂ et R₃ sont chacun le groupe méthyle.

11. Composé selon la revendication 1, dans lequel R₂ et R₃ sont chacun un groupe cycloalkyle en C₃₋₇.

12. Composé selon la revendication 1, dans lequel R₂ et R₃ forment, avec l'atome d'azote auquel ils sont fixés, un groupe pyrrolidinyle, pipéridinyle ou morpholinyle.

13. Composé selon la revendication 1, dans lequel R₄ est l'hydrogène.

14. Composé selon la revendication 1, dans lequel R₄ est un halogène ou le groupe trifluorométhyle.

15. Composé selon la revendication 14, dans lequel R₄ est le chlore ou le groupe trifluorométhyle et occupe la position 7 du noyau benzazépine.

16. Composé selon la revendication 1, dans lequel R₅ est un groupe alcoxy en C₁₋₁₀.

17. Composé selon la revendication 1, dans lequel R₅ est le groupe méthoxy et occupe la position 4 du noyau phényle auquel il est fixé.

18. Composé selon la revendication 1, dans lequel n est égal à 2.

19. Composé selon la revendication 1, dans lequel n est égal à 3.

20. L'énantiomère d-cis d'un composé selon la revendication 1.

21. Composé selon la revendication 1, qui est la (cis)-7-chloro-1-[2-(diméthylamino)éthyl]-1,3,4,5-tétrahydro-4-(4-méthoxyphényl)-3-méthyl-2H-1-benzazépin-2-one ou un sel pharmaceutiquement acceptable de celle-ci.

22. Composé selon la revendication 1, qui est la (cis)-7-chloro-1-[2-(diméthylamino)éthyl]-3-éthyl-1,3,4,5-tétrahydro-4-(4-méthoxyphényl)-2H-1-benzazépin-2-one ou un sel pharmaceutiquement acceptable de celle-ci.

23. Composé selon la revendication 1, qui est la (cis)-7-chloro-1-[2-(diméthylamino)éthyl]-1,3,4,5-tétrahydro-4-(4-méthoxyphényl)-3-(2-propényl)-2H-1-benzazépin-2-one ou un sel pharmaceutiquement acceptable de celle-ci.

24. Composé selon la revendication 1, qui est la (cis)-1-[2-(diméthylamino)éthyl]-1,3,4,5-tétrahydro-4-(4-méthoxyphényl)-3-méthyl-7-(trifluorométhyl)-2H-1-benzazépin-2-one ou un sel pharmaceutiquement acceptable de celle-ci.

25. Composé selon la revendication 1, qui est la (cis)-1-[2-(diméthylamino)éthyl]-1,3,4,5-tétrahydro-4-(4-méthoxyphényl)-3-(2-propényl)-7-(trifluorométhyl)-2H -1-benzazépin-2-one ou un sel pharmaceutiquement acceptable de celle-ci.

26. Composé selon la revendication 1, qui est la (cis)-1-[2-(diméthylamino)éthyl]-1,3,4,5-tétrahydro-4-(4-méthoxyphényl)-3-propyl-7-(trifluorométhyl)-2H-1-benzazépin-2-one ou un sel pharmaceutiquement acceptable de celle-ci.

27. Composé selon la revendication 1, qui est la (cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou un sel pharmaceutiquement acceptable de celle-ci.

28. Composé selon la revendication 1, qui est la (cis)-1-[2-(diméthylamino)éthyl]-1,3,4,5-tétrahydro-4-(4-méthoxyphényl)-3-propyl-6-(trifluorométhyl)-2H-1-benzazépin-2-one ou un sel pharmaceutiquement acceptable de celle-ci.

29. Composé selon la revendication 20, qui est la (d-cis)-1-[2-(diméthylamino)éthyl]-1,3,4,5-tétrahydro-4-(4-méthoxyphényl)-3-(2-propényl)-6-(trifluorométhyl)-2H-1-benzazépin-2-one ou un sel pharmaceutiquement acceptable de celle-ci.

30. Composé selon la revendication 20, qui est la (d-cis)-1-[2-(diméthylamino)éthyl]-1,3,4,5-tétrahydro-4-(4-méthoxyphényl)-3-éthyl-6-(trifluorométhyl)-2H-1-benzazépin-2-one ou un sel pharmaceutiquement acceptable de celle-ci.

31. Composé selon la revendication 20, qui est la (d-cis)-1-[2-(diméthylamino)éthyl]-1,3,4,5-tétra-hydro-4-(4-méthoxyphényl)-3-méthyl-6-(trifluorométhyl)-2H-1-benzaaépin-2-one ou un sel pharmaceutiquement acceptable de celle-ci.

32. L'énantiomère d-cis d'un composé ayant pour formule 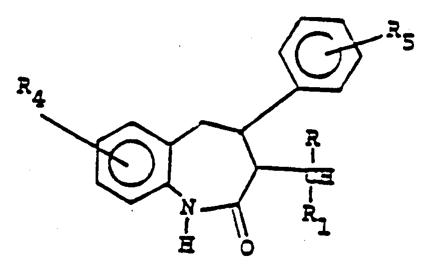 dans laquelle R et R₁ sont chacun l'hydrogène ou un groupe alkyle en C₁₋₁₀, R est l'hydrogène et R₁ est un groupe alkyle en C₁₋₁₀, alcényle en C₂₋₁₀, alcynyle en C₂₋₁₀, aryle ou cycloalkyle en C₃₋₇,
R₄ et R₅ sont chacun, indépendamment, l'hydrogène, un halogène, ou un groupe alkyle en C₁₋₁₀, alcoxy en C₂₋₁₀, aryloxy, aryl-alcoxy en C₁₋₁₀, aryl-alkyle en C₁₋₁₀, hydroxy, alcanoyloxy en C₂₋₁₁, 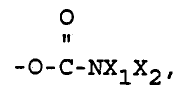 difluorométhoxy, trifluorométhyle, -NX₃X₄, -S(O)ₘ-alkyle en C₁₋₁₀, ou -S(O)ₘaryle;
m est égal à 0, 1 ou 2;
X₁ et X₂ sont chacun, indépendamment, l'hydrogène ou un groupe alkyle en C₁₋₁₀, aryle ou hétéroaryle, ou bien X₁ et X₂ forment, avec l'atome d'azote auquel ils sont fixés, un groupe hétéroaryle azoté;
X₃ et X₄ sont chacun, indépendamment, l'hydrogène ou un groupe alkyle en C₁₋₁₀, alcanoyle en C₂₋₁₁, arylcarbonyle, hétéroarylcarbonyle ou carbamoyle.

33. Procédé de préparation d'un composé selon l'une quelconque des revendications 1 à 31, qui consiste à faire réagir un composé de formule 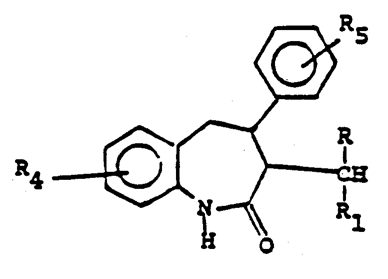 avec un composé de formule
halogène-(CH₂)ₙ-NR₂R₃
et à transformer facultativement ce produit en un sel pharmaceutiquement acceptable.

34. Procédé selon la revendication 33, dans lequel la benzazépine de départ et le produit obtenu sont chacun un énantiomère d-cis.

35. Procédé de préparation d'un composé selon la revendication 32, qui comprend la décarboxylation de l'énantiomère d-cis d'un composé de formule 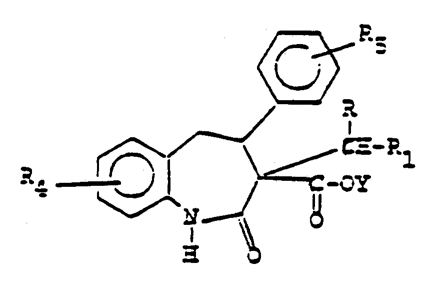 dans laquelle Y est un groupe alkyle en C₁₋₁₀.

36. Procédé de préparation d'un produit pharmaceutique qui consiste à mélanger un composé pouvant être obtenu par le procédé selon l'une quelconque des revendications 1 à 35, avec un porteur pharmaceutiquement acceptable.
